# EUROPEAN PATENT APPLICATION

(11) **EP 2 944 652 A1**
(43) Date of publication of application: **18.11.2015**
(21) Application number: 14168107.2
(22) Date of filing: 13.05.2014
(51) Int. Cl.: C07K 14/725, A61K 38/00, A61K 39/00, C07K 7/06

(54) **Glypican-3-specific T-cell receptors and their uses for immunotherapy of hepatocellular carcinoma**

(71) Applicant: Technische Universität München, 80333 München (DE)
(72) Inventor: Protzer, Ulrike, 81735 Munich (DE); Dargel, Christina, 80796 Munich (DE)
(74) Representative: Engelhard, Markus

(57) **Abstract**

The present invention relates to glypican-3-specific T-cell receptors. The present invention further relates to soluble TCR constructs, chimeric TCRs, bi-specific antibodies, nucleic acids, expression constructs and cells comprising said TCRs or TCR constructs. The present invention further relates to the use of the TCR or the soluble TCR constructs or chimeric TCRs or bi-specific antibodies as a medicament, preferably in the detection, diagnosis, prognosis, prevention and/or treatment of liver cancer, in particular hepatocellular carcinoma, or other cancers expressing GPC3. The present invention further relates to methods of detecting, diagnosing, prognosing, preventing and/or treating liver cancer, in particular hepatocellular carcinoma, or other cancers expressing GPC3. The present invention further relates to peptides comprising glypican-3 epitope(s) and respective nucleic acids encoding them, antibodies and compositions as well as their use as (peptide) vaccines. The present invention further relates to vaccines comprising the peptide(s).

SEQUENCE LISTING

<110>  Technische Universität München

<120>  Glypican-3-specific T-cell receptors and their uses for
       immunotherapy of hepatocellular carcinoma

<130>  U30560EP

<160>  46

<170>  PatentIn version 3.5

<210>  1
<211>  134
<212>  PRT
<213>  Homo sapiens

<400>  1

Met Leu Leu Glu Leu Ile Pro Leu Leu Gly Ile His Phe Val Leu Arg
1               5                   10                  15

Thr Ala Arg Ala Gln Ser Val Thr Gln Pro Asp Ile His Ile Thr Val
            20                  25                  30

Ser Glu Gly Ala Ser Leu Glu Leu Arg Cys Asn Tyr Ser Tyr Gly Ala
        35                  40                  45

Thr Pro Tyr Leu Phe Trp Tyr Val Gln Ser Pro Gly Gln Gly Leu Gln
    50                  55                  60

Leu Leu Leu Lys Tyr Phe Ser Gly Asp Thr Leu Val Gln Gly Ile Lys
65                  70                  75                  80

Gly Phe Glu Ala Glu Phe Lys Arg Ser Gln Ser Ser Phe Asn Leu Arg
                85                  90                  95

Lys Pro Ser Val His Trp Ser Asp Ala Ala Glu Tyr Phe Cys Ala Val
            100                 105                 110

Gly Val Leu Asn Phe Gly Asn Glu Lys Leu Thr Phe Gly Thr Gly Thr
        115                 120                 125

Arg Leu Thr Ile Ile Pro
    130

<210>  2
<211>  131
<212>  PRT
<213>  Homo sapiens

<400>  2

Met Leu Cys Ser Leu Leu Ala Leu Leu Leu Gly Thr Phe Phe Gly Val
1               5                   10                  15

Arg Ser Gln Thr Ile His Gln Trp Pro Ala Thr Leu Val Gln Pro Val
            20                  25                  30

Gly Ser Pro Leu Ser Leu Glu Cys Thr Val Glu Gly Thr Ser Asn Pro
        35                  40                  45

Asn Leu Tyr Trp Tyr Arg Gln Ala Ala Gly Arg Gly Leu Gln Leu Leu
    50                  55                  60

Phe Tyr Ser Val Gly Ile Gly Gln Ile Ser Ser Glu Val Pro Gln Asn
65                  70                  75                  80

Leu Ser Ala Ser Arg Pro Gln Asp Arg Gln Phe Ile Leu Ser Ser Lys
                85                  90                  95

Lys Leu Leu Leu Ser Asp Ser Gly Phe Tyr Leu Cys Ala Trp Ser Glu
            100                 105                 110

Asp Thr Ala Tyr Tyr Gly Tyr Thr Phe Gly Ser Gly Thr Arg Leu Thr
        115                 120                 125

Val Val Glu
    130

<210>  3
<211>  136
<212>  PRT
<213>  Mus musculus

<400>  3

Ile Gln Asn Pro Glu Pro Ala Val Tyr Gln Leu Lys Asp Pro Arg Ser
1               5                   10                  15

Gln Asp Ser Thr Leu Cys Leu Phe Thr Asp Phe Asp Ser Gln Ile Asn
            20                  25                  30

Val Pro Lys Thr Met Glu Ser Gly Thr Phe Ile Thr Asp Lys Cys Val
        35                  40                  45

Leu Asp Met Lys Ala Met Asp Ser Lys Ser Asn Gly Ala Ile Ala Trp
    50                  55                  60

Ser Asn Gln Thr Ser Phe Thr Cys Gln Asp Ile Phe Lys Glu Thr Asn
65                  70                  75                  80

Ala Thr Tyr Pro Ser Ser Asp Val Pro Cys Asp Ala Thr Leu Thr Glu
                85                  90                  95

Lys Ser Phe Glu Thr Asp Met Asn Leu Asn Phe Gln Asn Leu Ser Val
            100                 105                 110

Met Gly Leu Arg Ile Leu Leu Leu Lys Val Ala Gly Phe Asn Leu Leu
        115                 120                 125

Met Thr Leu Arg Leu Trp Ser Ser
    130                 135

<210>  4
<211>  173
<212>  PRT
<213>  Mus musculus

<400>  4

Glu Asp Leu Arg Asn Val Thr Pro Pro Lys Val Ser Leu Phe Glu Pro
1               5                   10                  15

Ser Lys Ala Glu Ile Ala Asn Lys Gln Lys Ala Thr Leu Val Cys Leu
            20                  25                  30

Ala Arg Gly Phe Phe Pro Asp His Val Glu Leu Ser Trp Trp Val Asn
        35                  40                  45

Gly Lys Glu Val His Ser Gly Val Cys Thr Asp Pro Gln Ala Tyr Lys
    50                  55                  60

Glu Ser Asn Tyr Ser Tyr Cys Leu Ser Ser Arg Leu Arg Val Ser Ala
65                  70                  75                  80

Thr Phe Trp His Asn Pro Arg Asn His Phe Arg Cys Gln Val Gln Phe
                85                  90                  95

His Gly Leu Ser Glu Glu Asp Lys Trp Pro Glu Gly Ser Pro Lys Pro
            100                 105                 110

Val Thr Gln Asn Ile Ser Ala Glu Ala Trp Gly Arg Ala Asp Cys Gly
        115                 120                 125

Ile Thr Ser Ala Ser Tyr His Gln Gly Val Leu Ser Ala Thr Ile Leu
    130                 135                 140

Tyr Glu Ile Leu Leu Gly Lys Ala Thr Leu Tyr Ala Val Leu Val Ser
145                 150                 155                 160

Gly Leu Val Leu Met Ala Met Val Lys Lys Lys Asn Ser
                165                 170

<210>  5
<211>  22
<212>  PRT
<213>  Artificial Sequence

<220>
<223>  P2A linker

<400>  5

Gly Ser Gly Ala Thr Asn Phe Ser Leu Leu Lys Gln Ala Gly Asp Val
1               5                   10                  15

Glu Glu Asn Pro Gly Pro
            20

<210>  6
<211>  270
<212>  PRT
<213>  Artificial Sequence

<220>
<223>  Alpha chain of TCR P1-1

<400>  6

Met Leu Leu Glu Leu Ile Pro Leu Leu Gly Ile His Phe Val Leu Arg
1               5                   10                  15

Thr Ala Arg Ala Gln Ser Val Thr Gln Pro Asp Ile His Ile Thr Val
            20                  25                  30

Ser Glu Gly Ala Ser Leu Glu Leu Arg Cys Asn Tyr Ser Tyr Gly Ala
        35                  40                  45

Thr Pro Tyr Leu Phe Trp Tyr Val Gln Ser Pro Gly Gln Gly Leu Gln
    50                  55                  60

Leu Leu Leu Lys Tyr Phe Ser Gly Asp Thr Leu Val Gln Gly Ile Lys
65                  70                  75                  80

Gly Phe Glu Ala Glu Phe Lys Arg Ser Gln Ser Ser Phe Asn Leu Arg
                85                  90                  95

Lys Pro Ser Val His Trp Ser Asp Ala Ala Glu Tyr Phe Cys Ala Val
            100                 105                 110

Gly Val Leu Asn Phe Gly Asn Glu Lys Leu Thr Phe Gly Thr Gly Thr
        115                 120                 125

Arg Leu Thr Ile Ile Pro Ile Gln Asn Pro Glu Pro Ala Val Tyr Gln
    130                 135                 140

Leu Lys Asp Pro Arg Ser Gln Asp Ser Thr Leu Cys Leu Phe Thr Asp
145                 150                 155                 160

Phe Asp Ser Gln Ile Asn Val Pro Lys Thr Met Glu Ser Gly Thr Phe
                165                 170                 175

Ile Thr Asp Lys Cys Val Leu Asp Met Lys Ala Met Asp Ser Lys Ser
            180                 185                 190

Asn Gly Ala Ile Ala Trp Ser Asn Gln Thr Ser Phe Thr Cys Gln Asp
        195                 200                 205

Ile Phe Lys Glu Thr Asn Ala Thr Tyr Pro Ser Ser Asp Val Pro Cys
    210                 215                 220

Asp Ala Thr Leu Thr Glu Lys Ser Phe Glu Thr Asp Met Asn Leu Asn
225                 230                 235                 240

Phe Gln Asn Leu Ser Val Met Gly Leu Arg Ile Leu Leu Leu Lys Val
                245                 250                 255

Ala Gly Phe Asn Leu Leu Met Thr Leu Arg Leu Trp Ser Ser
            260                 265                 270

<210>  7
<211>  304
<212>  PRT
<213>  Artificial Sequence

<220>
<223>  Beta chain of TCR P1-1

<400>  7

Met Leu Cys Ser Leu Leu Ala Leu Leu Leu Gly Thr Phe Phe Gly Val
1               5                   10                  15

Arg Ser Gln Thr Ile His Gln Trp Pro Ala Thr Leu Val Gln Pro Val
            20                  25                  30

Gly Ser Pro Leu Ser Leu Glu Cys Thr Val Glu Gly Thr Ser Asn Pro
        35                  40                  45

Asn Leu Tyr Trp Tyr Arg Gln Ala Ala Gly Arg Gly Leu Gln Leu Leu
    50                  55                  60

Phe Tyr Ser Val Gly Ile Gly Gln Ile Ser Ser Glu Val Pro Gln Asn
65                  70                  75                  80

Leu Ser Ala Ser Arg Pro Gln Asp Arg Gln Phe Ile Leu Ser Ser Lys
                85                  90                  95

Lys Leu Leu Leu Ser Asp Ser Gly Phe Tyr Leu Cys Ala Trp Ser Glu
            100                 105                 110

Asp Thr Ala Tyr Tyr Gly Tyr Thr Phe Gly Ser Gly Thr Arg Leu Thr
        115                 120                 125

Val Val Glu Glu Asp Leu Arg Asn Val Thr Pro Pro Lys Val Ser Leu
    130                 135                 140

Phe Glu Pro Ser Lys Ala Glu Ile Ala Asn Lys Gln Lys Ala Thr Leu
145                 150                 155                 160

Val Cys Leu Ala Arg Gly Phe Phe Pro Asp His Val Glu Leu Ser Trp
                165                 170                 175

Trp Val Asn Gly Lys Glu Val His Ser Gly Val Cys Thr Asp Pro Gln
            180                 185                 190

Ala Tyr Lys Glu Ser Asn Tyr Ser Tyr Cys Leu Ser Ser Arg Leu Arg
        195                 200                 205

Val Ser Ala Thr Phe Trp His Asn Pro Arg Asn His Phe Arg Cys Gln
    210                 215                 220

Val Gln Phe His Gly Leu Ser Glu Glu Asp Lys Trp Pro Glu Gly Ser
225                 230                 235                 240

Pro Lys Pro Val Thr Gln Asn Ile Ser Ala Glu Ala Trp Gly Arg Ala
                245                 250                 255

Asp Cys Gly Ile Thr Ser Ala Ser Tyr His Gln Gly Val Leu Ser Ala
            260                 265                 270

Thr Ile Leu Tyr Glu Ile Leu Leu Gly Lys Ala Thr Leu Tyr Ala Val
        275                 280                 285

Leu Val Ser Gly Leu Val Leu Met Ala Met Val Lys Lys Lys Asn Ser
    290                 295                 300

<210>  8
<211>  596
<212>  PRT

<213>  Artificial Sequence

<220>
<223>  TCR P1-1

<400>  8

Met Leu Cys Ser Leu Leu Ala Leu Leu Leu Gly Thr Phe Phe Gly Val
1               5                   10                  15

Arg Ser Gln Thr Ile His Gln Trp Pro Ala Thr Leu Val Gln Pro Val
            20                  25                  30

Gly Ser Pro Leu Ser Leu Glu Cys Thr Val Glu Gly Thr Ser Asn Pro
        35                  40                  45

Asn Leu Tyr Trp Tyr Arg Gln Ala Ala Gly Arg Gly Leu Gln Leu Leu
    50                  55                  60

Phe Tyr Ser Val Gly Ile Gly Gln Ile Ser Ser Glu Val Pro Gln Asn
65                  70                  75                  80

Leu Ser Ala Ser Arg Pro Gln Asp Arg Gln Phe Ile Leu Ser Ser Lys
                85                  90                  95

Lys Leu Leu Leu Ser Asp Ser Gly Phe Tyr Leu Cys Ala Trp Ser Glu
            100                 105                 110

Asp Thr Ala Tyr Tyr Gly Tyr Thr Phe Gly Ser Gly Thr Arg Leu Thr
        115                 120                 125

Val Val Glu Glu Asp Leu Arg Asn Val Thr Pro Pro Lys Val Ser Leu
    130                 135                 140

Phe Glu Pro Ser Lys Ala Glu Ile Ala Asn Lys Gln Lys Ala Thr Leu
145                 150                 155                 160

Val Cys Leu Ala Arg Gly Phe Phe Pro Asp His Val Glu Leu Ser Trp
                165                 170                 175

Trp Val Asn Gly Lys Glu Val His Ser Gly Val Cys Thr Asp Pro Gln
            180                 185                 190

Ala Tyr Lys Glu Ser Asn Tyr Ser Tyr Cys Leu Ser Ser Arg Leu Arg
        195                 200                 205

Val Ser Ala Thr Phe Trp His Asn Pro Arg Asn His Phe Arg Cys Gln
    210                 215                 220

Val Gln Phe His Gly Leu Ser Glu Glu Asp Lys Trp Pro Glu Gly Ser
225                 230                 235                 240

Pro Lys Pro Val Thr Gln Asn Ile Ser Ala Glu Ala Trp Gly Arg Ala
                245                 250                 255

Asp Cys Gly Ile Thr Ser Ala Ser Tyr His Gln Gly Val Leu Ser Ala
            260                 265                 270

Thr Ile Leu Tyr Glu Ile Leu Leu Gly Lys Ala Thr Leu Tyr Ala Val
        275                 280                 285

Leu Val Ser Gly Leu Val Leu Met Ala Met Val Lys Lys Lys Asn Ser
    290                 295                 300

Gly Ser Gly Ala Thr Asn Phe Ser Leu Leu Lys Gln Ala Gly Asp Val
305                 310                 315                 320

Glu Glu Asn Pro Gly Pro Met Leu Leu Glu Leu Ile Pro Leu Leu Gly
                325                 330                 335

Ile His Phe Val Leu Arg Thr Ala Arg Ala Gln Ser Val Thr Gln Pro
            340                 345                 350

Asp Ile His Ile Thr Val Ser Glu Gly Ala Ser Leu Glu Leu Arg Cys
        355                 360                 365

Asn Tyr Ser Tyr Gly Ala Thr Pro Tyr Leu Phe Trp Tyr Val Gln Ser
    370                 375                 380

Pro Gly Gln Gly Leu Gln Leu Leu Leu Lys Tyr Phe Ser Gly Asp Thr
385                 390                 395                 400

Leu Val Gln Gly Ile Lys Gly Phe Glu Ala Glu Phe Lys Arg Ser Gln
                405                 410                 415

Ser Ser Phe Asn Leu Arg Lys Pro Ser Val His Trp Ser Asp Ala Ala
            420                 425                 430

Glu Tyr Phe Cys Ala Val Gly Val Leu Asn Phe Gly Asn Glu Lys Leu
        435                 440                 445

Thr Phe Gly Thr Gly Thr Arg Leu Thr Ile Ile Pro Ile Gln Asn Pro
    450                 455                 460

Glu Pro Ala Val Tyr Gln Leu Lys Asp Pro Arg Ser Gln Asp Ser Thr
465                 470                 475                 480

Leu Cys Leu Phe Thr Asp Phe Asp Ser Gln Ile Asn Val Pro Lys Thr
                485                 490                 495

Met Glu Ser Gly Thr Phe Ile Thr Asp Lys Cys Val Leu Asp Met Lys
            500                 505                 510

Ala Met Asp Ser Lys Ser Asn Gly Ala Ile Ala Trp Ser Asn Gln Thr
        515                 520                 525

Ser Phe Thr Cys Gln Asp Ile Phe Lys Glu Thr Asn Ala Thr Tyr Pro
    530                 535                 540

Ser Ser Asp Val Pro Cys Asp Ala Thr Leu Thr Glu Lys Ser Phe Glu
545                 550                 555                 560

Thr Asp Met Asn Leu Asn Phe Gln Asn Leu Ser Val Met Gly Leu Arg
                565                 570                 575

Ile Leu Leu Leu Lys Val Ala Gly Phe Asn Leu Leu Met Thr Leu Arg
            580                 585                 590

Leu Trp Ser Ser
        595

<210>  9
<211>  402
<212>  DNA
<213>  Homo sapiens

<400>  9
atgctgctgg aactgatccc tctgctgggc atccacttcg tgctgagaac cgccagagcc      60

cagagcgtga cccagcccga tatccacatc accgtgtctg agggcgccag cctggaactg     120

cggtgcaact acagctacgg cgccaccccc tacctgtttt ggtacgtgca gagccccgga     180

cagggcctgc agctgctgct gaagtacttt agcggcgaca ccctggtgca gggcatcaag     240

ggattcgagg ccgagttcaa gcggagccag agcagcttca acctgcggaa gccctccgtg     300

cattggagcg acgccgccga gtacttttgt gccgtgggcg tgctgaactt cggcaacgag     360

aagctgacct ttggcaccgg cacccggctg accatcatcc ct                        402

<210>  10
<211>  393
<212>  DNA
<213>  Homo sapiens

<400>  10
atgctgtgtt ctctgctggc tctgctgctg ggcaccttct ttggagtgcg gagccagacc      60

atccaccagt ggcctgctac actggtgcag cctgtgggca gccctctgag cctggaatgt     120

accgtggaag gcaccagcaa ccccaacctg tactggtaca gacaggccgc tggcagaggc 180

ctgcagctgc tgttttacag cgtgggcatc ggccagatca gcagcgaggt gccccagaat 240

ctgagcgcca gcagacccca ggaccggcag tttatcctga gcagcaagaa gctgctgctg 300

agcgacagcg gcttctacct gtgcgcttgg agcgaggaca ccgcctacta cggctacacc 360

tttggcagcg gcacccggct gacagtggtg gaa 393

<210> 11
<211> 813
<212> DNA
<213> Artificial Sequence

<220>
<223> Alpha chain of TCR P1-1

<400> 11
atgctgctgg aactgatccc tctgctgggc atccacttcg tgctgagaac cgccagagcc 60

cagagcgtga cccagcccga tatccacatc accgtgtctg agggcgccag cctggaactg 120

cggtgcaact acagctacgg cgccaccccc tacctgtttt ggtacgtgca gagccccgga 180

cagggcctgc agctgctgct gaagtacttt agcggcgaca ccctggtgca gggcatcaag 240

ggattcgagg ccgagttcaa gcggagccag agcagcttca acctgcggaa gccctccgtg 300

cattggagcg acgccgccga gtacttttgt gccgtgggcg tgctgaactt cggcaacgag 360

aagctgacct ttggcaccgg cacccggctg accatcatcc ctatccagaa ccccgagccc 420

gccgtgtacc agctgaagga ccccagaagc caggacagca ccctgtgtct gttcaccgac 480

ttcgacagcc agatcaacgt gcccaagacc atggaaagcg gcaccttcat caccgataag 540

tgcgtgctgg acatgaaggc catggacagc aagagcaacg gcgccattgc ctggtccaac 600

cagaccagct tcacatgcca ggacatcttc aaagagacaa acgccaccta cccttccagc 660

gacgtgccct gtgacgccac cctgaccgag aagtccttcg agacagacat gaatctgaat 720

ttccagaacc tgagcgtgat gggcctgcgg atcctgctgc tgaaagtggc cggcttcaac 780

ctgctgatga ccctgcggct gtggtccagc tga 813

<210> 12
<211> 912
<212> DNA
<213> Artificial Sequence

<220>
<223> Beta chain of TCR P1-1

<400> 12
atgctgtgtt ctctgctggc tctgctgctg ggcaccttct ttggagtgcg gagccagacc 60

atccaccagt ggcctgctac actggtgcag cctgtgggca gccctctgag cctggaatgt 120

accgtggaag gcaccagcaa ccccaacctg tactggtaca gacaggccgc tggcagaggc 180

ctgcagctgc tgttttacag cgtgggcatc ggccagatca gcagcgaggt gccccagaat 240

ctgagcgcca gcagacccca ggaccggcag tttatcctga gcagcaagaa gctgctgctg 300

agcgacagcg gcttctacct gtgcgcttgg agcgaggaca ccgcctacta cggctacacc 360

tttggcagcg gcacccggct gacagtggtg gaagaagatc tgaggaacgt gacccccccc 420

aaggtgtccc tgttcgagcc cagcaaggcc gagatcgcca acaagcagaa agccaccctc 480

gtgtgcctgg ccagaggctt cttccccgac cacgtggaac tgtcctggtg ggtcaacggc 540

aaagaggtgc acagcggagt ctgcaccgac ccccaggctt acaaagagag caactacagc 600

tactgcctgt ccagcagact gcgggtgtcc gctaccttct ggcacaaccc ccggaaccac 660

ttcagatgcc aggtgcagtt ccacggcctg agcgaagagg acaagtggcc cgagggcagc 720

cccaagcccg tgacccagaa tatcagcgcc gaggcctggg gcagagccga ttgtggcatc 780

accagcgcca gctaccacca gggggtgctg agcgccacca tcctgtacga gatcctgctg 840

ggcaaggcca ccctgtacgc cgtgctggtg tccggactgg tgctgatggc catggtcaag 900

aagaagaaca gc 912

<210> 13
<211> 1791
<212> DNA
<213> Artificial Sequence

<220>
<223> TCR P1-1

<400> 13
atgctgtgtt ctctgctggc tctgctgctg ggcaccttct ttggagtgcg gagccagacc 60

atccaccagt ggcctgctac actggtgcag cctgtgggca gccctctgag cctggaatgt 120

accgtggaag gcaccagcaa ccccaacctg tactggtaca gacaggccgc tggcagaggc 180

ctgcagctgc tgttttacag cgtgggcatc ggccagatca gcagcgaggt gccccagaat 240

ctgagcgcca gcagacccca ggaccggcag tttatcctga gcagcaagaa gctgctgctg 300

agcgacagcg gcttctacct gtgcgcttgg agcgaggaca ccgcctacta cggctacacc 360

tttggcagcg gcacccggct gacagtggtg gaagaagatc tgaggaacgt gacccccccc 420

aaggtgtccc tgttcgagcc cagcaaggcc gagatcgcca acaagcagaa agccaccctc 480

gtgtgcctgg ccagaggctt cttccccgac cacgtggaac tgtcctggtg ggtcaacggc 540

aaagaggtgc acagcggagt ctgcaccgac ccccaggctt acaaagagag caactacagc 600

tactgcctgt ccagcagact gcgggtgtcc gctaccttct ggcacaaccc ccggaaccac 660

ttcagatgcc aggtgcagtt ccacggcctg agcgaagagg acaagtggcc cgagggcagc 720

cccaagcccg tgacccagaa tatcagcgcc gaggcctggg gcagagccga ttgtggcatc 780

accagcgcca gctaccacca gggggtgctg agcgccacca tcctgtacga gatcctgctg 840

ggcaaggcca ccctgtacgc cgtgctggtg tccggactgg tgctgatggc catggtcaag 900

aagaagaaca gcggcagcgg cgccaccaac ttcagcctgc tgaagcaggc cggcgacgtg 960

gaagagaacc ccgggcccat gctgctggaa ctgatccctc tgctgggcat ccacttcgtg 1020

ctgagaaccg ccagagccca gagcgtgacc cagcccgata tccacatcac cgtgtctgag 1080

ggcgccagcc tggaactgcg gtgcaactac agctacggcg ccacccccta cctgttttgg 1140

tacgtgcaga gccccggaca gggcctgcag ctgctgctga agtactttag cggcgacacc 1200

ctggtgcagg gcatcaaggg attcgaggcc gagttcaagc ggagccagag cagcttcaac 1260

ctgcggaagc cctccgtgca ttggagcgac gccgccgagt acttttgtgc cgtgggcgtg 1320

ctgaacttcg gcaacgagaa gctgaccttt ggcaccggca cccggctgac catcatccct 1380

atccagaacc ccgagcccgc cgtgtaccag ctgaaggacc ccagaagcca ggacagcacc 1440

ctgtgtctgt tcaccgactt cgacagccag atcaacgtgc ccaagaccat ggaaagcggc 1500

accttcatca ccgataagtg cgtgctggac atgaaggcca tggacagcaa gagcaacggc 1560

gccattgcct ggtccaacca gaccagcttc acatgccagg acatcttcaa agagacaaac 1620

gccacctacc cttccagcga cgtgccctgt gacgccaccc tgaccgagaa gtccttcgag 1680

acagacatga atctgaattt ccagaacctg agcgtgatgg gcctgcggat cctgctgctg 1740

aaagtggccg gcttcaacct gctgatgacc ctgcggctgt ggtccagctg a 1791

<210> 14
<211> 9
<212> PRT
<213> Homo sapiens

<400> 14

Phe Ile Asp Lys Lys Val Leu Lys Val
1               5

<210> 15
<211> 580
<212> PRT
<213> Homo sapiens

<400> 15

<210>  16
<211>  1743
<212>  DNA
<213>  Homo sapiens

<400>  16
atggccggga ccgtgcgcac cgcgtgcttg gtggtggcga tgctgctcag cttggacttc      60

ccgggacagg cgcagccccc gccgccgccg ccggacgcca cctgtcacca agtccgctcc     120

ttcttccaga gactgcagcc cggactcaag tgggtgccag aaactcccgt gccaggatca     180

gatttgcaag tatgtctccc taagggccca acatgctgct caagaaagat ggaagaaaaa     240

taccaactaa cagcacgatt gaacatggaa cagctgcttc agtctgcaag tatggagctc     300

aagttcttaa ttattcagaa tgctgcggtt ttccaagagg cctttgaaat tgttgttcgc     360

catgccaaga actacaccaa tgccatgttc aagaacaact acccaagcct gactccacaa     420

gcttttgagt ttgtgggtga atttttcaca gatgtgtctc tctacatctt gggttctgac     480

atcaatgtag atgacatggt caatgaattg tttgacagcc tgtttccagt catctatacc     540

cagctaatga acccaggcct gcctgattca gccttggaca tcaatgagtg cctccgagga     600

gcaagacgtg acctgaaagt atttgggaat ttccccaagc ttattatgac ccaggtttcc     660

aagtcactgc aagtcactag gatcttcctt caggctctga atcttggaat tgaagtgatc     720

aacacaactg atcacctgaa gttcagtaag gactgtggcc gaatgctcac cagaatgtgg     780

tactgctctt actgccaggg actgatgatg gttaaaccct gtggcggtta ctgcaatgtg     840

gtcatgcaag gctgtatggc aggtgtggtg gagattgaca agtactggag agaatacatt     900

ctgtcccttg aagaacttgt gaatggcatg tacagaatct atgacatgga gaacgtactg     960

cttggtctct tttcaacaat ccatgattct atccagtatg tccagaagaa tgcaggaaag    1020

ctgaccacca ctattggcaa gttatgtgcc cattctcaac aacgccaata tagatctgct    1080

tattatcctg aagatctctt tattgacaag aaagtattaa aagttgctca tgtagaacat    1140

gaagaaacct tatccagccg aagaagggaa ctaattcaga agttgaagtc tttcatcagc    1200

ttctatagtg ctttgcctgg ctacatctgc agccatagcc ctgtggcgga aaacgacacc    1260

ctttgctgga atggacaaga actcgtggag agatacagcc aaaaggcagc aaggaatgga    1320

atgaaaaacc agttcaatct ccatgagctg aaaatgaagg gccctgagcc agtggtcagt 1380

caaattattg acaaactgaa gcacattaac cagctcctga gaaccatgtc tatgcccaaa 1440

ggtagagttc tggataaaaa cctggatgag gaagggtttg aaagtggaga ctgcggtgat 1500

gatgaagatg agtgcattgg aggctctggt gatggaatga taaaagtgaa gaatcagctc 1560

cgcttccttg cagaactggc ctatgatctg gatgtggatg atgcgcctgg aaacagtcag 1620

caggcaactc cgaaggacaa cgagataagc acctttcaca acctcgggaa cgttcattcc 1680

ccgctgaagc ttctcaccag catggccatc tcggtggtgt gcttcttctt cctggtgcac 1740

tga 1743

<210> 17
<211> 9
<212> PRT
<213> Homo sapiens

<400> 17

Thr Ile His Asp Ser Ile Gln Tyr Val
1               5

<210> 18
<211> 133
<212> PRT
<213> Homo sapiens

<400> 18

Met Lys Ser Leu Arg Val Leu Leu Val Ile Leu Trp Leu Gln Leu Ser
1               5                   10                  15

Trp Val Trp Ser Gln Gln Lys Glu Val Glu Gln Asn Ser Gly Pro Leu
            20                  25                  30

Ser Val Pro Glu Gly Ala Ile Ala Ser Leu Asn Cys Thr Tyr Ser Asp
        35                  40                  45

Arg Gly Ser Gln Ser Phe Phe Trp Tyr Arg Gln Tyr Ser Gly Lys Ser
    50                  55                  60

Pro Glu Leu Ile Met Phe Ile Tyr Ser Asn Gly Asp Lys Glu Asp Gly
65                  70                  75                  80

Arg Phe Thr Ala Gln Leu Asn Lys Ala Ser Gln Tyr Val Ser Leu Leu
                85                  90                  95

Ile Arg Asp Ser Gln Pro Ser Asp Ser Ala Thr Tyr Leu Cys Ala Ala
            100                 105                 110

Lys Met Lys Thr Ser Tyr Asp Lys Val Ile Phe Gly Pro Gly Thr Ser
        115                 120                 125

Leu Ser Val Ile Pro
    130

<210>  19
<211>  142
<212>  PRT
<213>  Homo sapiens

<220>
<221>  misc_feature
<222>  (142)..(142)
<223>  Xaa can be any naturally occurring amino acid

<400>  19

Met Lys Ser Leu Arg Val Leu Leu Val Ile Leu Trp Leu Gln Leu Ser
1               5                   10                  15

Trp Val Trp Ser Gln Gln Lys Glu Val Glu Gln Asn Ser Gly Pro Leu
            20                  25                  30

Ser Val Pro Glu Gly Ala Ile Ala Ser Leu Asn Cys Thr Tyr Ser Asp
        35                  40                  45

Arg Gly Ser Gln Ser Phe Phe Trp Tyr Arg Gln Tyr Ser Gly Lys Ser
    50                  55                  60

Pro Glu Leu Ile Met Phe Ile Tyr Ser Asn Gly Asp Lys Glu Asp Gly
65                  70                  75                  80

Arg Phe Thr Ala Gln Leu Asn Lys Ala Ser Gln Tyr Val Ser Leu Leu
                85                  90                  95

Ile Arg Asp Ser Gln Pro Ser Asp Ser Ala Thr Tyr Leu Cys Ala Gly
            100                 105                 110

Tyr Ser Gly Gly Gly Ala Asp Gly Leu Thr Phe Gly Lys Gly Thr His
        115                 120                 125

Leu Ile Ile Gln Pro Glu Asp Leu Arg Asn Val Thr Pro Xaa
    130                 135                 140

<210>  20
<211>  131
<212>  PRT
<213>  Homo sapiens

<400>  20

Met Thr Ser Ile Arg Ala Val Phe Ile Phe Leu Trp Leu Gln Leu Asp
1               5                   10                  15

Leu Val Asn Gly Glu Asn Val Glu Gln His Pro Ser Thr Leu Ser Val
            20                  25                  30

Gln Glu Gly Asp Ser Ala Val Ile Lys Cys Thr Tyr Ser Asp Ser Ala
        35                  40                  45

Ser Asn Tyr Phe Pro Trp Tyr Lys Gln Glu Leu Gly Lys Arg Pro Gln
    50                  55                  60

Leu Ile Ile Asp Ile Arg Ser Asn Val Gly Glu Lys Lys Asp Gln Arg
65                  70                  75                  80

Ile Ala Val Thr Leu Asn Lys Thr Ala Lys His Phe Ser Leu His Ile
                85                  90                  95

Thr Glu Thr Gln Pro Glu Asp Ser Ala Val Tyr Phe Cys Ala Ala Ser
            100                 105                 110

Asn Gly Gln Gly Ala Gln Lys Leu Val Phe Gly Gln Gly Thr Arg Leu
        115                 120                 125

Thr Ile Asn
    130

<210>  21
<211>  133
<212>  PRT
<213>  Homo sapiens

<400>  21

Met Gly Phe Arg Leu Leu Cys Cys Val Ala Phe Cys Leu Leu Gly Ala
1               5                   10                  15

Gly Pro Val Asp Ser Gly Val Thr Gln Thr Pro Lys His Leu Ile Thr
            20                  25                  30

Ala Thr Gly Gln Arg Val Thr Leu Arg Cys Ser Pro Arg Ser Gly Asp
        35                  40                  45

Leu Ser Val Tyr Trp Tyr Gln Gln Ser Leu Asp Gln Gly Leu Gln Phe
    50                  55                  60

Leu Ile Gln Tyr Tyr Asn Gly Glu Glu Arg Ala Lys Gly Asn Ile Leu
65                  70                  75                  80

Glu Arg Phe Ser Ala Gln Gln Phe Pro Asp Leu His Ser Glu Leu Asn
                85                  90                  95

Leu Ser Ser Leu Glu Leu Gly Asp Ser Ala Leu Tyr Phe Cys Ala Ser
            100                 105                 110

Ser Pro Gly Gly Ile Gly Tyr Glu Gln Tyr Phe Gly Pro Gly Thr Arg
        115                 120                 125

Leu Thr Val Thr Glu
    130

<210>  22
<211>  136
<212>  PRT
<213>  Mus musculus

<400>  22

Ile Gln Asn Pro Glu Pro Ala Val Tyr Gln Leu Lys Asp Pro Arg Ser
1               5                   10                  15

Gln Asp Ser Thr Leu Cys Leu Phe Thr Asp Phe Asp Ser Gln Ile Asn
            20                  25                  30

Val Pro Lys Thr Met Glu Ser Gly Thr Phe Ile Thr Asp Lys Cys Val
        35                  40                  45

Leu Asp Met Lys Ala Met Asp Ser Lys Ser Asn Gly Ala Ile Ala Trp
    50                  55                  60

Ser Asn Gln Thr Ser Phe Thr Cys Gln Asp Ile Phe Lys Glu Thr Asn
65                  70                  75                  80

Ala Thr Tyr Pro Ser Ser Asp Val Pro Cys Asp Ala Thr Leu Thr Glu
                85                  90                  95

Lys Ser Phe Glu Thr Asp Met Asn Leu Asn Phe Gln Asn Leu Ser Val
            100                 105                 110

Met Gly Leu Arg Ile Leu Leu Leu Lys Val Ala Gly Phe Asn Leu Leu
        115                 120                 125

Met Thr Leu Arg Leu Trp Ser Ser
    130                 135

<210>  23
<211>  173
<212>  PRT

<213>  Mus musculus

<400>  23

Glu Asp Leu Arg Asn Val Thr Pro Pro Lys Val Ser Leu Phe Glu Pro
1               5                   10                  15

Ser Lys Ala Glu Ile Ala Asn Lys Gln Lys Ala Thr Leu Val Cys Leu
            20                  25                  30

Ala Arg Gly Phe Phe Pro Asp His Val Glu Leu Ser Trp Trp Val Asn
        35                  40                  45

Gly Lys Glu Val His Ser Gly Val Cys Thr Asp Pro Gln Ala Tyr Lys
    50                  55                  60

Glu Ser Asn Tyr Ser Tyr Cys Leu Ser Ser Arg Leu Arg Val Ser Ala
65                  70                  75                  80

Thr Phe Trp His Asn Pro Arg Asn His Phe Arg Cys Gln Val Gln Phe
                85                  90                  95

His Gly Leu Ser Glu Glu Asp Lys Trp Pro Glu Gly Ser Pro Lys Pro
            100                 105                 110

Val Thr Gln Asn Ile Ser Ala Glu Ala Trp Gly Arg Ala Asp Cys Gly
        115                 120                 125

Ile Thr Ser Ala Ser Tyr His Gln Gly Val Leu Ser Ala Thr Ile Leu
    130                 135                 140

Tyr Glu Ile Leu Leu Gly Lys Ala Thr Leu Tyr Ala Val Leu Val Ser
145                 150                 155                 160

Gly Leu Val Leu Met Ala Met Val Lys Lys Lys Asn Ser
                165                 170


<210>  24
<211>  269
<212>  PRT
<213>  Artificial Sequence

<220>
<223>  alpha chain of TCR P2-1 (with variable alpha of SEQ ID NO. 18)

<400>  24

Met Lys Ser Leu Arg Val Leu Leu Val Ile Leu Trp Leu Gln Leu Ser
1               5                   10                  15

Trp Val Trp Ser Gln Gln Lys Glu Val Glu Gln Asn Ser Gly Pro Leu

20                  25                  30

Ser Val Pro Glu Gly Ala Ile Ala Ser Leu Asn Cys Thr Tyr Ser Asp
        35                  40                  45

Arg Gly Ser Gln Ser Phe Phe Trp Tyr Arg Gln Tyr Ser Gly Lys Ser
    50                  55                  60

Pro Glu Leu Ile Met Phe Ile Tyr Ser Asn Gly Asp Lys Glu Asp Gly
65                  70                  75                  80

Arg Phe Thr Ala Gln Leu Asn Lys Ala Ser Gln Tyr Val Ser Leu Leu
                85                  90                  95

Ile Arg Asp Ser Gln Pro Ser Asp Ser Ala Thr Tyr Leu Cys Ala Ala
            100                 105                 110

Lys Met Lys Thr Ser Tyr Asp Lys Val Ile Phe Gly Pro Gly Thr Ser
        115                 120                 125

Leu Ser Val Ile Pro Ile Gln Asn Pro Glu Pro Ala Val Tyr Gln Leu
    130                 135                 140

Lys Asp Pro Arg Ser Gln Asp Ser Thr Leu Cys Leu Phe Thr Asp Phe
145                 150                 155                 160

Asp Ser Gln Ile Asn Val Pro Lys Thr Met Glu Ser Gly Thr Phe Ile
                165                 170                 175

Thr Asp Lys Cys Val Leu Asp Met Lys Ala Met Asp Ser Lys Ser Asn
            180                 185                 190

Gly Ala Ile Ala Trp Ser Asn Gln Thr Ser Phe Thr Cys Gln Asp Ile
        195                 200                 205

Phe Lys Glu Thr Asn Ala Thr Tyr Pro Ser Ser Asp Val Pro Cys Asp
    210                 215                 220

Ala Thr Leu Thr Glu Lys Ser Phe Glu Thr Asp Met Asn Leu Asn Phe
225                 230                 235                 240

Gln Asn Leu Ser Val Met Gly Leu Arg Ile Leu Leu Leu Lys Val Ala
                245                 250                 255

Gly Phe Asn Leu Leu Met Thr Leu Arg Leu Trp Ser Ser
            260                 265

<210>  25
<211>  278
<212>  PRT
<213>  Artificial Sequence

<220>
<223>  alpha chain of TCR P2-1 (with variable alpha of SEQ ID NO. 19)


<220>
<221>  misc_feature
<222>  (142)..(142)
<223>  Xaa can be any naturally occurring amino acid

<400>  25

Met Lys Ser Leu Arg Val Leu Leu Val Ile Leu Trp Leu Gln Leu Ser
1               5                   10                  15


Trp Val Trp Ser Gln Gln Lys Glu Val Glu Gln Asn Ser Gly Pro Leu
            20                  25                  30


Ser Val Pro Glu Gly Ala Ile Ala Ser Leu Asn Cys Thr Tyr Ser Asp
        35                  40                  45


Arg Gly Ser Gln Ser Phe Phe Trp Tyr Arg Gln Tyr Ser Gly Lys Ser
    50                  55                  60


Pro Glu Leu Ile Met Phe Ile Tyr Ser Asn Gly Asp Lys Glu Asp Gly
65                  70                  75                  80


Arg Phe Thr Ala Gln Leu Asn Lys Ala Ser Gln Tyr Val Ser Leu Leu
                85                  90                  95


Ile Arg Asp Ser Gln Pro Ser Asp Ser Ala Thr Tyr Leu Cys Ala Gly
            100                 105                 110


Tyr Ser Gly Gly Gly Ala Asp Gly Leu Thr Phe Gly Lys Gly Thr His
        115                 120                 125


Leu Ile Ile Gln Pro Glu Asp Leu Arg Asn Val Thr Pro Xaa Ile Gln
    130                 135                 140


Asn Pro Glu Pro Ala Val Tyr Gln Leu Lys Asp Pro Arg Ser Gln Asp
145                 150                 155                 160


Ser Thr Leu Cys Leu Phe Thr Asp Phe Asp Ser Gln Ile Asn Val Pro
                165                 170                 175


Lys Thr Met Glu Ser Gly Thr Phe Ile Thr Asp Lys Cys Val Leu Asp
            180                 185                 190

Met Lys Ala Met Asp Ser Lys Ser Asn Gly Ala Ile Ala Trp Ser Asn
        195                 200                 205

Gln Thr Ser Phe Thr Cys Gln Asp Ile Phe Lys Glu Thr Asn Ala Thr
    210                 215                 220

Tyr Pro Ser Ser Asp Val Pro Cys Asp Ala Thr Leu Thr Glu Lys Ser
225                 230                 235                 240

Phe Glu Thr Asp Met Asn Leu Asn Phe Gln Asn Leu Ser Val Met Gly
                245                 250                 255

Leu Arg Ile Leu Leu Leu Lys Val Ala Gly Phe Asn Leu Leu Met Thr
            260                 265                 270

Leu Arg Leu Trp Ser Ser
        275

<210>  26
<211>  267
<212>  PRT
<213>  Artificial Sequence

<220>
<223>  alpha chain of TCR P2-1 (with variable alpha of SEQ ID NO. 20)

<400>  26

Met Thr Ser Ile Arg Ala Val Phe Ile Phe Leu Trp Leu Gln Leu Asp
1               5                   10                  15

Leu Val Asn Gly Glu Asn Val Glu Gln His Pro Ser Thr Leu Ser Val
            20                  25                  30

Gln Glu Gly Asp Ser Ala Val Ile Lys Cys Thr Tyr Ser Asp Ser Ala
        35                  40                  45

Ser Asn Tyr Phe Pro Trp Tyr Lys Gln Glu Leu Gly Lys Arg Pro Gln
    50                  55                  60

Leu Ile Ile Asp Ile Arg Ser Asn Val Gly Glu Lys Lys Asp Gln Arg
65                  70                  75                  80

Ile Ala Val Thr Leu Asn Lys Thr Ala Lys His Phe Ser Leu His Ile
                85                  90                  95

Thr Glu Thr Gln Pro Glu Asp Ser Ala Val Tyr Phe Cys Ala Ala Ser
            100                 105                 110

Asn Gly Gln Gly Ala Gln Lys Leu Val Phe Gly Gln Gly Thr Arg Leu
        115                 120                 125

Thr Ile Asn Ile Gln Asn Pro Glu Pro Ala Val Tyr Gln Leu Lys Asp
    130                 135                 140

Pro Arg Ser Gln Asp Ser Thr Leu Cys Leu Phe Thr Asp Phe Asp Ser
145                 150                 155                 160

Gln Ile Asn Val Pro Lys Thr Met Glu Ser Gly Thr Phe Ile Thr Asp
                165                 170                 175

Lys Cys Val Leu Asp Met Lys Ala Met Asp Ser Lys Ser Asn Gly Ala
            180                 185                 190

Ile Ala Trp Ser Asn Gln Thr Ser Phe Thr Cys Gln Asp Ile Phe Lys
        195                 200                 205

Glu Thr Asn Ala Thr Tyr Pro Ser Ser Asp Val Pro Cys Asp Ala Thr
    210                 215                 220

Leu Thr Glu Lys Ser Phe Glu Thr Asp Met Asn Leu Asn Phe Gln Asn
225                 230                 235                 240

Leu Ser Val Met Gly Leu Arg Ile Leu Leu Leu Lys Val Ala Gly Phe
                245                 250                 255

Asn Leu Leu Met Thr Leu Arg Leu Trp Ser Ser
            260                 265

<210>  27
<211>  306
<212>  PRT
<213>  Artificial Sequence

<220>
<223>  beta chain of TCR P2-1

<400>  27

Met Gly Phe Arg Leu Leu Cys Cys Val Ala Phe Cys Leu Leu Gly Ala
1               5                   10                  15

Gly Pro Val Asp Ser Gly Val Thr Gln Thr Pro Lys His Leu Ile Thr
            20                  25                  30

Ala Thr Gly Gln Arg Val Thr Leu Arg Cys Ser Pro Arg Ser Gly Asp
        35                  40                  45

Leu Ser Val Tyr Trp Tyr Gln Gln Ser Leu Asp Gln Gly Leu Gln Phe

50                  55                  60

Leu Ile Gln Tyr Tyr Asn Gly Glu Glu Arg Ala Lys Gly Asn Ile Leu
65                  70                  75                  80

Glu Arg Phe Ser Ala Gln Gln Phe Pro Asp Leu His Ser Glu Leu Asn
                85                  90                  95

Leu Ser Ser Leu Glu Leu Gly Asp Ser Ala Leu Tyr Phe Cys Ala Ser
            100                 105                 110

Ser Pro Gly Gly Ile Gly Tyr Glu Gln Tyr Phe Gly Pro Gly Thr Arg
        115                 120                 125

Leu Thr Val Thr Glu Glu Asp Leu Arg Asn Val Thr Pro Pro Lys Val
    130                 135                 140

Ser Leu Phe Glu Pro Ser Lys Ala Glu Ile Ala Asn Lys Gln Lys Ala
145                 150                 155                 160

Thr Leu Val Cys Leu Ala Arg Gly Phe Phe Pro Asp His Val Glu Leu
                165                 170                 175

Ser Trp Trp Val Asn Gly Lys Glu Val His Ser Gly Val Cys Thr Asp
            180                 185                 190

Pro Gln Ala Tyr Lys Glu Ser Asn Tyr Ser Tyr Cys Leu Ser Ser Arg
        195                 200                 205

Leu Arg Val Ser Ala Thr Phe Trp His Asn Pro Arg Asn His Phe Arg
    210                 215                 220

Cys Gln Val Gln Phe His Gly Leu Ser Glu Glu Asp Lys Trp Pro Glu
225                 230                 235                 240

Gly Ser Pro Lys Pro Val Thr Gln Asn Ile Ser Ala Glu Ala Trp Gly
                245                 250                 255

Arg Ala Asp Cys Gly Ile Thr Ser Ala Ser Tyr His Gln Gly Val Leu
            260                 265                 270

Ser Ala Thr Ile Leu Tyr Glu Ile Leu Leu Gly Lys Ala Thr Leu Tyr
        275                 280                 285

Ala Val Leu Val Ser Gly Leu Val Leu Met Ala Met Val Lys Lys Lys
    290                 295                 300

Asn Ser
305

<210> 28
<211> 597
<212> PRT
<213> Artificial Sequence

<220>
<223> TCR P2-1 (with variable alpha of SEQ ID NO. 18)

<400> 28

Met Gly Phe Arg Leu Leu Cys Cys Val Ala Phe Cys Leu Leu Gly Ala
1               5                   10                  15

Gly Pro Val Asp Ser Gly Val Thr Gln Thr Pro Lys His Leu Ile Thr
            20                  25                  30

Ala Thr Gly Gln Arg Val Thr Leu Arg Cys Ser Pro Arg Ser Gly Asp
        35                  40                  45

Leu Ser Val Tyr Trp Tyr Gln Gln Ser Leu Asp Gln Gly Leu Gln Phe
    50                  55                  60

Leu Ile Gln Tyr Tyr Asn Gly Glu Glu Arg Ala Lys Gly Asn Ile Leu
65                  70                  75                  80

Glu Arg Phe Ser Ala Gln Gln Phe Pro Asp Leu His Ser Glu Leu Asn
                85                  90                  95

Leu Ser Ser Leu Glu Leu Gly Asp Ser Ala Leu Tyr Phe Cys Ala Ser
            100                 105                 110

Ser Pro Gly Gly Ile Gly Tyr Glu Gln Tyr Phe Gly Pro Gly Thr Arg
        115                 120                 125

Leu Thr Val Thr Glu Glu Asp Leu Arg Asn Val Thr Pro Pro Lys Val
    130                 135                 140

Ser Leu Phe Glu Pro Ser Lys Ala Glu Ile Ala Asn Lys Gln Lys Ala
145                 150                 155                 160

Thr Leu Val Cys Leu Ala Arg Gly Phe Phe Pro Asp His Val Glu Leu
                165                 170                 175

Ser Trp Trp Val Asn Gly Lys Glu Val His Ser Gly Val Cys Thr Asp
            180                 185                 190

Pro Gln Ala Tyr Lys Glu Ser Asn Tyr Ser Tyr Cys Leu Ser Ser Arg

195                 200                 205

Leu Arg Val Ser Ala Thr Phe Trp His Asn Pro Arg Asn His Phe Arg
    210                 215                 220

Cys Gln Val Gln Phe His Gly Leu Ser Glu Glu Asp Lys Trp Pro Glu
225                 230                 235                 240

Gly Ser Pro Lys Pro Val Thr Gln Asn Ile Ser Ala Glu Ala Trp Gly
                245                 250                 255

Arg Ala Asp Cys Gly Ile Thr Ser Ala Ser Tyr His Gln Gly Val Leu
            260                 265                 270

Ser Ala Thr Ile Leu Tyr Glu Ile Leu Leu Gly Lys Ala Thr Leu Tyr
        275                 280                 285

Ala Val Leu Val Ser Gly Leu Val Leu Met Ala Met Val Lys Lys Lys
    290                 295                 300

Asn Ser Gly Ser Gly Ala Thr Asn Phe Ser Leu Leu Lys Gln Ala Gly
305                 310                 315                 320

Asp Val Glu Glu Asn Pro Gly Pro Met Lys Ser Leu Arg Val Leu Leu
                325                 330                 335

Val Ile Leu Trp Leu Gln Leu Ser Trp Val Trp Ser Gln Gln Lys Glu
            340                 345                 350

Val Glu Gln Asn Ser Gly Pro Leu Ser Val Pro Glu Gly Ala Ile Ala
        355                 360                 365

Ser Leu Asn Cys Thr Tyr Ser Asp Arg Gly Ser Gln Ser Phe Phe Trp
    370                 375                 380

Tyr Arg Gln Tyr Ser Gly Lys Ser Pro Glu Leu Ile Met Phe Ile Tyr
385                 390                 395                 400

Ser Asn Gly Asp Lys Glu Asp Gly Arg Phe Thr Ala Gln Leu Asn Lys
                405                 410                 415

Ala Ser Gln Tyr Val Ser Leu Leu Ile Arg Asp Ser Gln Pro Ser Asp
            420                 425                 430

Ser Ala Thr Tyr Leu Cys Ala Ala Lys Met Lys Thr Ser Tyr Asp Lys
        435                 440                 445

Val Ile Phe Gly Pro Gly Thr Ser Leu Ser Val Ile Pro Ile Gln Asn
    450                 455                 460

Pro Glu Pro Ala Val Tyr Gln Leu Lys Asp Pro Arg Ser Gln Asp Ser
465                 470                 475                 480

Thr Leu Cys Leu Phe Thr Asp Phe Asp Ser Gln Ile Asn Val Pro Lys
                485                 490                 495

Thr Met Glu Ser Gly Thr Phe Ile Thr Asp Lys Cys Val Leu Asp Met
            500                 505                 510

Lys Ala Met Asp Ser Lys Ser Asn Gly Ala Ile Ala Trp Ser Asn Gln
        515                 520                 525

Thr Ser Phe Thr Cys Gln Asp Ile Phe Lys Glu Thr Asn Ala Thr Tyr
    530                 535                 540

Pro Ser Ser Asp Val Pro Cys Asp Ala Thr Leu Thr Glu Lys Ser Phe
545                 550                 555                 560

Glu Thr Asp Met Asn Leu Asn Phe Gln Asn Leu Ser Val Met Gly Leu
                565                 570                 575

Arg Ile Leu Leu Leu Lys Val Ala Gly Phe Asn Leu Leu Met Thr Leu
            580                 585                 590

Arg Leu Trp Ser Ser
        595

<210>  29
<211>  606
<212>  PRT
<213>  Artificial Sequence

<220>
<223>  TCR P2-1 (with variable alpha of SEQ ID NO. 19)


<220>
<221>  misc_feature
<222>  (470)..(470)
<223>  Xaa can be any naturally occurring amino acid

<400>  29

Met Gly Phe Arg Leu Leu Cys Cys Val Ala Phe Cys Leu Leu Gly Ala
1               5                   10                  15

Gly Pro Val Asp Ser Gly Val Thr Gln Thr Pro Lys His Leu Ile Thr
            20                  25                  30

Ala Thr Gly Gln Arg Val Thr Leu Arg Cys Ser Pro Arg Ser Gly Asp
        35                  40                  45

Leu Ser Val Tyr Trp Tyr Gln Gln Ser Leu Asp Gln Gly Leu Gln Phe
    50                  55                  60

Leu Ile Gln Tyr Tyr Asn Gly Glu Glu Arg Ala Lys Gly Asn Ile Leu
65                  70                  75                  80

Glu Arg Phe Ser Ala Gln Gln Phe Pro Asp Leu His Ser Glu Leu Asn
                85                  90                  95

Leu Ser Ser Leu Glu Leu Gly Asp Ser Ala Leu Tyr Phe Cys Ala Ser
            100                 105                 110

Ser Pro Gly Gly Ile Gly Tyr Glu Gln Tyr Phe Gly Pro Gly Thr Arg
        115                 120                 125

Leu Thr Val Thr Glu Glu Asp Leu Arg Asn Val Thr Pro Pro Lys Val
    130                 135                 140

Ser Leu Phe Glu Pro Ser Lys Ala Glu Ile Ala Asn Lys Gln Lys Ala
145                 150                 155                 160

Thr Leu Val Cys Leu Ala Arg Gly Phe Phe Pro Asp His Val Glu Leu
                165                 170                 175

Ser Trp Trp Val Asn Gly Lys Glu Val His Ser Gly Val Cys Thr Asp
            180                 185                 190

Pro Gln Ala Tyr Lys Glu Ser Asn Tyr Ser Tyr Cys Leu Ser Ser Arg
        195                 200                 205

Leu Arg Val Ser Ala Thr Phe Trp His Asn Pro Arg Asn His Phe Arg
    210                 215                 220

Cys Gln Val Gln Phe His Gly Leu Ser Glu Glu Asp Lys Trp Pro Glu
225                 230                 235                 240

Gly Ser Pro Lys Pro Val Thr Gln Asn Ile Ser Ala Glu Ala Trp Gly
                245                 250                 255

Arg Ala Asp Cys Gly Ile Thr Ser Ala Ser Tyr His Gln Gly Val Leu
            260                 265                 270

Ser Ala Thr Ile Leu Tyr Glu Ile Leu Leu Gly Lys Ala Thr Leu Tyr
        275                 280                 285

Ala Val Leu Val Ser Gly Leu Val Leu Met Ala Met Val Lys Lys Lys
    290                 295                 300

Asn Ser Gly Ser Gly Ala Thr Asn Phe Ser Leu Leu Lys Gln Ala Gly
305                 310                 315                 320

Asp Val Glu Glu Asn Pro Gly Pro Met Lys Ser Leu Arg Val Leu Leu
                325                 330                 335

Val Ile Leu Trp Leu Gln Leu Ser Trp Val Trp Ser Gln Gln Lys Glu
            340                 345                 350

Val Glu Gln Asn Ser Gly Pro Leu Ser Val Pro Glu Gly Ala Ile Ala
        355                 360                 365

Ser Leu Asn Cys Thr Tyr Ser Asp Arg Gly Ser Gln Ser Phe Phe Trp
    370                 375                 380

Tyr Arg Gln Tyr Ser Gly Lys Ser Pro Glu Leu Ile Met Phe Ile Tyr
385                 390                 395                 400

Ser Asn Gly Asp Lys Glu Asp Gly Arg Phe Thr Ala Gln Leu Asn Lys
                405                 410                 415

Ala Ser Gln Tyr Val Ser Leu Leu Ile Arg Asp Ser Gln Pro Ser Asp
            420                 425                 430

Ser Ala Thr Tyr Leu Cys Ala Gly Tyr Ser Gly Gly Gly Ala Asp Gly
        435                 440                 445

Leu Thr Phe Gly Lys Gly Thr His Leu Ile Ile Gln Pro Glu Asp Leu
    450                 455                 460

Arg Asn Val Thr Pro Xaa Ile Gln Asn Pro Glu Pro Ala Val Tyr Gln
465                 470                 475                 480

Leu Lys Asp Pro Arg Ser Gln Asp Ser Thr Leu Cys Leu Phe Thr Asp
                485                 490                 495

Phe Asp Ser Gln Ile Asn Val Pro Lys Thr Met Glu Ser Gly Thr Phe
            500                 505                 510

Ile Thr Asp Lys Cys Val Leu Asp Met Lys Ala Met Asp Ser Lys Ser
        515                 520                 525

Asn Gly Ala Ile Ala Trp Ser Asn Gln Thr Ser Phe Thr Cys Gln Asp

530                 535                 540

Ile Phe Lys Glu Thr Asn Ala Thr Tyr Pro Ser Ser Asp Val Pro Cys
545                 550                 555                 560

Asp Ala Thr Leu Thr Glu Lys Ser Phe Glu Thr Asp Met Asn Leu Asn
                565                 570                 575

Phe Gln Asn Leu Ser Val Met Gly Leu Arg Ile Leu Leu Leu Lys Val
            580                 585                 590

Ala Gly Phe Asn Leu Leu Met Thr Leu Arg Leu Trp Ser Ser
        595                 600                 605

<210>  30
<211>  595
<212>  PRT
<213>  Artificial Sequence

<220>
<223>  TCR P2-1 (with variable alpha of SEQ ID NO. 20)

<400>  30

Met Gly Phe Arg Leu Leu Cys Cys Val Ala Phe Cys Leu Leu Gly Ala
1               5                   10                  15

Gly Pro Val Asp Ser Gly Val Thr Gln Thr Pro Lys His Leu Ile Thr
            20                  25                  30

Ala Thr Gly Gln Arg Val Thr Leu Arg Cys Ser Pro Arg Ser Gly Asp
        35                  40                  45

Leu Ser Val Tyr Trp Tyr Gln Gln Ser Leu Asp Gln Gly Leu Gln Phe
    50                  55                  60

Leu Ile Gln Tyr Tyr Asn Gly Glu Glu Arg Ala Lys Gly Asn Ile Leu
65                  70                  75                  80

Glu Arg Phe Ser Ala Gln Gln Phe Pro Asp Leu His Ser Glu Leu Asn
                85                  90                  95

Leu Ser Ser Leu Glu Leu Gly Asp Ser Ala Leu Tyr Phe Cys Ala Ser
            100                 105                 110

Ser Pro Gly Gly Ile Gly Tyr Glu Gln Tyr Phe Gly Pro Gly Thr Arg
        115                 120                 125

Leu Thr Val Thr Glu Glu Asp Leu Arg Asn Val Thr Pro Pro Lys Val
    130                 135                 140

Ser Leu Phe Glu Pro Ser Lys Ala Glu Ile Ala Asn Lys Gln Lys Ala
145                 150                 155                 160

Thr Leu Val Cys Leu Ala Arg Gly Phe Phe Pro Asp His Val Glu Leu
                165                 170                 175

Ser Trp Trp Val Asn Gly Lys Glu Val His Ser Gly Val Cys Thr Asp
            180                 185                 190

Pro Gln Ala Tyr Lys Glu Ser Asn Tyr Ser Tyr Cys Leu Ser Ser Arg
        195                 200                 205

Leu Arg Val Ser Ala Thr Phe Trp His Asn Pro Arg Asn His Phe Arg
    210                 215                 220

Cys Gln Val Gln Phe His Gly Leu Ser Glu Glu Asp Lys Trp Pro Glu
225                 230                 235                 240

Gly Ser Pro Lys Pro Val Thr Gln Asn Ile Ser Ala Glu Ala Trp Gly
                245                 250                 255

Arg Ala Asp Cys Gly Ile Thr Ser Ala Ser Tyr His Gln Gly Val Leu
            260                 265                 270

Ser Ala Thr Ile Leu Tyr Glu Ile Leu Leu Gly Lys Ala Thr Leu Tyr
        275                 280                 285

Ala Val Leu Val Ser Gly Leu Val Leu Met Ala Met Val Lys Lys Lys
    290                 295                 300

Asn Ser Gly Ser Gly Ala Thr Asn Phe Ser Leu Leu Lys Gln Ala Gly
305                 310                 315                 320

Asp Val Glu Glu Asn Pro Gly Pro Met Thr Ser Ile Arg Ala Val Phe
                325                 330                 335

Ile Phe Leu Trp Leu Gln Leu Asp Leu Val Asn Gly Glu Asn Val Glu
            340                 345                 350

Gln His Pro Ser Thr Leu Ser Val Gln Glu Gly Asp Ser Ala Val Ile
        355                 360                 365

Lys Cys Thr Tyr Ser Asp Ser Ala Ser Asn Tyr Phe Pro Trp Tyr Lys
    370                 375                 380

Gln Glu Leu Gly Lys Arg Pro Gln Leu Ile Ile Asp Ile Arg Ser Asn

385                 390                 395                 400

Val Gly Glu Lys Lys Asp Gln Arg Ile Ala Val Thr Leu Asn Lys Thr
                405                 410                 415

Ala Lys His Phe Ser Leu His Ile Thr Glu Thr Gln Pro Glu Asp Ser
            420                 425                 430

Ala Val Tyr Phe Cys Ala Ala Ser Asn Gly Gln Gly Ala Gln Lys Leu
        435                 440                 445

Val Phe Gly Gln Gly Thr Arg Leu Thr Ile Asn Ile Gln Asn Pro Glu
    450                 455                 460

Pro Ala Val Tyr Gln Leu Lys Asp Pro Arg Ser Gln Asp Ser Thr Leu
465                 470                 475                 480

Cys Leu Phe Thr Asp Phe Asp Ser Gln Ile Asn Val Pro Lys Thr Met
                485                 490                 495

Glu Ser Gly Thr Phe Ile Thr Asp Lys Cys Val Leu Asp Met Lys Ala
            500                 505                 510

Met Asp Ser Lys Ser Asn Gly Ala Ile Ala Trp Ser Asn Gln Thr Ser
        515                 520                 525

Phe Thr Cys Gln Asp Ile Phe Lys Glu Thr Asn Ala Thr Tyr Pro Ser
    530                 535                 540

Ser Asp Val Pro Cys Asp Ala Thr Leu Thr Glu Lys Ser Phe Glu Thr
545                 550                 555                 560

Asp Met Asn Leu Asn Phe Gln Asn Leu Ser Val Met Gly Leu Arg Ile
                565                 570                 575

Leu Leu Leu Lys Val Ala Gly Phe Asn Leu Leu Met Thr Leu Arg Leu
            580                 585                 590

Trp Ser Ser
        595

<210>  31
<211>  399
<212>  DNA
<213>  Homo sapiens

<400>  31
atgaaatcct tgagagtttt actagtgatc ctgtggcttc agttgagctg ggtttggagc        60

caacagaagg aggtggagca gaattctgga cccctcagtg ttccagaggg agccattgcc 120

tctctcaact gcacttacag tgaccgaggt tcccagtcct tcttctggta cagacaatat 180

tctgggaaaa gccctgagtt gataatgttc atatactcca atggtgacaa agaagatgga 240

aggtttacag cacagctcaa taaagccagc cagtatgttt ctctgctcat cagagactcc 300

cagcccagtg attcagccac ctacctctgt gccgcgaaaa tgaaaacctc ctacgacaag 360

gtgatatttg ggccagggac aagcttatca gtcattcca 399

<210> 32
<211> 425
<212> DNA
<213> Homo sapiens

<400> 32
atgaaatcct tgagagtttt actagtgatc ctgtggcttc agttgagctg ggtttggagc 60

caacagaagg aggtggagca gaattctgga cccctcagtg ttccagaggg agccattgcc 120

tctctcaact gcacttacag tgaccgaggt tcccagtcct tcttctggta cagacaatat 180

tctgggaaaa gccctgagtt gataatgttc atatactcca atggtgacaa agaagatgga 240

aggtttacag cacagctcaa taaagccagc cagtatgttt ctctgctcat cagagactcc 300

cagcccagtg attcagccac ctacctctgt gccgggtatt caggaggagg tgctgacgga 360

ctcacctttg gcaaagggac tcatctaatc atccagcccg aagatctgag gaacgtgacc 420

ccccc 425

<210> 33
<211> 393
<212> DNA
<213> Homo sapiens

<400> 33
atgacatcca ttcgagctgt atttatattc ctgtggctgc agctggactt ggtgaatgga 60

gagaatgtgg agcagcatcc ttcaaccctg agtgtccagg agggagacag cgctgttatc 120

aagtgtactt attcagacag tgcctcaaac tacttccctt ggtataagca agaacttgga 180

aaaagacctc agcttattat agacattcgt tcaaatgtgg gcgaaaagaa agaccaacga 240

attgctgtta cattgaacaa gacagccaaa catttctccc tgcacatcac agagacccaa 300

cctgaagact cggctgtcta cttctgtgca gcaagtaatg ggcagggagc ccagaagctg 360

gtatttggcc aaggaaccag gctgactatc aac 393

<210> 34
<211> 399
<212> DNA
<213> Homo sapiens

<400> 34

atgggcttca ggctcctctg ctgtgtggcc ttttgtctcc tgggagcagg cccagtggat 60

tctggagtca cacaaacccc aaagcacctg atcacagcaa ctggacagcg agtgacgctg 120

agatgctccc ctaggtctgg agacctctct gtgtactggt accaacagag cctggaccag 180

ggcctccagt tcctcattca gtattataat ggagaagaga gagcaaaagg aaacattctt 240

gaacgattct ccgcacaaca gttccctgac ttgcactctg aactaaacct gagctctctg 300

gagctggggg actcagcttt gtatttctgt gccagcagcc ccggggggat cgggtacgag 360

cagtacttcg ggccgggcac caggctcacg gtcacagag 399

<210> 35
<211> 810
<212> DNA
<213> Artificial Sequence

<220>
<223> Alpha chain of TCR P2-1 (with variable alpha of SEQ ID NO. 31)

<400> 35
atgaaatcct tgagagtttt actagtgatc ctgtggcttc agttgagctg ggtttggagc 60

caacagaagg aggtggagca gaattctgga cccctcagtg ttccagaggg agccattgcc 120

tctctcaact gcacttacag tgaccgaggt tcccagtcct tcttctggta cagacaatat 180

tctgggaaaa gccctgagtt gataatgttc atatactcca atggtgacaa agaagatgga 240

aggtttacag cacagctcaa taaagccagc cagtatgttt ctctgctcat cagagactcc 300

cagcccagtg attcagccac ctacctctgt gccgcgaaaa tgaaaacctc ctacgacaag 360

gtgatatttg ggccagggac aagcttatca gtcattccaa tccagaaccc cgagcccgcc 420

gtgtaccagc tgaaggaccc cagaagccag gacagcaccc tgtgtctgtt caccgacttc 480

gacagccaga tcaacgtgcc caagaccatg gaaagcggca ccttcatcac cgataagtgc 540

gtgctggaca tgaaggccat ggacagcaag agcaacggcg ccattgcctg gtccaaccag 600

accagcttca catgccagga catcttcaaa gagacaaacg ccacctaccc ttccagcgac 660

gtgccctgtg acgccaccct gaccgagaag tccttcgaga cagacatgaa tctgaatttc 720

cagaacctga gcgtgatggg cctgcggatc ctgctgctga aagtggccgg cttcaacctg 780

ctgatgaccc tgcggctgtg gtccagctga 810

<210> 36
<211> 836
<212> DNA
<213> Artificial Sequence

<220>
<223> Alpha chain of TCR P2-1 (with variable alpha of SEQ ID NO. 32)

<400> 36
atgaaatcct tgagagtttt actagtgatc ctgtggcttc agttgagctg ggtttggagc 60

caacagaagg aggtggagca gaattctgga cccctcagtg ttccagaggg agccattgcc 120

tctctcaact gcacttacag tgaccgaggt tcccagtcct tcttctggta cagacaatat 180

tctgggaaaa gccctgagtt gataatgttc atatactcca atggtgacaa agaagatgga 240

aggtttacag cacagctcaa taaagccagc cagtatgttt ctctgctcat cagagactcc 300

cagcccagtg attcagccac ctacctctgt gccgggtatt caggaggagg tgctgacgga 360

ctcacctttg gcaaagggac tcatctaatc atccagcccg aagatctgag gaacgtgacc 420

cccccatcca gaaccccgag cccgccgtgt accagctgaa ggaccccaga agccaggaca 480

gcaccctgtg tctgttcacc gacttcgaca gccagatcaa cgtgcccaag accatggaaa 540

gcggcacctt catcaccgat aagtgcgtgc tggacatgaa ggccatggac agcaagagca 600

acggcgccat tgcctggtcc aaccagacca gcttcacatg ccaggacatc ttcaaagaga 660

caaacgccac ctacccttcc agcgacgtgc cctgtgacgc caccctgacc gagaagtcct 720

tcgagacaga catgaatctg aatttccaga acctgagcgt gatgggcctg cggatcctgc 780

tgctgaaagt ggccggcttc aacctgctga tgaccctgcg gctgtggtcc agctga 836

<210> 37
<211> 804
<212> DNA
<213> Artificial Sequence

<220>
<223> Alpha chain of TCR P2-1 (with variable alpha of SEQ ID NO. 33)

<400> 37
atgacatcca ttcgagctgt atttatattc ctgtggctgc agctggactt ggtgaatgga 60

gagaatgtgg agcagcatcc ttcaaccctg agtgtccagg agggagacag cgctgttatc 120

aagtgtactt attcagacag tgcctcaaac tacttccctt ggtataagca agaacttgga 180

aaaagacctc agcttattat agacattcgt tcaaatgtgg gcgaaaagaa agaccaacga 240

attgctgtta cattgaacaa gacagccaaa catttctccc tgcacatcac agagacccaa 300

cctgaagact cggctgtcta cttctgtgca gcaagtaatg ggcagggagc ccagaagctg 360

gtatttggcc aaggaaccag gctgactatc aacatccaga accccgagcc cgccgtgtac 420

cagctgaagg accccagaag ccaggacagc accctgtgtc tgttcaccga cttcgacagc 480

cagatcaacg tgcccaagac catggaaagc ggcaccttca tcaccgataa gtgcgtgctg 540

gacatgaagg ccatggacag caagagcaac ggcgccattg cctggtccaa ccagaccagc 600

ttcacatgcc aggacatctt caaagagaca aacgccacct acccttccag cgacgtgccc 660

tgtgacgcca ccctgaccga gaagtccttc gagacagaca tgaatctgaa tttccagaac 720

ctgagcgtga tgggcctgcg gatcctgctg ctgaaagtgg ccggcttcaa cctgctgatg 780

accctgcggc tgtggtccag ctga 804

<210> 38
<211> 918
<212> DNA
<213> Artificial Sequence

<220>
<223> Beta chain of TCR P2-1

<400> 38
atgggcttca ggctcctctg ctgtgtggcc ttttgtctcc tgggagcagg cccagtggat 60

tctggagtca cacaaacccc aaagcacctg atcacagcaa ctggacagcg agtgacgctg 120

agatgctccc ctaggtctgg agacctctct gtgtactggt accaacagag cctggaccag 180

ggcctccagt tcctcattca gtattataat ggagaagaga gagcaaaagg aaacattctt 240

gaacgattct ccgcacaaca gttccctgac ttgcactctg aactaaacct gagctctctg 300

gagctggggg actcagcttt gtatttctgt gccagcagcc ccggggggat cgggtacgag 360

cagtacttcg ggccgggcac caggctcacg gtcacagagg aagatctgag gaacgtgacc 420

ccccccaagg tgtccctgtt cgagcccagc aaggccgaga tcgccaacaa gcagaaagcc 480

accctcgtgt gcctggccag aggcttcttc cccgaccacg tggaactgtc ctggtgggtc 540

aacggcaaag aggtgcacag cggagtctgc accgaccccc aggcttacaa agagagcaac 600

tacagctact gcctgtccag cagactgcgg gtgtccgcta ccttctggca caacccccgg 660

aaccacttca gatgccaggt gcagttccac ggcctgagcg aagaggacaa gtggcccgag 720

ggcagcccca agcccgtgac ccagaatatc agcgccgagg cctggggcag agccgattgt 780

ggcatcacca gcgccagcta ccaccagggg gtgctgagcg ccaccatcct gtacgagatc 840

ctgctgggca aggccaccct gtacgccgtg ctggtgtccg gactggtgct gatggccatg 900

gtcaagaaga agaacagc 918

<210> 39
<211> 1794
<212> DNA
<213> Artificial Sequence

<220>
<223> TCR P2-1 (with variable alpha of SEQ ID NO. 31)

<400> 39
atgggcttca ggctcctctg ctgtgtggcc ttttgtctcc tgggagcagg cccagtggat 60

tctggagtca cacaaacccc aaagcacctg atcacagcaa ctggacagcg agtgacgctg 120

agatgctccc ctaggtctgg agacctctct gtgtactggt accaacagag cctggaccag 180

ggcctccagt tcctcattca gtattataat ggagaagaga gagcaaaagg aaacattctt 240

gaacgattct ccgcacaaca gttccctgac ttgcactctg aactaaacct gagctctctg 300

gagctggggg actcagcttt gtatttctgt gccagcagcc ccggggggat cgggtacgag 360

cagtacttcg ggccgggcac caggctcacg gtcacagagg aagatctgag gaacgtgacc 420

ccccccaagg tgtccctgtt cgagcccagc aaggccgaga tcgccaacaa gcagaaagcc 480

accctcgtgt gcctggccag aggcttcttc cccgaccacg tggaactgtc ctggtgggtc 540

aacggcaaag aggtgcacag cggagtctgc accgaccccc aggcttacaa agagagcaac 600

tacagctact gcctgtccag cagactgcgg gtgtccgcta ccttctggca caacccccgg 660

aaccacttca gatgccaggt gcagttccac ggcctgagcg aagaggacaa gtggcccgag 720

ggcagcccca agcccgtgac ccagaatatc agcgccgagg cctggggcag agccgattgt 780

ggcatcacca gcgccagcta ccaccagggg gtgctgagcg ccaccatcct gtacgagatc 840

ctgctgggca aggccaccct gtacgccgtg ctggtgtccg gactggtgct gatggccatg 900

gtcaagaaga agaacagcgg cagcggcgcc accaacttca gcctgctgaa gcaggccggc 960

gacgtggaag agaaccccgg gcccatgaaa tccttgagag ttttactagt gatcctgtgg 1020

cttcagttga gctgggtttg gagccaacag aaggaggtgg agcagaattc tggacccctc 1080

agtgttccag agggagccat tgcctctctc aactgcactt acagtgaccg aggttcccag 1140

tccttcttct ggtacagaca atattctggg aaaagccctg agttgataat gttcatatac 1200

tccaatggtg acaaagaaga tggaaggttt acagcacagc tcaataaagc cagccagtat 1260

gtttctctgc tcatcagaga ctcccagccc agtgattcag ccacctacct ctgtgccgcg 1320

aaaatgaaaa cctcctacga caaggtgata tttgggccag ggacaagctt atcagtcatt 1380

ccaatccaga accccgagcc cgccgtgtac cagctgaagg accccagaag ccaggacagc 1440

accctgtgtc tgttcaccga cttcgacagc cagatcaacg tgcccaagac catggaaagc 1500

ggcaccttca tcaccgataa gtgcgtgctg gacatgaagg ccatggacag caagagcaac 1560

ggcgccattg cctggtccaa ccagaccagc ttcacatgcc aggacatctt caaagagaca 1620

aacgccacct acccttccag cgacgtgccc tgtgacgcca ccctgaccga gaagtccttc 1680

gagacagaca tgaatctgaa tttccagaac ctgagcgtga tgggcctgcg gatcctgctg 1740

ctgaaagtgg ccggcttcaa cctgctgatg accctgcggc tgtggtccag ctga 1794

<210> 40
<211> 1820
<212> DNA
<213> Artificial Sequence

<220>
<223> TCR P2-1 (with variable alpha of SEQ ID NO. 32)

<400> 40
atgggcttca ggctcctctg ctgtgtggcc ttttgtctcc tgggagcagg cccagtggat 60

tctggagtca cacaaacccc aaagcacctg atcacagcaa ctggacagcg agtgacgctg 120

agatgctccc ctaggtctgg agacctctct gtgtactggt accaacagag cctggaccag 180

ggcctccagt tcctcattca gtattataat ggagaagaga gagcaaaagg aaacattctt 240

gaacgattct ccgcacaaca gttccctgac ttgcactctg aactaaacct gagctctctg 300

gagctggggg actcagcttt gtatttctgt gccagcagcc ccggggggat cgggtacgag 360

cagtacttcg ggccgggcac caggctcacg gtcacagagg aagatctgag gaacgtgacc 420

ccccccaagg tgtccctgtt cgagcccagc aaggccgaga tcgccaacaa gcagaaagcc 480

accctcgtgt gcctggccag aggcttcttc cccgaccacg tggaactgtc ctggtgggtc 540

aacggcaaag aggtgcacag cggagtctgc accgaccccc aggcttacaa agagagcaac 600

tacagctact gcctgtccag cagactgcgg gtgtccgcta ccttctggca caacccccgg 660

aaccacttca gatgccaggt gcagttccac ggcctgagcg aagaggacaa gtggcccgag 720

ggcagcccca agcccgtgac ccagaatatc agcgccgagg cctggggcag agccgattgt 780

ggcatcacca gcgccagcta ccaccagggg gtgctgagcg ccaccatcct gtacgagatc 840

ctgctgggca aggccaccct gtacgccgtg ctggtgtccg gactggtgct gatggccatg 900

gtcaagaaga agaacagcgg cagcggcgcc accaacttca gcctgctgaa gcaggccggc 960

gacgtggaag agaaccccgg gcccatgaaa tccttgagag ttttactagt gatcctgtgg 1020

cttcagttga gctgggtttg gagccaacag aaggaggtgg agcagaattc tggacccctc 1080

agtgttccag agggagccat tgcctctctc aactgcactt acagtgaccg aggttcccag 1140

tccttcttct ggtacagaca atattctggg aaaagccctg agttgataat gttcatatac 1200

tccaatggtg acaaagaaga tggaaggttt acagcacagc tcaataaagc cagccagtat 1260

gtttctctgc tcatcagaga ctcccagccc agtgattcag ccacctacct ctgtgccggg 1320

tattcaggag gaggtgctga cggactcacc tttggcaaag ggactcatct aatcatccag 1380

cccgaagatc tgaggaacgt gaccccccca tccagaaccc cgagcccgcc gtgtaccagc 1440

tgaaggaccc cagaagccag gacagcaccc tgtgtctgtt caccgacttc gacagccaga 1500

tcaacgtgcc caagaccatg gaaagcggca ccttcatcac cgataagtgc gtgctggaca 1560

tgaaggccat ggacagcaag agcaacggcg ccattgcctg gtccaaccag accagcttca 1620

catgccagga catcttcaaa gagacaaacg ccacctaccc ttccagcgac gtgccctgtg 1680

acgccaccct gaccgagaag tccttcgaga cagacatgaa tctgaatttc cagaacctga 1740

gcgtgatggg cctgcggatc ctgctgctga aagtggccgg cttcaacctg ctgatgaccc 1800

tgcggctgtg gtccagctga 1820

<210> 41
<211> 1788

<212>  DNA
<213>  Artificial Sequence

<220>
<223>  TCR P2-1 (with variable alpha of SEQ ID NO. 33)

<400>  41
atgggcttca ggctcctctg ctgtgtggcc ttttgtctcc tgggagcagg cccagtggat      60

tctggagtca cacaaacccc aaagcacctg atcacagcaa ctggacagcg agtgacgctg     120

agatgctccc ctaggtctgg agacctctct gtgtactggt accaacagag cctggaccag     180

ggcctccagt tcctcattca gtattataat ggagaagaga gagcaaaagg aaacattctt     240

gaacgattct ccgcacaaca gttccctgac ttgcactctg aactaaacct gagctctctg     300

gagctggggg actcagcttt gtatttctgt gccagcagcc ccggggggat cgggtacgag     360

cagtacttcg ggccgggcac caggctcacg gtcacagagg aagatctgag gaacgtgacc     420

ccccccaagg tgtccctgtt cgagcccagc aaggccgaga tcgccaacaa gcagaaagcc     480

accctcgtgt gcctggccag aggcttcttc cccgaccacg tggaactgtc ctggtgggtc     540

aacggcaaag aggtgcacag cggagtctgc accgaccccc aggcttacaa agagagcaac     600

tacagctact gcctgtccag cagactgcgg gtgtccgcta ccttctggca caacccccgg      660

aaccacttca gatgccaggt gcagttccac ggcctgagcg aagaggacaa gtggcccgag     720

ggcagcccca agcccgtgac ccagaatatc agcgccgagg cctggggcag agccgattgt     780

ggcatcacca gcgccagcta ccaccagggg gtgctgagcg ccaccatcct gtacgagatc     840

ctgctgggca aggccaccct gtacgccgtg ctggtgtccg gactggtgct gatggccatg     900

gtcaagaaga agaacagcgg cagcggcgcc accaacttca gcctgctgaa gcaggccggc     960

gacgtggaag agaaccccgg gcccatgaca tccattcgag ctgtatttat attcctgtgg    1020

ctgcagctgg acttggtgaa tggagagaat gtggagcagc atccttcaac cctgagtgtc    1080

caggagggag acagcgctgt tatcaagtgt acttattcag acagtgcctc aaactacttc    1140

ccttggtata agcaagaact tggaaaaaga cctcagctta ttatagacat tcgttcaaat    1200

gtgggcgaaa agaaagacca acgaattgct gttacattga acaagacagc caaacatttc    1260

tccctgcaca tcacagagac ccaacctgaa gactcggctg tctacttctg tgcagcaagt    1320

aatgggcagg gagcccagaa gctggtattt ggccaaggaa ccaggctgac tatcaacatc    1380

cagaaccccg agcccgccgt gtaccagctg aaggacccca gaagccagga cagcaccctg    1440

tgtctgttca ccgacttcga cagccagatc aacgtgccca agaccatgga aagcggcacc    1500

ttcatcaccg ataagtgcgt gctggacatg aaggccatgg acagcaagag caacggcgcc    1560

attgcctggt ccaaccagac cagcttcaca tgccaggaca tcttcaaaga gacaaacgcc    1620

acctaccctt ccagcgacgt gccctgtgac gccaccctga ccgagaagtc cttcgagaca    1680

gacatgaatc tgaatttcca gaacctgagc gtgatgggcc tgcggatcct gctgctgaaa     1740

gtggccggct tcaacctgct gatgaccctg cggctgtggt ccagctga                 1788

<210>  42
<211>  18
<212>  DNA
<213>  Artificial Sequence

<220>
<223>  Primer (with R = A or G; D = A, G or T)

<400>  42
tgagtgtccc rgarggdr                                                    18

<210>  43
<211>  22
<212>  DNA
<213>  Artificial Sequence

<220>
<223>  Primer

<400>  43
gtgacacatt tgtttgagaa tc                                               22

<210>  44
<211>  20
<212>  DNA
<213>  Artificial Sequence

<220>
<223>  Primer (with N = inosine, W = A/T, M = A/C, Y = C/T, K = G/T)

<220>
<221>  misc_feature
<222>  (3)..(4)
<223>  n is a, c, g, or t

<220>
<221>  misc_feature
<222>  (8)..(8)
<223>  n is a, c, g, or t

<400>  44
gcnntktnyt ggtaymgaca                                                  20

<210>  45
<211>  20
<212>  DNA
<213>  Artificial Sequence

<220>
<223>  Primer (with N = inosine, W = A/T, M = A/C, Y = C/T, K = G/T)

<220>
<221>  misc_feature

<222>  (3)..(3)
<223>  n is a, c, g, or t

<220>
<221>  misc_feature
<222>  (16)..(16)
<223>  n is a, c, g, or t

<400>  45
ctntktwttg gtaycnkcag                                                    20


<210>  46
<211>  19
<212>  DNA
<213>  Artificial Sequence

<220>
<223>  Primer

<400>  46
gcacctcctt cccattcac                                                     19

## Description

The present invention relates to glypican-3-specific T-cell receptors. The present invention further relates to soluble TCR constructs, chimeric TCRs, bi-specific antibodies, nucleic acids, expression constructs and cells comprising said TCRs or TCR constructs. The present invention further relates to the use of the TCR or the soluble TCR constructs or chimeric TCRs or bi-specific antibodies as a medicament, preferably in the detection, diagnosis, prognosis, prevention and/or treatment of liver cancer, in particular hepatocellular carcinoma, or other cancers expressing GPC3. The present invention further relates to methods of detecting, diagnosing, prognosing, preventing and/or treating liver cancer, in particular hepatocellular carcinoma, or other cancers expressing GPC3. The present invention further relates to peptides comprising glypican-3 epitope(s) and respective nucleic acids encoding them, antibodies and compositions as well as their use as (peptide) vaccines. The present invention further relates to vaccines comprising the peptide(s).

### BACKGROUND OF THE INVENTION

Hepatocellular carcinoma (HCC) is the most common primary liver cancer and the third most common cause of cancer related death worldwide. Patients chronically infected with hepatitis B or C are at high risk for development of HCC, but alcohol abuses and metabolic disorders can also lead to HCC development.

Less than 40% of patients fulfil the criteria for curative treatment like tumour resection or liver transplantation. Furthermore, the risk of tumour recurrence is high. Patients with advanced HCC can only undergo palliative treatment such as local ablation or the multikinase inhibitor sorafenib. Nevertheless these treatments result in a marginal beneficial effect on the expected lifetime of patients. As a result, the prognosis of HCC remains poor and new therapies are urgently needed.

WO 2012/070468 A1 discloses a glypican-3 (GPC3) specific antibody and a human chimeric antigen receptor (CAR) able to target GPC3. WO 2012/070468 A1 further discloses compositions and methods for diagnosing and treating diseases associated with dysregulated expression of GPC3, such as liver cancer, using said antibodies.

There is a need in the art for improved means and methods for detecting, diagnosing, prognosing, preventing and/or treating liver cancer, in particular hepatocellular carcinoma (HCC), such as via specific T cells or by immunotherapy or peptide-based vaccines.

### SUMMARY OF THE INVENTION

According to the present invention this object is solved by a T-cell receptor (TCR) comprising:
(i) a T cell receptor α-chain comprising the amino acid sequence of SEQ ID NO. 1 *(variable alpha)*
   or an amino acid sequence that has at least 80 % identity to the amino acid sequence of SEQ ID NO. 1, more preferably at least 85 % identity, more preferably 90 % or 95 % identity,
   or any variation of the TCR sequence provided that said variation retains its functional ability to bind to the epitope with the amino acid sequence of SEQ ID NO. 14 *(GPC3 peptide 367)* or to its HLA-A2 bound form
   and/or
(ii) a T-cell receptor β-chain comprising the amino acid sequence of SEQ ID NO. 2 *(variable beta)*
   or an amino acid sequence that has at least 80 % identity to the amino acid sequence of SEQ ID NO. 2, more preferably at least 85 % identity, more preferably 90 % or 95 % identity,
   or any variation of the TCR sequence provided that said variation retains its functional ability to bind to the epitope with the amino acid sequence of SEQ ID NO. 14 *(GPC3 peptide 367)* or to its HLA-A2 bound form.

According to the present invention this object is solved by a T-cell receptor (TCR) comprising:
(i) a T cell receptor α-chain comprising an amino acid sequence selected from the group of SEQ ID NOs. 18-20 *(3x variable alpha)*
   or an amino acid sequence that has at least 80 % identity to the amino acid sequence of SEQ ID NO. 1, more preferably at least 85 % identity, more preferably 90 % or 95 % identity,
   or any variation of the TCR sequence provided that said variation retains its functional ability to bind to the epitope with the amino acid sequence of SEQ ID NO. 17 *(GPC3 peptide 326)* or to its HLA-A2 bound form
   and/or
(ii) a T-cell receptor β-chain comprising the amino acid sequence of SEQ ID NO. 21 *(variable beta)*
   or an amino acid sequence that has at least 80 % identity to the amino acid sequence of SEQ ID NO. 2, more preferably at least 85 % identity, more preferably 90 % or 95 % identity,
   or any variation of the TCR sequence provided that said variation retains its functional ability to bind to the epitope with the amino acid sequence of SEQ ID NO. 17 *(GPC3 peptide 326)* or to its HLA-A2 bound form.

According to the present invention this object is solved by a soluble T-cell receptor (sTCR) construct comprising
(1) at least one of T-cell receptor α-chain(s) as defined herein, and/or T-cell receptor β-chain(s) as defined herein,
   preferably covalently linked to each other to form TCR heterodimers or multimers, and (2) fusion component(s)
   preferably selected from Fc receptors and/or Fc domains, cytokines, such as IL-2 or
   IL-15, toxins, antibodies, such as anti-CD3, anti-CD28, anti-CD5, anti-CD16 or anti-CD56 antibodies,, or combinations thereof,
   wherein the at least one T-cell receptor chain (1) is bound to the fusion component(s) (2),

According to the present invention this object is solved by a chimeric T-cell receptor comprising
at least one of
the T-cell receptor α-chain as defined herein,
the T-cell receptor β-chain as defined herein, or
the T-cell receptor according to the present invention,
wherein the TCR α-chain and/or the TCR β-chain is/are fused to CD3-zeta chain(s) and/or other TCR stimulation domains, such as the intracellular CD28, CD137 or CD134 domain, preferably via a linker.

According to the present invention this object is solved by a bi-specific antibody comprising
(a) the T-cell receptor α- and β-chain(s) of the TCR defined herein,
   which are linked with each other
   and fused, preferably via a linker, to
(b) an antibody or a single chain antibody fragment (scFv) which is directed against an antigen or epitope on the surface of lymphocytes.

According to the present invention this object is solved by a nucleic acid encoding the T-cell receptor according to the present invention or encoding the soluble T-cell receptor construct according to the present invention or encoding the chimeric T-cell receptor according to the present invention or encoding the bi-specific antibody according to the present invention.

According to the present invention this object is solved by an expression construct for expressing the T-cell receptor or soluble T-cell receptor construct or the chimeric T-cell receptor or the bi-specific antibody according to the present invention in a cell.

According to the present invention this object is solved by a cell comprising the T-cell receptor or soluble T-cell receptor construct or chimeric T-cell receptor or bi-specific antibody according to the present invention or the nucleic acid(s) according to the present invention or the expression construct according to the present invention.

According to the present invention this object is solved by a pharmaceutical composition comprising one or more of:
(i) the T-cell receptor of the present invention;
(ii) the soluble T-cell receptor construct of the present invention;
(iii) the chimeric T-cell receptor of the present invention;
(iv) the bi-specific antibody of the present invention;
(v) the nucleic acid(s) of the present invention or the expression construct of the present invention; and/or
(vi) the cell of the present invention,
   and, optionally, pharmaceutically excipient(s).

According to the present invention this object is solved by the use of a T-cell receptor of the present invention, a chimeric T-cell receptor of the present invention, a bi-specific antibody of the present invention, a nucleic acid of the present invention or an expression construct of the present invention for generating genetically modified lymphocytes.

According to the present invention this object is solved by providing the T-cell receptor of the present invention, the chimeric T-cell receptor of the present invention, the bi-specific antibody of the present invention, the nucleic acid of the present invention or the expression construct of the present invention for use as a medicament.

According to the present invention this object is solved by providing the T-cell receptor of the present invention, the chimeric T-cell receptor of the present invention, the bi-specific antibody of the present invention, the nucleic acid of the present invention or the expression construct of the present invention for use in the detection, diagnosis, prognosis, prevention and/or treatment of liver cancer, in particular hepatocellular carcinoma (HCC), or other cancers expressing GPC3.

According to the present invention this object is solved a method of preventing and/or treating liver cancer, in particular hepatocellular carcinoma (HCC), or other cancers expressing GPC3, comprising the steps of
(a) providing lymphocytes of a patient or a blood donor;
(b) providing one or more of
   (i) a T-cell receptor of the present invention or a soluble T-cell receptor construct of the present invention or a chimeric T-cell receptor of the present invention or a bi-specific antibody of the present invention,
   (ii) a nucleic acid of the present invention,
   (iii) an expression construct of the present invention,
   (iv) a cell of the present invention, and
   (v) a pharmaceutical composition of the present invention;
(c) *ex vivo* introduction of one or more of (i) to (v) of step (b) into the lymphocytes of step (a) and, thereby, obtaining genetically modified lymphocytes,
(d) administering the genetically modified lymphocytes of step (c) to a subject or patient in need thereof;
(e) optional, combining any of the steps (b) to (d) with radiation, checkpoint inhibitor or cancer chemotherapy.

According to the present invention this object is solved a method of preventing and/or treating liver cancer, in particular hepatocellular carcinoma (HCC), or other cancers expressing GPC3, comprising the steps of
(a) providing one or more of
   (i) a T-cell receptor of the present invention or a soluble T-cell receptor construct of the present invention or a chimeric T-cell receptor of the present invention or a bi-specific antibody of the present invention,
   (ii) a nucleic acid of the present invention,
   (iii) an expression construct of the present invention,
   (iv) a cell of the present invention, and
   (v) a pharmaceutical composition of the present invention;
(b) direct application, preferably via injection or infusion, of one or more of (i) to (v) of step (a) to a subject or patient in need thereof,
(c) optional, combining step (b) with radiation, checkpoint inhibitor or cancer chemotherapy.

According to the present invention this object is solved a method of detecting, diagnosing, prognosing, preventing and/or treating liver cancer, in particular hepatocellular carcinoma (HCC), or other cancers expressing GPC3,
comprising the detection and/or destruction of (liver) cancer cells, in particular hepatocellular carcinoma (HCC) cells, of a patient with the use of the soluble T-cell receptor construct of the present invention.

According to the present invention this object is solved by a peptide comprising at least one GPC3 epitope comprising the amino acid sequence of SEQ ID NO. 13 *(GPC3 peptide 367).*

According to the present invention this object is solved by a nucleic acid molecule coding for at least one peptide according to the present invention or a plasmid comprising at least one such nucleic acid molecule.

According to the present invention this object is solved by an antibody against a peptide according to the present invention.

According to the present invention this object is solved by a composition comprising
(i) at least one peptide according to the present invention or at least one nucleic acid according to the present invention,
(ii) optionally, a carrier,
(iii) optionally, an adjuvant.

According to the present invention this object is solved by providing the peptide of the present invention, the nucleic acid molecule of the present invention or the pharmaceutical composition of the present invention for use in medicine.

According to the present invention this object is solved by providing the peptide of the present invention, the nucleic acid molecule of the present invention or the pharmaceutical composition of the present invention for use as a vaccine.

According to the present invention this object is solved by a vaccine comprising
(i) at least one peptide according to the present invention or at least one nucleic acid according to the present invention or a composition of the present invention,
(ii) optionally, an excipient.

According to the present invention this object is solved by the use of the vaccine of the present invention for preventing and/or treating liver cancer, in particular hepatocellular carcinoma (HCC), or other cancers expressing GPC3.

According to the present invention this object is solved by a method of preventing and/or treating liver cancer, in particular hepatocellular carcinoma (HCC), or other cancers expressing GPC3,
comprising the step of administering a peptide of the present invention, a nucleic acid molecule of the present invention, a pharmaceutical composition of the present invention or a vaccine of the present invention to a subject in need thereof.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "5 to 50 amino acids" should be interpreted to include not only the explicitly recited values of 5 to 50, but also include individual values and sub-ranges within the indicated range. Thus, included in this numerical range are individual values such as 5, 6, 7, 8, 9, 10, 11, 12, 13, 14, 15, 16, 17, 18, 19, 20,... 48, 49, 50 and sub-ranges such as from 9 to 50, from5 to 25, from 9 to 25, from 10 to 25, from 10 to 20 and from 15 to 25, etc. This same principle applies to ranges reciting only one numerical value, such as "at least 8 amino acids". Furthermore, such an interpretation should apply regardless of the breadth of the range or the characteristics being described.

### GPC3-specific T-cell receptors

As discussed above, the present invention provides GPC3-specific T-cell receptors (TCRs).

A majority of T cells have a T cell receptor (TCR) existing as a complex of several proteins. The actual T cell receptor is composed of two (separate) peptide chains, which are produced from the independent T cell receptor alpha and beta (TCR α and TCR β) genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells.

The structure of the T cell receptor is very similar to immunoglobulin Fab fragments, which are regions defined as the combined light and heavy chain of an antibody arm. Each chain of the TCR is a member of the immunoglobulin superfamily and possesses one N-terminal immunoglobulin (Ig)-variable (V) domain, one Ig-constant (C) domain, a transmembrane/cell membrane-spanning region, and a short cytoplasmic tail at the C-terminal end.

According to the invention, the term "variable region of a T cell receptor" relates to the variable domains of the TCR chains. The variable domain of both the TCR α-chain and β-chain have three hypervariable or complementarity determining regions (CDRs), whereas the variable region of the β-chain has an additional area of hypervariability (HV4) that does not normally contact antigen and therefore is not considered a CDR.

The first signal in activation of T cells is provided by binding of the T cell receptor to a short peptide presented by the major histocompatibility complex (MHC) on another cell. This ensures that only a T cell with a TCR specific to that peptide is activated.

HCC expresses like many other tumours tumour-associated antigens. Glypican-3 (GPC3) and alpha-fetoprotein are not expressed in healthy human liver but reactivated in up to 70% and 80% of all HCCs, respectively.

### - TCR specific for GPC3 peptide 367

The inventors have now identified immunodominant GPC3 epitopes, namely the HLA-A2 bound GPC3 peptide 367, and cloned a TCR that is specific for GPC3 peptide 367.

Preferably, the GPC3-specific T-cell receptor (TCR) of the present invention
(1) comprises an alpha and a beta chain each comprising variable and constant domain(s),
(2) comprises codon-optimized variable domains for higher expression levels,
(3) is murinized, i.e. comprises murine constant domains, which helps to avoid miss pairing with endogenous TCR chains,
   and/or
(4) comprises a linker or hinge region to link the alpha and beta chain.

As discussed above, the GPC3-specificTCR of the present invention comprises:
(i) a T cell receptor α-chain comprising the amino acid sequence of SEQ ID NO. 1 *(variable alpha)*
   or an amino acid sequence that has at least 80 % identity to the amino acid sequence of SEQ ID NO. 1, more preferably at least 85 % identity, more preferably 90 % or 95 % identity,
   or any variation of the TCR sequence provided that said variation retains its functional ability to bind to the epitope with the amino acid sequence of SEQ ID NO. 14 *(GPC3 peptide* 367) or to its HLA-A2 bound form
   and/or
(ii) a T-cell receptor β-chain comprising the amino acid sequence of SEQ ID NO. 2 *(variable beta)*
   or an amino acid sequence that has at least 80 % identity to the amino acid sequence of SEQ ID NO. 2, more preferably at least 85 % identity, more preferably 90 % or 95 % identity,
   or any variation of the TCR sequence provided that said variation retains its functional ability to bind to the epitope with the amino acid sequence of SEQ ID NO. 14 *(GPC3 peptide 367)* or to its HLA-A2 bound form.

The terms "any variation of the TCR sequence provided that the biological activity is retained" or "any variation of the TCR sequence provided that said variation retains its functional ability to bind to the epitope with the amino acid sequence of SEQ ID NO. 14 *(GPC3 peptide 367)* or to its HLA-A2 bound form" as interchangeably used herein refers to amino acid sequences of the TCR chain(s)/receptor(s) of the present invention that retain their functional activity with regard to TCR recognition of the GPC3 peptide 367, preferably bound to the HLA A2-molecule.

In a preferred embodiment, the T-cell receptor (TCR) of the present invention comprises
(i) a T cell receptor α-chain further comprising the amino acid sequence of SEQ ID NO. 3 *(murine alpha)*
   or an amino acid sequence that has at least 80 % identity to the amino acid sequence of SEQ ID NO. 3, more preferably at least 85 % identity, more preferably 90 % or 95 % identity,
   and/or
(ii) a T-cell receptor β-chain further comprising the amino acid sequence of SEQ ID NO. *4 (murine beta)*
   or an amino acid sequence that has at least 80 % identity to the amino acid sequence of SEQ ID NO. 4, more preferably at least 85 % identity, more preferably 90 % or 95 % identity.

In a preferred embodiment, the T-cell receptor (TCR) of the present invention comprises
(iii) a linker to hinge region comprising the amino acid sequence of SEQ ID NO. 5 *(P2A)* or an amino acid sequence that has at least 80 % identity to the amino acid sequence of SEQ ID NO. 5.

In a preferred embodiment, the T-cell receptor (TCR) of the present invention comprises
(i) a T cell receptor α-chain comprising the amino acid sequence of SEQ ID NO. 1 *(variable alpha)* and SEQ ID NO. 3 *(murine alpha),*
   preferably comprising or consisting of the amino acid sequence of SEQ ID NO. 6 *(variable alpha + murine alpha)*
   or an amino acid sequence that has at least 80 % identity to the amino acid sequence of SEQ ID NO. 6, more preferably at least 85 % identity, more preferably 90 % or 95 % identity,
   or any variation of the TCR sequence provided that said variation retains its functional ability to bind to the epitope with the amino acid sequence of SEQ ID NO. 14 *(GPC3 peptide 367)* or to its HLA-A2 bound form
   and/or
(ii) a T-cell receptor β-chain comprising the amino acid sequence of SEQ ID NO. 2 *(variable beta)* and SEQ ID NO. 4 *(murine beta),*
   preferably comprising or consisting of the amino acid sequence of SEQ ID NO. 7 *(variable beta + murine beta)*
   or an amino acid sequence that has at least 80 % identity to the amino acid sequence of SEQ ID NO. 7, more preferably at least 85 % identity, more preferably 90 % or 95 % identity,
or any variation of the TCR sequence provided that said variation retains its functional ability to bind to the epitope with the amino acid sequence of SEQ ID NO. 14 *(GPC3 peptide 367)* or to its HLA-A2 bound form.

In a preferred embodiment, the T-cell receptor (TCR) of the present invention comprises or consists of the amino acid sequence of SEQ ID NO. 8 *(aa sequence of the entire TCR P1-1).*

SEQ ID NO. 1 shows the amino acid sequence of the variable region of the T-cell receptor alpha-chain *(Variable alpha)*

MLLELIPLLGIHFVLRTARAQSVTQPDIHITVSEGASLELRCNYSYGATPYLFWYVQSPGQG
LQLLLKYFSGDTLVQGIKGFEAEFKRSQSSFNLRKPSVHWSDAAEYFCAVGVLNFGNEKLTF
GTGTRLTIIP

SEQ ID NO. 2 shows the amino acid sequence of the variable region of the T-cell receptor beta-chain *(variable beta)*

MLCSLLALLLGTFFGVRSQTIHQWPATLVQPVGSPLSLECTVEGTSNPNLYWYRQAAGRGLQ
LLFYSVGIGQISSEVPQNLSASRPQDRQFILSSKKLLLSDSGFYLCAWSEDTAYYGYTFGSG
TRLTVVE

SEQ ID NO. 3 shows the amino acid sequence of the constant region of the T-cell receptor alpha-chain, which comprises murine constant domains *(murine alpha)*

IQNPEPAVYQLKDPRSQDSTLCLFTDFDSQINVPKTMESGTFITDKCVLDMKAMDSKSNGAI
AWSNQTSFTCQDIFKETNATYPSSDVPCDATLTEKSFETDMNLNFQNLSVMGLRILLLKVAG
FNLLMTLRLWSS

SEQ ID NO. 4 shows the amino acid sequence of the constant region of the T-cell receptor beta-chain, which comprises murine constant domains *(murine beta)*

EDLRNVTPPKVSLFEPSKAEIANKQKATLVCLARGFFPDHVELSWWVNGKEVHSGVCTDPQA
YKESNYSYCLSSRLRVSATFWHNPRNHFRCQVQFHGLSEEDKWPEGSPKPVTQNISAEAWGR
ADCGITSASYHQGVLSATILYEILLGKATLYAVLVSGLVLMAMVKKKNS

SEQ ID NO. 5 shows the amino acid sequence of the linker or hinge region which links the beta chain and alpha chain:
GSGATNFSLLKQAGDVEENPGP

SEQ ID NO. 6 shows the full length amino acid sequence of the T-cell receptor alpha-chain (wherein SEQ ID NO. 1 is underlined):

MLLELIPLLGIHFVLRTARAQSVTQPDIHITVSEGASLELRCNYSYGATPYLFWYVQSPGQG
LQLLLKYFSGDTLVQGIKGFEAEFKRSQSSFNLRKPSVHWSDAAEYFCAVGVLNFGNEKLTF
GTGTRLTIIPIQNPEPAVYQLKDPRSQDSTLCLFTDFDSQINVPKTMESGTFITDKCVLDMK
AMDSKSNGAIAWSNQTSFTCQDIFKETNATYPSSDVPCDATLTEKSFETDMNLNFQNLSVMG
LRILLLKVAGFNLLMTLRLWSS

SEQ ID NO. 7 shows the full length amino acid sequence of the T-cell receptor beta-chain (wherein SEQ ID NO. 2 is underlined):

MLCSLLALLLGTFFGVRSQTIHQWPATLVQPVGSPLSLECTVEGTSNPNLYWYRQAAGRGLQ
LLFYSVGIGQISSEVPQNLSASRPQDRQFILSSKKLLLSDSGFYLCAWSEDTAYYGYTFGSG
TRLTVVEEDLRNVTPPKVSLFEPSKAEIANKQKATLVCLARGFFPDHVELSWWVNGKEVHSG
VCTDPQAYKESNYSYCLSSRLRVSATFWHNPRNHFRCQVQFHGLSEEDKWPEGSPKPVTQNI
SAEAWGRADCGITSASYHQGVLSATILYEILLGKATLYAVLVSGLVLMAMVKKKNS

SEQ ID NO. 8 shows the full length amino acid sequence of the T-cell receptor, including beta chain, linker and alpha chain:

MLCSLLALLLGTFFGVRSQTIHQWPATLVQPVGSPLSLECTVEGTSNPNLYWYRQAAGRGLQ
LLFYSVGIGQISSEVPQNLSASRPQDRQFILSSKKLLLSDSGFYLCAWSEDTAYYGYTFGSG
TRLTVVEEDLRNVTPPKVSLFEPSKAEIANKQKATLVCLARGFFPDHVELSWWVNGKEVHSG
VCTDPQAYKESNYSYCLSSRLRVSATFWHNPRNHFRCQVQFHGLSEEDKWPEGSPKPVTQNI
SAEAWGRADCGITSASYHQGVLSATILYEILLGKATLYAVLVSGLVLMAMVKKKNSGSGATN
FSLLKQAGDVEENPGPMLLELIPLLGIHFVLRTARAQSVTQPDIHITVSEGASLELRCNYSY
GATPYLFWYVQSPGQGLQLLLKYFSGDTLVQGIKGFEAEFKRSQSSFNLRKPSVHWSDAAEY
FCAVGVLNFGNEKLTFGTGTRLTIIPIQNPEPAVYQLKDPRSQDSTLCLFTDFDSQINVPKT
MESGTFITDKCVLDMKAMDSKSNGAIAWSNQTSFTCQDIFKETNATYPSSDVPCDATLTEKS
FETDMNLNFQNLSVMGLRILLLKVAGFNLLMTLRLWSS*

### - TCR specific for GPC3 peptide 326

The inventors have furthermore cloned *a TCR that is specific for GPC3 peptide 326.*

Preferably, the GPC3-specific T-cell receptor (TCR) of the present invention
(1) comprises an alpha and a beta chain each comprising variable and constant domain(s),
(2) comprises codon-optimized variable domains for higher expression levels,
(3) is murinized, i.e. comprises murine constant domains, which helps to avoid miss pairing with endogenous TCR chains,
   and/or
(4) comprises a linker or hinge region to link the alpha and beta chain.

As discussed above, the GPC3-specificTCR of the present invention comprises:
(i) a T cell receptor α-chain comprising an amino acid sequence selected from the group of SEQ ID NOs. 18-20 *(3x variable alpha)*
   or an amino acid sequence that has at least 80 % identity to the amino acid sequence of SEQ ID NO. 1, more preferably at least 85 % identity, more preferably 90 % or 95 % identity,
   or any variation of the TCR sequence provided that said variation retains its functional ability to bind to the epitope with the amino acid sequence of SEQ ID NO. 17 *(GPC3 peptide* 326) or to its HLA-A2 bound form
   and/or
(ii) a T-cell receptor β-chain comprising the amino acid sequence of SEQ ID NO. 21 *(Variable beta)*
   or an amino acid sequence that has at least 80 % identity to the amino acid sequence of SEQ ID NO. 2, more preferably at least 85 % identity, more preferably 90 % or 95 % identity,
   or any variation of the TCR sequence provided that said variation retains its functional ability to bind to the epitope with the amino acid sequence of SEQ ID NO. 17 *(GPC3 peptide 326)* or to its HLA-A2 bound form.

The terms "any variation of the TCR sequence provided that the biological activity is retained" or "any variation of the TCR sequence provided that said variation retains its functional ability to bind to the epitope with the amino acid sequence of SEQ ID NO. 17 *(GPC3 peptide 326)* or to its HLA-A2 bound form" as interchangeably used herein refers to amino acid sequences of the TCR chain(s)/receptor(s) of the present invention that retain their functional activity with regard to TCR recognition of the GPC3 peptide 326, preferably bound to the HLA A2-molecule.

In a preferred embodiment, the T-cell receptor (TCR) of the present invention comprises
(i) a T cell receptor α-chain further comprising an amino acid sequence selected from the group of SEQ ID NOs. 22-24 *(3x murine alpha)*
   or an amino acid sequence that has at least 80 % identity to the amino acid sequence of SEQ ID NO. 3, more preferably at least 85 % identity, more preferably 90 % or 95 % identity,
   and/or
(ii) a T-cell receptor β-chain further comprising the amino acid sequence of SEQ ID NO. *25 (murine beta)*
   or an amino acid sequence that has at least 80 % identity to the amino acid sequence of SEQ ID NO. 4, more preferably at least 85 % identity, more preferably 90 % or 95 % identity.

In a preferred embodiment, the T-cell receptor (TCR) of the present invention comprises
(iii) a linker to hinge region comprising the amino acid sequence of SEQ ID NO. 5 *(P2A)* or an amino acid sequence that has at least 80 % identity to the amino acid sequence of SEQ ID NO. 5.

In a preferred embodiment, the T-cell receptor (TCR) of the present invention comprises
(i) a T cell receptor α-chain comprising an amino acid sequence selected from the group of
   (i-1) the amino acid sequence of SEQ ID NO. 18 *(variable alpha)* and SEQ ID NO. *22 (murine alpha),*
      preferably comprising or consisting of the amino acid sequence of SEQ ID NO. 26 *(Variable alpha + murine alpha)*
      or an amino acid sequence that has at least 80 % identity to the amino acid sequence of SEQ ID NO. 26, more preferably at least 85 % identity, more preferably 90 % or 95 % identity,
   (i-2) the amino acid sequence of SEQ ID NO. 19 *(variable alpha)* and SEQ ID NO. *23 (murine alpha),*
      preferably comprising or consisting of the amino acid sequence of SEQ ID NO. 27 *(variable alpha + marine alpha)*
      or an amino acid sequence that has at least 80 % identity to the amino acid sequence of SEQ ID NO. 27, more preferably at least 85 % identity, more preferably 90 % or 95 % identity,
   (i-3) the amino acid sequence of SEQ ID NO. 20 *(Variable alpha)* and SEQ ID NO. *24 (murine alpha),*
      preferably comprising or consisting of the amino acid sequence of SEQ ID NO. 28 *(variable alpha + murine alpha)*
      or an amino acid sequence that has at least 80 % identity to the amino acid sequence of SEQ ID NO. 28, more preferably at least 85 % identity, more preferably 90 % or 95 % identity,
   or any variation of the TCR sequence provided that said variation retains its functional ability to bind to the epitope with the amino acid sequence of SEQ ID NO. 17 *(GPC3 peptide 326)* or to its HLA-A2 bound form
   and/or
(ii) a T-cell receptor β-chain comprising the amino acid sequence of SEQ ID NO. 21 *(variable beta)* and SEQ ID NO. 25 *(murine beta),*
   preferably comprising or consisting of the amino acid sequence of SEQ ID NO. 29 *(variable beta + murine beta)*
   or an amino acid sequence that has at least 80 % identity to the amino acid sequence of SEQ ID NO. 29, more preferably at least 85 % identity, more preferably 90 % or 95 % identity,
   or any variation of the TCR sequence provided that said variation retains its functional ability to bind to the epitope with the amino acid sequence of SEQ ID NO. 17 *(GPC3 peptide 326)* or to its HLA-A2 bound form.

In a preferred embodiment, the T-cell receptor (TCR) of the present invention comprises or consists of an amino acid sequence selected from the group of SEQ ID NOs. 30-32 *(3x aa sequence of the entire TCR P2-1).*

SEQ ID NO. 18 shows the amino acid sequence of the variable region of one T-cell receptor alpha-chain *(Variable alpha)*

MKSLRVLLVILWLQLSWVWSQQKEVEQNSGPLSVPEGAIASLNCTYSDRGSQSFFWYRQYSG
KSPELIMFIYSNGDKEDGRFTAQLNKASQYVSLLIRDSQPSDSATYLCAAKMKTSYDKVIFG
PGTSLSVIP

SEQ ID NO. 19 shows the amino acid sequence of the variable region of another T-cell receptor alpha-chain *(variable alpha)*

MKSLRVLLVILWLQLSWVWSQQKEVEQNSGPLSVPEGAIASLNCTYSDRGSQSFFWYRQYSG
KSPELIMFIYSNGDKEDGRFTAQLNKASQYVSLLIRDSQPSDSATYLCAGYSGGGADGLTFG
KGTHLIIQPEDLRNVTPX

SEQ ID NO. 20 shows the amino acid sequence of the variable region of another T-cell receptor alpha-chain *(variable alpha)*

MTSIRAVFIFLWLQLDLVNGENVEQHPSTLSVQEGDSAVIKCTYSDSASNYFPWYKQELGKR
PQLIIDIRSNVGEKKDQRIAVTLNKTAKHFSLHITETQPEDSAVYFCAASNGQGAQKLVFGQ
GTRLTIN

SEQ ID NO. 21 shows the amino acid sequence of the variable region of the T-cell receptor beta-chain *(variable beta)*

MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSPGGIGYEQYFG
PGTRLTVTE

SEQ ID NO. 22 shows the amino acid sequence of the constant region of the T-cell receptor alpha-chain, which comprises murine constant domains *(murine alpha)*

IQNPEPAVYQLKDPRSQDSTLCLFTDFDSQINVPKTMESGTFITDKCVLDMKAMDSKSNGAI
AWSNQTSFTCQDIFKETNATYPSSDVPCDATLTEKSFETDMNLNFQNLSVMGLRILLLKVAG
FNLLMTLRLWSS

SEQ ID NO. 23 shows the amino acid sequence of the constant region of the T-cell receptor beta-chain, which comprises murine constant domains *(murine beta)*

EDLRNVTPPKVSLFEPSKAEIANKQKATLVCLARGFFPDHVELSWWVNGKEVHSGVCTDPQA
YKESNYSYCLSSRLRVSATFWHNPRNHFRCQVQFHGLSEEDKWPEGSPKPVTQNISAEAWGR
ADCGITSASYHQGVLSATILYEILLGKATLYAVLVSGLVLMAMVKKKNS

SEQ ID NO. 5 shows the amino acid sequence of the linker or hinge region which links the beta chain and alpha chain:
GSGATNFSLLKQAGDVEENPGP

SEQ ID NO. 24 shows the full length amino acid sequence of the T-cell receptor alpha-chain consisting of SEQ ID NO. 18 and 22 (wherein SEQ ID NO. 18 is underlined):

MKSLRVLLVILWLQLSWVWSQQKEVEQNSGPLSVPEGAIASLNCTYSDRGSQSFFWYRQYSG
KSPELIMFIYSNGDKEDGRFTAQLNKASQYVSLLIRDSQPSDSATYLCAAKMKTSYDKVIFG
PGTSLSVIPIQNPEPAVYQLKDPRSQDSTLCLFTDFDSQINVPKTMESGTFITDKCVLDMKA
MDSKSNGAIAWSNQTSFTCQDIFKETNATYPSSDVPCDATLTEKSFETDMNLNFQNLSVMGL
RILLLKVAGFNLLMTLRLWSS*

SEQ ID NO. 25 shows the full length amino acid sequence of the T-cell receptor alpha-chain consisting of SEQ ID NO. 19 and 22 (wherein SEQ ID NO. 19 is underlined):

MKSLRVLLVILWLQLSWVWSQQKEVEQNSGPLSVPEGAIASLNCTYSDRGSQSFFWYRQYSG
KSPELIMFIYSNGDKEDGRFTAQLNKASQYVSLLIRDSQPSDSATYLCAGYSGGGADGLTFG

KGTHLIIQPEDLRNVTPXIQNPEPAVYQLKDPRSQDSTLCLFTDFDSQINVPKTMESGTFIT
DKCVLDMKAMDSKSNGAIAWSNQTSFTCQDIFKETNATYPSSDVPCDATLTEKSFETDMNLN
FQNLSVMGLRILLLKVAGFNLLMTLRLWSS*

SEQ ID NO. 26 shows the full length amino acid sequence of the T-cell receptor alpha-chain consisting of SEQ ID NO. 20 and 22 (wherein SEQ ID NO. 20 is underlined):

MTSIRAVFIFLWLQLDLVNGENVEQHPSTLSVQEGDSAVIKCTYSDSASNYFPWYKQELGKR
PQLIIDIRSNVGEKKDQRIAVTLNKTAKHFSLHITETQPEDSAVYFCAASNGQGAQKLVFGQ
GTRLTINIQNPEPAVYQLKDPRSQDSTLCLFTDFDSQINVPKTMESGTFITDKCVLDMKAMD
SKSNGAIAWSNQTSFTCQDIFKETNATYPSSDVPCDATLTEKSFETDMNLNFQNLSVMGLRI
LLLKVAGFNLLMTLRLWSS*

SEQ ID NO. 27 shows the full length amino acid sequence of the T-cell receptor beta-chain (wherein SEQ ID NO. 21 is underlined):

MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSPGGIGYEQYFG
PGTRLTVTEEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYHQGVLSATILYEILLGKATLYAVLVSGLVLMAMVKKKNS

SEQ ID NO. 28 shows the full length amino acid sequence of one T-cell receptor, including beta chain, linker and the alpha chain of SEQ ID NO. 24:

MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSPGGIGYEQYFG
PGTRLTVTEEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYHQGVLSATILYEILLGKATLYAVLVSGLVLMAMVKKKNSGSGA
TNFSLLKQAGDVEENPGPMKSLRVLLVILWLQLSWVWSQQKEVEQNSGPLSVPEGAIASLNC
TYSDRGSQSFFWYRQYSGKSPELIMFIYSNGDKEDGRFTAQLNKASQYVSLLIRDSQPSDSA
TYLCAAKMKTSYDKVIFGPGTSLSVIPIQNPEPAVYQLKDPRSQDSTLCLFTDFDSQINVPK
TMESGTFITDKCVLDMKAMDSKSNGAIAWSNQTSFTCQDIFKETNATYPSSDVPCDATLTEK
SFETDMNLNFQNLSVMGLRILLLKVAGFNLLMTLRLWSS*

SEQ ID NO. 29 shows the full length amino acid sequence of another T-cell receptor, including beta chain, linker and the alpha chain of SEQ ID NO. 25:

MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSPGGIGYEQYFG
PGTRLTVTEEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYHQGVLSATILYEILLGKATLYAVLVSGLVLMAMVKKKNSGSGA
TNFSLLKQAGDVEENPGPMKSLRVLLVILWLQLSWVWSQQKEVEQNSGPLSVPEGAIASLNC
TYSDRGSQSFFWYRQYSGKSPELIMFIYSNGDKEDGRFTAQLNKASQYVSLLIRDSQPSDSA
TYLCAGYSGGGADGLTFGKGTHLIIQPEDLRNVTPXIQNPEPAVYQLKDPRSQDSTLCLFTD
FDSQINVPKTMESGTFITDKCVLDMKAMDSKSNGAIAWSNQTSFTCQDIFKETNATYPSSDV
PCDATLTEKSFETDMNLNFQNLSVMGLRILLLKVAGFNLLMTLRLWSS*

SEQ ID NO. 30 shows the full length amino acid sequence of another T-cell receptor, including beta chain, linker and the alpha chain of SEQ ID NO. 26:

MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSPGGIGYEQYFG
PGTRLTVTEEDLRNVTPPKVSLFEPSKAEIANKQKATLVCLARGFFPDHVELSWWVNGKEVH
SGVCTDPQAYKESNYSYCLSSRLRVSATFWHNPRNHFRCQVQFHGLSEEDKWPEGSPKPVTQ
NISAEAWGRADCGITSASYHQGVLSATILYEILLGKATLYAVLVSGLVLMAMVKKKNSGSGA
TNFSLLKQAGDVEENPGPMTSIRAVFIFLWLQLDLVNGENVEQHPSTLSVQEGDSAVIKCTY
SDSASNYFPWYKQELGKRPQLIIDIRSNVGEKKDQRIAVTLNKTAKHFSLHITETQPEDSAV
YFCAASNGQGAQKLVFGQGTRLTINIQNPEPAVYQLKDPRSQDSTLCLFTDFDSQINVPKTM
ESGTFITDKCVLDMKAMDSKSNGAIAWSNQTSFTCQDIFKETNATYPSSDVPCDATLTEKSF
ETDMNLNFQNLSVMGLRILLLKVAGFNLLMTLRLWSS*

### Soluble TCR (sTCR) constructs

As discussed above, the present invention provides soluble T-cell receptor (sTCR) constructs comprising the T-cell receptors/chains of the present invention.

Said soluble T-cell receptor (sTCR) constructs comprise
(1) at least one of
   - T-cell receptor α-chain(s) as defined herein,
   - T-cell receptor β-chain(s) as defined herein,
   that are preferably covalently linked to each other to form TCR heterodimers or multimers, and
(2) fusion component(s)
   preferably selected from
   Fc receptors and/or Fc domains,
   cytokines, such as IL-2 or IL-15,
   toxins, such as exotoxins, e.g. pseudomonas exotoxin,
   antibodies, such as anti-CD3, anti-CD28, anti-CD5, anti-CD16 or anti-CD56 antibodies,
   or combinations thereof,
   wherein the at least one T-cell receptor chain (1) is bound to the fusion component(s) (2),

Preferably, the soluble T-cell receptor comprises furthermore labels, such as radionuclides, gold (particles), fluorophors (such as fluorescein), which are preferably covalently attached/coupled to the soluble TCR, such as to the fusion component(s) (2).

The soluble TCRs can comprise different of the fusion components (2), such as cytokines and Fc domains.

TCR-alpha- and TCR-beta-chains can be solubilized by chemical modifications and linked covalently to each other and to other molecules such as Fc-receptors, cytokines and toxins (as described in Boulter et al, 2005; Lunde et al 2010).

These soluble TCR constructs are suitable to be directly used for the detection and destruction of liver cancer cells, such as HCC cells, without the need of T-cells. This can be achieved by application of soluble T-cell receptor constructs to patients by intravenous, subcutaneous or intramuscular infusion or injections, respectively, or by application to the mucosa of the respiratory tract by inhalation or by spraying.

In one embodiment, the soluble TCR constructs of the present invention are used as recombinant proteins.

Such recombinant proteins are expressed, preferably in E.coli or mammalian cells, and then purified before further use or application.

The present invention provides the expressed and purified soluble TCR constructs.

### Chimeric TCRs

As discussed above, the present invention provides chimeric T-cell receptors
comprising at least one of
the T-cell receptor α-chain as defined herein,
the T-cell receptor β-chain as defined herein, or
the T-cell receptor of the present invention,
wherein the TCR α-chain and/or the TCR β-chain is/are fused to CD3-zeta chain(s) and/or other TCR stimulation domains, such as the intracellular CD28, CD137 or CD134 domain, / more intracellular signaling domains of CD28, CD137 or CD134, preferably via a linker.

In one embodiment, a chimeric TCR of the invention comprises the T-cell receptor α-chain of the invention and the T-cell receptor β-chain of the invention, which are fused to each other via a linker and which are furthermore fused to CD3-zeta chain(s).

Preferred CD3-zeta chain(s) are the zeta-chain(s) of the human CD3 complex of the T-cell receptor. Preferred CD28, CD137 and CD 134 signalling domains are of human origin.

The chimeric TCRs can comprise further components.

Chimeric T-cell receptors and chimeric antigen receptors (CAR) are known in the art. See, for example, Han et al. 2013.

### Bi-specific antibodies,

As discussed above, the present invention provides bi-specific antibodies
comprising
(a) the T-cell receptor α- and β-chain(s) of the TCR defined herein,
   which are linked with each other
   and fused, preferably via a linker, to
(b) an antibody or a single chain antibody fragment (scFv) which is directed against an antigen or epitope on the surface of lymphocytes.

In one embodiment, the antibody or scFv is/are directed against the CD3 or CD5 receptor of T cells.

In this embodiment, the bi-specific antibody will bind to T cells and redirect them to GPC3-presenting tumor cells.

In another embodiment, the antibody or scFv is/are directed against the co-stimulatory CD28 receptor on T cells.

In this embodiment, the bi-specific antibody will bind to and co-activate T cells and direct them to GPC3-presenting tumor cells.

In another embodiment, antibodies or scFV against CD3 or CD5 and CD28 are combined in tetravalent antibody constructs.

In one embodiment, the antibody or scFv is/are directed against the CD56 receptor of NK cells.

In this embodiment, the bi-specific antibody will bind to NK cells and redirect them to GPC3-presenting tumor cells.

In another embodiment, the antibody or scFv is/are directed against the activating receptor CD 16 on NK cells.

In this embodiment, the bi-specific antibody will bind to and activate NK cells and direct them to GPC3-presenting tumor cells.

In another embodiment, antibodies or scFV against CD56 and CD16 are combined in tetravalent antibody constructs.

For example, a bi-specific antibody can be a bi-specific T-cell engager (BiTE) which is a class of artificial bispecific monoclonal antibodies that are investigated for the use as anticancer drugs. They direct a host's immune system, more specifically the T cells' cytotoxic activity, against cancer cells. *BiTE* is a registered trademark of Micromet AG.^{[}BiTEs are fusion proteins consisting of two single-chain variable fragments (scFvs) of different antibodies, or amino acid sequences from four different genes, on a single peptide chain of about 55 kilodaltons. One of the scFvs binds to T cells such as via the CD3 receptor or to CD56 for NK cell activation, and the other to a tumor cell via a tumor specific molecule (see e.g. Csoka et al., 1996).

Preferably, the bi-specific antibodies of the present invention are used as recombinant proteins.

Such recombinant proteins are expressed, preferably in E.coli or mammalian cells, and then purified before further use or application.

The present invention provides the expressed and purified bi-specific antibodies.

### Nucleic acids, expression constructs and cells comprising the GPC3-specific TCR

As discussed above, the present invention provides a nucleic acid encoding the T-cell receptor according to the present invention.

### - Nucleic acids for TCR specific for GPC3 peptide 367

Preferably, the nucleic acid comprises
the nucleic acid encoding for the amino acid sequence of SEQ ID NO. 1 *(variable alpha)* and/or SEQ ID NO. 2 *(variable beta)*
or the nucleotide sequence of SEQ ID NO. 9 *(Variable alpha)* and/or SEQ ID NO. 10 *(Variable beta)*
or their complementary sequence(s)
or sequence(s) that have at least 80 % identity to the nucleotide sequence of SEQ ID NO. 9 or 10, more preferably at least 85 % identity, more preferably 90 or 95 or 99 % identity.

SEQ ID NO. 9 shows the nucleotide sequence of the variable region of the T-cell receptor α-chain:

ATGCTGCTGGAACTGATCCCTCTGCTGGGCATCCACTTCGTGCTGAGAACCGCCAGAGCCCAGAGCGTGACCCAG

CCCGATATCCACATCACCGTGTCTGAGGGCGCCAGCCTGGAACTGCGGTGCAACTACAGCTACGGCGCCACCCCC

TACCTGTTTTGGTACGTGCAGAGCCCCGGACAGGGCCTGCAGCTGCTGCTGAAGTACTTTAGCGGCGACACCCTG

GTGCAGGGCATCAAGGGATTCGAGGCCGAGTTCAAGCGGAGCCAGAGCAGCTTCAACCTGCGGAAGCCCTCCGTG

CATTGGAGCGACGCCGCCGAGTACTTTTGTGCCGTGGGCGTGCTGAACTTCGGCAACGAGAAGCTGACCTTTGGC

ACCGGCACCCGGCTGACCATCATCCCT

SEQ ID NO. 10 shows the nucleotide sequence of the variable region of the T-cell receptor β-chain:

ATGCTGTGTTCTCTGCTGGCTCTGCTGCTGGGCACCTTCTTTGGAGTGCGGAGCCAGACCATCCACCAGTGGCCT
GCTACACTGGTGCAGCCTGTGGGCAGCCCTCTGAGCCTGGAATGTACCGTGGAAGGCACCAGCAACCCCAACCTG
TACTGGTACAGACAGGCCGCTGGCAGAGGCCTGCAGCTGCTGTTTTACAGCGTGGGCATCGGCCAGATCAGCAGC
GAGGTGCCCCAGAATCTGAGCGCCAGCAGACCCCAGGACCGGCAGTTTATCCTGAGCAGCAAGAAGCTGCTGCTG

AGCGACAGCGGCTTCTACCTGTGCGCTTGGAGCGAGGACACCGCCTACTACGGCTACACCTTTGGCAGCGGCACC
CGGCTGACAGTGGTGGAA

In one embodiment, the nucleic acid comprises
the nucleic acid encoding for the amino acid sequence of SEQ ID NO. 6 *(variable alpha + murine alpha)* and/or SEQ ID NO. 7 *(variable beta + murine beta)*
the nucleotide sequence of SEQ ID NO. 11 *(variable alpha + murine alpha)* and/or SEQ ID NO. 12 *(variable beta* + *murine beta)*
or their complementary sequence(s)
or sequence(s) that have at least 80 % identity to the nucleotide sequence of SEQ ID NO. 11 or 12, more preferably at least 85 % identity, more preferably 90 or 95 or 99 % identity.

SEQ ID NO. 11 shows the full length nucleotide sequence of the T-cell receptor alpha-chain (underlined is the variable region / SEQ ID NO. 9):

ATGCTGCTGGAACTGATCCCTCTGCTGGGCATCCACTTCGTGCTGAGAACCGCCAGAGCCCAGAGCGTGACCCAG
CCCGATATCCACATCACCGTGTCTGAGGGCGCCAGCCTGGAACTGCGGTGCAACTACAGCTACGGCGCCACCCCC
TACCTGTTTTGGTACGTGCAGAGCCCCGGACAGGGCCTGCAGCTGCTGCTGAAGTACTTTAGCGGCGACACCCTG
GTGCAGGGCATCAAGGGATTCGAGGCCGAGTTCAAGCGGAGCCAGAGCAGCTTCAACCTGCGGAAGCCCTCCGTG
CATTGGAGCGACGCCGCCGAGTACTTTTGTGCCGTGGGCGTGCTGAACTTCGGCAACGAGAAGCTGACCTTTGGC
ACCGGCACCCGGCTGACCATCATCCCTATCCAGAACCCCGAGCCCGCCGTGTACCAGCTGAAGGACCCCAGAAGC
CAGGACAGCACCCTGTGTCTGTTCACCGACTTCGACAGCCAGATCAACGTGCCCAAGACCATGGAAAGCGGCACC
TTCATCACCGATAAGTGCGTGCTGGACATGAAGGCCATGGACAGCAAGAGCAACGGCGCCATTGCCTGGTCCAAC
CAGACCAGCTTCACATGCCAGGACATCTTCAAAGAGACAAACGCCACCTACCCTTCCAGCGACGTGCCCTGTGAC
GCCACCCTGACCGAGAAGTCCTTCGAGACAGACATGAATCTGAATTTCCAGAACCTGAGCGTGATGGGCCTGCGG
ATCCTGCTGCTGAAAGTGGCCGGCTTCAACCTGCTGATGACCCTGCGGCTGTGGTCCAGCTGA

SEQ ID NO. 12 shows the full length nucleotide sequence of the T-cell receptor beta-chain (underlined is the variable region / SEQ ID NO. 10):

ATGCTGTGTTCTCTGCTGGCTCTGCTGCTGGGCACCTTCTTTGGAGTGCGGAGCCAGACCATCCACCAGTGGCCT
GCTACACTGGTGCAGCCTGTGGGCAGCCCTCTGAGCCTGGAATGTACCGTGGAAGGCACCAGCAACCCCAACCTG
TACTGGTACAGACAGGCCGCTGGCAGAGGCCTGCAGCTGCTGTTTTACAGCGTGGGCATCGGCCAGATCAGCAGC
GAGGTGCCCCAGAATCTGAGCGCCAGCAGACCCCAGGACCGGCAGTTTATCCTGAGCAGCAAGAAGCTGCTGCTG
AGCGACAGCGGCTTCTACCTGTGCGCTTGGAGCGAGGACACCGCCTACTACGGCTACACCTTTGGCAGCGGCACC
CGGCTGACAGTGGTGGAAGAAGATCTGAGGAACGTGACCCCCCCCAAGGTGTCCCTGTTCGAGCCCAGCAAGGCC
GAGATCGCCAACAAGCAGAAAGCCACCCTCGTGTGCCTGGCCAGAGGCTTCTTCCCCGACCACGTGGAACTGTCC
TGGTGGGTCAACGGCAAAGAGGTGCACAGCGGAGTCTGCACCGACCCCCAGGCTTACAAAGAGAGCAACTACAGC
TACTGCCTGTCCAGCAGACTGCGGGTGTCCGCTACCTTCTGGCACAACCCCCGGAACCACTTCAGATGCCAGGTG
CAGTTCCACGGCCTGAGCGAAGAGGACAAGTGGCCCGAGGGCAGCCCCAAGCCCGTGACCCAGAATATCAGCGCC
GAGGCCTGGGGCAGAGCCGATTGTGGCATCACCAGCGCCAGCTACCACCAGGGGGTGCTGAGCGCCACCATCCTG

TACGAGATCCTGCTGGGCAAGGCCACCCTGTACGCCGTGCTGGTGTCCGGACTGGTGCTGATGGCCATGGTCAAG
AAGAAGAACAGC

In one embodiment, the nucleic acid comprises
the nucleic acid encoding for the amino acid sequence of SEQ ID NO. 8 *(aa sequence of the entire TCR P1-1)*
the nucleotide sequence of SEQ ID NO. 13 (*entire TCR P1-1*)
or their complementary sequence(s)
or sequence(s) that have at least 80 % identity to the nucleotide sequence of SEQ ID NO. 13, more preferably at least 85 % identity, more preferably 90 or 95 or 99 % identity.

SEQ ID NO. 13 shows the nucleotide sequence of the T-cell receptor (underlined is the linker or hinge region):

ATGCTGTGTTCTCTGCTGGCTCTGCTGCTGGGCACCTTCTTTGGAGTGCGGAGCCAGACCATCCACCAGTGGCCT
GCTACACTGGTGCAGCCTGTGGGCAGCCCTCTGAGCCTGGAATGTACCGTGGAAGGCACCAGCAACCCCAACCTG
TACTGGTACAGACAGGCCGCTGGCAGAGGCCTGCAGCTGCTGTTTTACAGCGTGGGCATCGGCCAGATCAGCAGC
GAGGTGCCCCAGAATCTGAGCGCCAGCAGACCCCAGGACCGGCAGTTTATCCTGAGCAGCAAGAAGCTGCTGCTG
AGCGACAGCGGCTTCTACCTGTGCGCTTGGAGCGAGGACACCGCCTACTACGGCTACACCTTTGGCAGCGGCACC
CGGCTGACAGTGGTGGAAGAAGATCTGAGGAACGTGACCCCCCCCAAGGTGTCCCTGTTCGAGCCCAGCAAGGCC
GAGATCGCCAACAAGCAGAAAGCCACCCTCGTGTGCCTGGCCAGAGGCTTCTTCCCCGACCACGTGGAACTGTCC
TGGTGGGTCAACGGCAAAGAGGTGCACAGCGGAGTCTGCACCGACCCCCAGGCTTACAAAGAGAGCAACTACAGC
TACTGCCTGTCCAGCAGACTGCGGGTGTCCGCTACCTTCTGGCACAACCCCCGGAACCACTTCAGATGCCAGGTG
CAGTTCCACGGCCTGAGCGAAGAGGACAAGTGGCCCGAGGGCAGCCCCAAGCCCGTGACCCAGAATATCAGCGCC
GAGGCCTGGGGCAGAGCCGATTGTGGCATCACCAGCGCCAGCTACCACCAGGGGGTGCTGAGCGCCACCATCCTG
TACGAGATCCTGCTGGGCAAGGCCACCCTGTACGCCGTGCTGGTGTCCGGACTGGTGCTGATGGCCATGGTCAAG
AAGAAGAACAGCGGCAGCGGCGCCACCAACTTCAGCCTGCTGAAGCAGGCCGGCGACGTGGAAGAGAACCCCGGG
CCCATGCTGCTGGAACTGATCCCTCTGCTGGGCATCCACTTCGTGCTGAGAACCGCCAGAGCCCAGAGCGTGACC
CAGCCCGATATCCACATCACCGTGTCTGAGGGCGCCAGCCTGGAACTGCGGTGCAACTACAGCTACGGCGCCACC
CCCTACCTGTTTTGGTACGTGCAGAGCCCCGGACAGGGCCTGCAGCTGCTGCTGAAGTACTTTAGCGGCGACACC
CTGGTGCAGGGCATCAAGGGATTCGAGGCCGAGTTCAAGCGGAGCCAGAGCAGCTTCAACCTGCGGAAGCCCTCC
GTGCATTGGAGCGACGCCGCCGAGTACTTTTGTGCCGTGGGCGTGCTGAACTTCGGCAACGAGAAGCTGACCTTT
GGCACCGGCACCCGGCTGACCATCATCCCTATCCAGAACCCCGAGCCCGCCGTGTACCAGCTGAAGGACCCCAGA
AGCCAGGACAGCACCCTGTGTCTGTTCACCGACTTCGACAGCCAGATCAACGTGCCCAAGACCATGGAAAGCGGC
ACCTTCATCACCGATAAGTGCGTGCTGGACATGAAGGCCATGGACAGCAAGAGCAACGGCGCCATTGCCTGGTCC
AACCAGACCAGCTTCACATGCCAGGACATCTTCAAAGAGACAAACGCCACCTACCCTTCCAGCGACGTGCCCTGT
GACGCCACCCTGACCGAGAAGTCCTTCGAGACAGACATGAATCTGAATTTCCAGAACCTGAGCGTGATGGGCCTG
CGGATCCTGCTGCTGAAAGTGGCCGGCTTCAACCTGCTGATGACCCTGCGGCTGTGGTCCAGCTGA

The nucleotide sequences of SEQ ID NO. 9 to 13 are derived from cDNA or are DNA.

The nucleotide sequences of SEQ ID NO. 9 to 13 are codon-optimized.

### - Nucleic acids for TCR specific for GPC3 peptide 326

Preferably, the nucleic acid comprises
the nucleic acid encoding for the amino acid sequence of one of SEQ ID NOs. 18-20 *(3x Variable alpha)* and/or SEQ ID NO. 21 *(variable beta)*
or the nucleotide sequence of one of SEQ ID NOs. 31-33 *(3x variable alpha)* and/or SEQ ID NO. 34 *(variable beta)*
or their complementary sequence(s)
or sequence(s) that have at least 80 % identity to the nucleotide sequence of one of SEQ ID NOs. 31-34, more preferably at least 85 % identity, more preferably 90 or 95 or 99 % identity.

SEQ ID NO. 31 shows the nucleotide sequence of the variable region of one T-cell receptor α-chain:

ATGAAATCCTTGAGAGTTTTACTAGTGATCCTGTGGCTTCAGTTGAGCTGGGTTTGGAGCCAACAGAAGGAGGTG
GAGCAGAATTCTGGACCCCTCAGTGTTCCAGAGGGAGCCATTGCCTCTCTCAACTGCACTTACAGTGACCGAGGT
TCCCAGTCCTTCTTCTGGTACAGACAATATTCTGGGAAAAGCCCTGAGTTGATAATGTTCATATACTCCAATGGT
GACAAAGAAGATGGAAGGTTTACAGCACAGCTCAATAAAGCCAGCCAGTATGTTTCTCTGCTCATCAGAGACTCC
CAGCCCAGTGATTCAGCCACCTACCTCTGTGCCGCGAAAATGAAAACCTCCTACGACAAGGTGATATTTGGGCCA
GGGACAAGCTTATCAGTCATTCCA

SEQ ID NO. 32 shows the nucleotide sequence of the variable region of another T-cell receptor α-chain:

ATGAAATCCTTGAGAGTTTTACTAGTGATCCTGTGGCTTCAGTTGAGCTGGGTTTGGAGCCAACAGAAGGAGGTG
GAGCAGAATTCTGGACCCCTCAGTGTTCCAGAGGGAGCCATTGCCTCTCTCAACTGCACTTACAGTGACCGAGGT
TCCCAGTCCTTCTTCTGGTACAGACAATATTCTGGAAAAGCCCTGAGTTGATAATGTTCATATACTCCAATGGT
GACAAAGAAGATGGAAGGTTTACAGCACAGCTCAATAAAGCCAGCCAGTATGTTTCTCTGCTCATCAGAGACTCC
CAGCCCAGTGATTCAGCCACCTACCTCTGTGCCGGGTATTCAGGAGGAGGTGCTGACGGACTCACCTTTGGCAAA
GGGACTCATCTAATCATCCAGCCCGAAGATCTGAGGAACGTGACCCCCCC

SEQ ID NO. 33 shows the nucleotide sequence of the variable region of another T-cell receptor α-chain:

ATGACATCCATTCGAGCTGTATTTATATTCCTGTGGCTGCAGCTGGACTTGGTGAATGGAGAGAATGTGGAGCAG
CATCCTTCAACCCTGAGTGTCCAGGAGGGAGACAGCGCTGTTATCAAGTGTACTTATTCAGACAGTGCCTCAAAC
TACTTCCCTTGGTATAAGCAAGAACTTGGAAAAGACCTCAGCTTATTATAGACATTCGTTCAAATGTGGGCGAA
AAGAAAGACCAACGAATTGCTGTTACATTGAACAAGACAGCCAAACATTTCTCCCTGCACATCACAGAGACCCAA

CCTGAAGACTCGGCTGTCTACTTCTGTGCAGCAAGTAATGGGCAGGGAGCCCAGAAGCTGGTATTTGGCCAAGGA
ACCAGGCTGACTATCAAC

SEQ ID NO. 34 shows the nucleotide sequence of the variable region of the T-cell receptor β-chain:

ATGGGCTTCAGGCTCCTCTGCTGTGTGGCCTTTTGTCTCCTGGGAGCAGGCCCAGTGGATTCTGGAGTCACACAA
ACCCCAAAGCACCTGATCACAGCAACTGGACAGCGAGTGACGCTGAGATGCTCCCCTAGGTCTGGAGACCTCTCT
GTGTACTGGTACCAACAGAGCCTGGACCAGGGCCTCCAGTTCCTCATTCAGTATTATAATGGAGAAGAGAGAGCA
AAAGGAAACATTCTTGAACGATTCTCCGCACAACAGTTCCCTGACTTGCACTCTGAACTAAACCTGAGCTCTCTG
GAGCTGGGGGACTCAGCTTTGTATTTCTGTGCCAGCAGCCCCGGGGGGATCGGGTACGAGCAGTACTTCGGGCCG
GGCACCAGGCTCACGGTCACAGAG

In one embodiment, the nucleic acid comprises
the nucleic acid encoding for the amino acid sequence of one of SEQ ID NOs. 24-26 *(variable alpha + murine alpha)* and/or SEQ ID NO. 27 *(variable beta + murine beta)*
the nucleotide sequence of one of SEQ ID NOs. 35-37 *(variable alpha + murine alpha)* and/or SEQ ID NO. 38 *(variable beta + murine beta)*
or their complementary sequence(s)
or sequence(s) that have at least 80 % identity to the nucleotide sequence of one of SEQ ID NOs. 35-38, more preferably at least 85 % identity, more preferably 90 or 95 or 99 % identity.

SEQ ID NO. 35 shows the full length nucleotide sequence of one T-cell receptor alpha-chain (underlined is the variable region / SEQ ID NO. 31):

ATGAAATCCTTGAGAGTTTTACTAGTGATCCTGTGGCTTCAGTTGAGCTGGGTTTGGAGCCAACAGAAGGAGGTG
GAGCAGAATTCTGGACCCCTCAGTGTTCCAGAGGGAGCCATTGCCTCTCTCAACTGCACTTACAGTGACCGAGGT
TCCCAGTCCTTCTTCTGGTACAGACAATATTCTGGAAAAGCCCTGAGTTGATAATGTTCATATACTCCAATGGT
GACAAAGAAGATGGAAGGTTTACAGCACAGCTCAATAAAGCCAGCCAGTATGTTTCTCTGCTCATCAGAGACTCC
CAGCCCAGTGATTCAGCCACCTACCTCTGTGCCGCGAAAATGAAAACCTCCTACGACAAGGTGATATTTGGGCCA
GGGACAAGCTTATCAGTCATTCCAATCCAGAACCCCGAGCCCGCCGTGTACCAGCTGAAGGACCCCAGAAGCCAG
GACAGCACCCTGTGTCTGTTCACCGACTTCGACAGCCAGATCAACGTGCCCAAGACCATGGAAAGCGGCACCTTC
ATCACCGATAAGTGCGTGCTGGACATGAAGGCCATGGACAGCAAGAGCAACGGCGCCATTGCCTGGTCCAACCAG
ACCAGCTTCACATGCCAGGACATCTTCAAAGAGACAAACGCCACCTACCCTTCCAGCGACGTGCCCTGTGACGCC
ACCCTGACCGAGAAGTCCTTCGAGACAGACATGAATCTGAATTTCCAGAACCTGAGCGTGATGGGCCTGCGGATC
CTGCTGCTGAAAGTGGCCGGCTTCAACCTGCTGATGACCCTGCGGCTGTGGTCCAGCTGA

SEQ ID NO. 36 shows the full length nucleotide sequence of another T-cell receptor alpha-chain (underlined is the variable region / SEQ ID NO. 32):

ATGAAATCCTTGAGAGTTTTACTAGTGATCCTGTGGCTTCAGTTGAGCTGGGTTTGGAGCCAACAGAAGGAGGTG
GAGCAGAATTCTGGACCCCTCAGTGTTCCAGAGGGAGCCATTGCCTCTCTCAACTGCACTTACAGTGACCGAGGT
TCCCAGTCCTTCTTCTGGTACAGACAATATTCTGGAAAAGCCCTGAGTTGATAATGTTCATATACTCCAATGGT
GACAAAGAAGATGGAAGGTTTACAGCACAGCTCAATAAAGCCAGCCAGTATGTTTCTCTGCTCATCAGAGACTCC
CAGCCCAGTGATTCAGCCACCTACCTCTGTGCCGGGTATTCAGGAGGAGGTGCTGACGGACTCACCTTTGGCAAA
GGGACTCATCTAATCATCCAGCCCGAAGATCTGAGGAACGTGACCCCCCCATCCAGAACCCCGAGCCCGCCGTGT
ACCAGCTGAAGGACCCCAGAAGCCAGGACAGCACCCTGTGTCTGTTCACCGACTTCGACAGCCAGATCAACGTGC
CCAAGACCATGGAAAGCGGCACCTTCATCACCGATAAGTGCGTGCTGGACATGAAGGCCATGGACAGCAAGAGCA
ACGGCGCCATTGCCTGGTCCAACCAGACCAGCTTCACATGCCAGGACATCTTCAAAGAGACAAACGCCACCTACC
CTTCCAGCGACGTGCCCTGTGACGCCACCCTGACCGAGAAGTCCTTCGAGACAGACATGAATCTGAATTTCCAGA
ACCTGAGCGTGATGGGCCTGCGGATCCTGCTGCTGAAAGTGGCCGGCTTCAACCTGCTGATGACCCTGCGGCTGT
GGTCCAGCTGA

SEQ ID NO. 37 shows the full length nucleotide sequence of another T-cell receptor alpha-chain (underlined is the variable region / SEQ ID NO. 33):

ATGACATCCATTCGAGCTGTATTTATATTCCTGTGGCTGCAGCTGGACTTGGTGAATGGAGAGAATGTGGAGCAG
CATCCTTCAACCCTGAGTGTCCAGGAGGGAGACAGCGCTGTTATCAAGTGTACTTATTCAGACAGTGCCTCAAAC
TACTTCCCTTGGTATAAGCAAGAACTTGGAAAAAGACCTCAGCTTATTATAGACATTCGTTCAAATGTGGGCGAA
AAGAAAGACCAACGAATTGCTGTTACATTGAACAAGACAGCCAAACATTTCTCCCTGCACATCACAGAGACCCAA
CCTGAAGACTCGGCTGTCTACTTCTGTGCAGCAAGTAATGGGCAGGGAGCCCAGAAGCTGGTATTTGGCCAAGGA
ACCAGGCTGACTATCAACATCCAGAACCCCGAGCCCGCCGTGTACCAGCTGAAGGACCCCAGAAGCCAGGACAGC
ACCCTGTGTCTGTTCACCGACTTCGACAGCCAGATCAACGTGCCCAAGACCATGGAAAGCGGCACCTTCATCACC
GATAAGTGCGTGCTGGACATGAAGGCCATGGACAGCAAGAGCAACGGCGCCATTGCCTGGTCCAACCAGACCAGC
TTCACATGCCAGGACATCTTCAAAGAGACAAACGCCACCTACCCTTCCAGCGACGTGCCCTGTGACGCCACCCTG
ACCGAGAAGTCCTTCGAGACAGACATGAATCTGAATTTCCAGAACCTGAGCGTGATGGGCCTGCGGATCCTGCTG
CTGAAAGTGGCCGGCTTCAACCTGCTGATGACCCTGCGGCTGTGGTCCAGCTGA

SEQ ID NO. 38 shows the full length nucleotide sequence of the T-cell receptor beta-chain (underlined is the variable region / SEQ ID NO. 34):

ATGGGCTTCAGGCTCCTCTGCTGTGTGGCCTTTTGTCTCCTGGGAGCAGGCCCAGTGGATTCTGGAGTCACACAA
ACCCCAAAGCACCTGATCACAGCAACTGGACAGCGAGTGACGCTGAGATGCTCCCCTAGGTCTGGAGACCTCTCT
GTGTACTGGTACCAACAGAGCCTGGACCAGGGCCTCCAGTTCCTCATTCAGTATTATAATGGAGAAGAGAGAGCA
AAAGGAAACATTCTTGAACGATTCTCCGCACAACAGTTCCCTGACTTGCACTCTGAACTAAACCTGAGCTCTCTG
GAGCTGGGGGACTCAGCTTTGTATTTCTGTGCCAGCAGCCCCGGGGGATCGGGTACGAGCAGTACTTCGGGCCG
GGCACCAGGCTCACGGTCACAGAGGAAGATCTGAGGAACGTGACCCCCCCCAAGGTGTCCCTGTTCGAGCCCAGC
AAGGCCGAGATCGCCAACAAGCAGAAAGCCACCCTCGTGTGCCTGGCCAGAGGCTTCTTCCCCGACCACGTGGAA
CTGTCCTGGTGGGTCAACGGCAAAGAGGTGCACAGCGGAGTCTGCACCGACCCCCAGGCTTACAAAGAGAGCAAC
TACAGCTACTGCCTGTCCAGCAGACTGCGGGTGTCCGCTACCTTCTGGCACAACCCCCGGAACCACTTCAGATGC
CAGGTGCAGTTCCACGGCCTGAGCGAAGAGGACAAGTGGCCCGAGGGCAGCCCCAAGCCCGTGACCCAGAATATC
AGCGCCGAGGCCTGGGGCAGAGCCGATTGTGGCATCACCAGCGCCAGCTACCACCAGGGGGTGCTGAGCGCCACC

ATCCTGTACGAGATCCTGCTGGGCAAGGCCACCCTGTACGCCGTGCTGGTGTCCGGACTGGTGCTGATGGCCATG
GTCAAGAAGAAGAACAGC

In one embodiment, thee nucleic acid comprises
the nucleic acid encoding for the amino acid sequence of one of SEQ ID NOs. 28-30 *(3x aa sequence of the entire TCR P2-1)*
the nucleotide sequence of one of SEQ ID NOs. 39-41 *(entire TCR P2-1)*
or their complementary sequence(s)
or sequence(s) that have at least 80 % identity to the nucleotide sequence of one of SEQ ID NOs. 39-41, more preferably at least 85 % identity, more preferably 90 or 95 or 99 % identity.

SEQ ID NO. 39 shows the nucleotide sequence of one T-cell receptor (underlined is the linker or hinge region):

ATGGGCTTCAGGCTCCTCTGCTGTGTGGCCTTTTGTCTCCTGGGAGCAGGCCCAGTGGATTCTGGAGTCACACAA
ACCCCAAAGCACCTGATCACAGCAACTGGACAGCGAGTGACGCTGAGATGCTCCCCTAGGTCTGGAGACCTCTCT
GTGTACTGGTACCAACAGAGCCTGGACCAGGGCCTCCAGTTCCTCATTCAGTATTATAATGGAGAAGAGAGAGCA
AAAGGAAACATTCTTGAACGATTCTCCGCACAACAGTTCCCTGACTTGCACTCTGAACTAAACCTGAGCTCTCTG
GAGCTGGGGGACTCAGCTTTGTATTTCTGTGCCAGCAGCCCCGGGGGATCGGGTACGAGCAGTACTTCGGGCCG
GGCACCAGGCTCACGGTCACAGAGGAAGATCTGAGGAACGTGACCCCCCCCAAGGTGTCCCTGTTCGAGCCCAGC
AAGGCCGAGATCGCCAACAAGCAGAAAGCCACCCTCGTGTGCCTGGCCAGAGGCTTCTTCCCCGACCACGTGGAA
CTGTCCTGGTGGGTCAACGGCAAAGAGGTGCACAGCGGAGTCTGCACCGACCCCCAGGCTTACAAAGAGAGCAAC
TACAGCTACTGCCTGTCCAGCAGACTGCGGGTGTCCGCTACCTTCTGGCACAACCCCCGGAACCACTTCAGATGC
CAGGTGCAGTTCCACGGCCTGAGCGAAGAGGACAAGTGGCCCGAGGGCAGCCCCAAGCCCGTGACCCAGAATATC
AGCGCCGAGGCCTGGGGCAGAGCCGATTGTGGCATCACCAGCGCCAGCTACCACCAGGGGGTGCTGAGCGCCACC
ATCCTGTACGAGATCCTGCTGGGCAAGGCCACCCTGTACGCCGTGCTGGTGTCCGGACTGGTGCTGATGGCCATG
GTCAAGAAGAAGAACAGCGGCAGCGGCGCCACCAACTTCAGCCTGCTGAAGCAGGCCGGCGACGTGGAAGAGAAC
CCCGGCCCATGAAATCCTTGAGAGTTTTACTAGTGATCCTGTGGCTTCAGTTGAGCTGGGTTTGGAGCCAACAG
AAGGAGGTGGAGCAGAATTCTGGACCCCTCAGTGTTCCAGAGGGAGCCATTGCCTCTCTCAACTGCACTTACAGT
GACCGAGGTTCCCAGTCCTTCTTCTGGTACAGACAATATTCTGGGAAAAGCCCTGAGTTGATAATGTTCATATAC
TCCAATGGTGACAAAGAAGATGGAAGGTTTACAGCACAGCTCAATAAAGCCAGCCAGTATGTTTCTCTGCTCATC
AGAGACTCCCAGCCCAGTGATTCAGCCACCTACCTCTGTGCCGCGAAAATGAAAACCTCCTACGACAAGGTGATA
TTTGGGCCAGGGACAAGCTTATCAGTCATTCCAATCCAGAACCCCGAGCCCGCCGTGTACCAGCTGAAGGACCCC
AGAAGCCAGGACAGCACCCTGTGTCTGTTCACCGACTTCGACAGCCAGATCAACGTGCCCAAGACCATGGAAAGC
GGCACCTTCATCACCGATAAGTGCGTGCTGGACATGAAGGCCATGGACAGCAAGAGCAACGGCGCCATTGCCTGG
TCCAACCAGACCAGCTTCACATGCCAGGACATCTTCAAAGAGACAAACGCCACCTACCCTTCCAGCGACGTGCCC
TGTGACGCCACCCTGACCGAGAAGTCCTTCGAGACAGACATGAATCTGAATTTCCAGAACCTGAGCGTGATGGGC
CTGCGGATCCTGCTGCTGAAAGTGGCCGGCTTCAACCTGCTGATGACCCTGCGGCTGTGGTCCAGCTGA

SEQ ID NO. 40 shows the nucleotide sequence of another T-cell receptor (underlined is the linker or hinge region):

ATGGGCTTCAGGCTCCTCTGCTGTGTGGCCTTTTGTCTCCTGGGAGCAGGCCCAGTGGATTCTGGAGTCACACAA
ACCCCAAAGCACCTGATCACAGCAACTGGACAGCGAGTGACGCTGAGATGCTCCCCTAGGTCTGGAGACCTCTCT
GTGTACTGGTACCAACAGAGCCTGGACCAGGGCCTCCAGTTCCTCATTCAGTATTATAATGGAGAAGAGAGAGCA
AAAGGAAACATTCTTGAACGATTCTCCGCACAACAGTTCCCTGACTTGCACTCTGAACTAAACCTGAGCTCTCTG
GAGCTGGGGGACTCAGCTTTGTATTTCTGTGCCAGCAGCCCCGGGGGATCGGGTACGAGCAGTACTTCGGGCCG
GGCACCAGGCTCACGGTCACAGAGGAAGATCTGAGGAACGTGACCCCCCCAAGGTGTCCCTGTTCGAGCCCAGC
AAGGCCGAGATCGCCAACAAGCAGAAAGCCACCCTCGTGTGCCTGGCCAGAGGCTTCTTCCCCGACCACGTGGAA
CTGTCCTGGTGGGTCAACGGCAAAGAGGTGCACAGCGGAGTCTGCACCGACCCCCAGGCTTACAAAGAGAGCAAC
TACAGCTACTGCCTGTCCAGCAGACTGCGGGTGTCCGCTACCTTCTGGCACAACCCCCGGAACCACTTCAGATGC
CAGGTGCAGTTCCACGGCCTGAGCGAAGAGGACAAGTGGCCCGAGGGCAGCCCCAAGCCCGTGACCCAGAATATC
AGCGCCGAGGCCTGGGGCAGAGCCGATTGTGGCATCACCAGCGCCAGCTACCACCAGGGGGTGCTGAGCGCCACC
ATCCTGTACGAGATCCTGCTGGGCAAGGCCACCCTGTACGCCGTGCTGGTGTCCGGACTGGTGCTGATGGCCATG
GTCAAGAAGAAGAACAGCGGCAGCGGCGCCACCAACTTCAGCCTGCTGAAGCAGGCCGGCGACGTGGAAGAGAAC
CCCGGCCCATGAAATCCTTGAGAGTTTTACTAGTGATCCTGTGGCTTCAGTTGAGCTGGGTTTGGAGCCAACAG
AAGGAGGTGGAGCAGAATTCTGGACCCCTCAGTGTTCCAGAGGGAGCCATTGCCTCTCTCAACTGCACTTACAGT
GACCGAGGTTCCCAGTCCTTCTTCTGGTACAGACAATATTCTGGGAAAAGCCCTGAGTTGATAATGTTCATATAC
TCCAATGGTGACAAAGAAGATGGAAGGTTTACAGCACAGCTCAATAAAGCCAGCCAGTATGTTTCTCTGCTCATC
AGAGACTCCCAGCCCAGTGATTCAGCCACCTACCTCTGTGCCGGGTATTCAGGAGGAGGTGCTGACGGACTCACC
TTTGGCAAAGGGACTCATCTAATCATCCAGCCCGAAGATCTGAGGAACGTGACCCCCCCATCCAGAACCCCGAGC
CCGCCGTGTACCAGCTGAAGGACCCCAGAAGCCAGGACAGCACCCTGTGTCTGTTCACCGACTTCGACAGCCAGA
TCAACGTGCCCAAGACCATGGAAAGCGGCACCTTCATCACCGATAAGTGCGTGCTGGACATGAAGGCCATGGACA
GCAAGAGCAACGGCGCCATTGCCTGGTCCAACCAGACCAGCTTCACATGCCAGGACATCTTCAAAGAGACAAACG
CCACCTACCCTTCCAGCGACGTGCCCTGTGACGCCACCCTGACCGAGAAGTCCTTCGAGACAGACATGAATCTGA
ATTTCCAGAACCTGAGCGTGATGGGCCTGCGGATCCTGCTGCTGAAAGTGGCCGGCTTCAACCTGCTGATGACCC
TGCGGCTGTGGTCCAGCTGA

SEQ ID NO. 41 shows the nucleotide sequence of another T-cell receptor (underlined is the linker or hinge region):

ATGGGCTTCAGGCTCCTCTGCTGTGTGGCCTTTTGTCTCCTGGGAGCAGGCCCAGTGGATTCTGGAGTCACACAA
ACCCCAAAGCACCTGATCACAGCAACTGGACAGCGAGTGACGCTGAGATGCTCCCCTAGGTCTGGAGACCTCTCT
GTGTACTGGTACCAACAGAGCCTGGACCAGGGCCTCCAGTTCCTCATTCAGTATTATAATGGAGAAGAGAGAGCA
AAAGGAAACATTCTTGAACGATTCTCCGCACAACAGTTCCCTGACTTGCACTCTGAACTAAACCTGAGCTCTCTG
GAGCTGGGGGACTCAGCTTTGTATTTCTGTGCCAGCAGCCCCGGGGGATCGGGTACGAGCAGTACTTCGGGCCG
GGCACCAGGCTCACGGTCACAGAGGAAGATCTGAGGAACGTGACCCCCCCCAAGGTGTCCCTGTTCGAGCCCAGC
AAGGCCGAGATCGCCAACAAGCAGAAAGCCACCCTCGTGTGCCTGGCCAGAGGCTTCTTCCCCGACCACGTGGAA
CTGTCCTGGTGGGTCAACGGCAAAGAGGTGCACAGCGGAGTCTGCACCGACCCCCAGGCTTACAAAGAGAGCAAC
TACAGCTACTGCCTGTCCAGCAGACTGCGGGTGTCCGCTACCTTCTGGCACAACCCCCGGAACCACTTCAGATGC
CAGGTGCAGTTCCACGGCCTGAGCGAAGAGGACAAGTGGCCCGAGGGCAGCCCCAAGCCCGTGACCCAGAATATC
AGCGCCGAGGCCTGGGGCAGAGCCGATTGTGGCATCACCAGCGCCAGCTACCACCAGGGGGTGCTGAGCGCCACC

ATCCTGTACGAGATCCTGCTGGGCAAGGCCACCCTGTACGCCGTGCTGGTGTCCGGACTGGTGCTGATGGCCATG
GTCAAGAAGAAGAACAGCGGCAGCGGCGCCACCAACTTCAGCCTGCTGAAGCAGGCCGGCGACGTGGAAGAGAAC
CCCGGCCCATGACATCCATTCGAGCTGTATTTATATTCCTGTGGCTGCAGCTGGACTTGGTGAATGGAGAGAAT
GTGGAGCAGCATCCTTCAACCCTGAGTGTCCAGGAGGGAGACAGCGCTGTTATCAAGTGTACTTATTCAGACAGT
GCCTCAAACTACTTCCCTTGGTATAAGCAAGAACTTGGAAAAGACCTCAGCTTATTATAGACATTCGTTCAAAT
GTGGGCGAAAAGAAAGACCAACGAATTGCTGTTACATTGAACAAGACAGCCAAACATTTCTCCCTGCACATCACA
GAGACCCAACCTGAAGACTCGGCTGTCTACTTCTGTGCAGCAAGTAATGGGCAGGGAGCCCAGAAGCTGGTATTT
GGCCAAGGAACCAGGCTGACTATCAACATCCAGAACCCCGAGCCCGCCGTGTACCAGCTGAAGGACCCCAGAAGC
CAGGACAGCACCCTGTGTCTGTTCACCGACTTCGACAGCCAGATCAACGTGCCCAAGACCATGGAAAGCGGCACC
TTCATCACCGATAAGTGCGTGCTGGACATGAAGGCCATGGACAGCAAGAGCAACGGCGCCATTGCCTGGTCCAAC
CAGACCAGCTTCACATGCCAGGACATCTTCAAAGAGACAAACGCCACCTACCCTTCCAGCGACGTGCCCTGTGAC
GCCACCCTGACCGAGAAGTCCTTCGAGACAGACATGAATCTGAATTTCCAGAACCTGAGCGTGATGGGCCTGCGG
ATCCTGCTGCTGAAAGTGGCCGGCTTCAACCTGCTGATGACCCTGCGGCTGTGGTCCAGCTGA

The nucleotide sequences of SEQ ID NO. 31 to 41 are derived from cDNA or are DNA.

As discussed above, the present invention provides a nucleic acid encoding the soluble T-cell receptor construct according to the present invention.

As discussed above, the present invention provides a nucleic acid encoding the chimeric T-cell receptor according to the present invention.

As discussed above, the present invention provides a nucleic acid encoding the bi-specific antibody according to the present invention.

The nucleic acids according to this invention comprise DNA (such as dsDNA, ssDNA, cDNA), RNA (such as dsRNA, ssRNA, mRNA), combinations thereof or derivatives (such as PNA) thereof.

Preferably, the nucleic acid sequences of the present invention are codon-optimized for expression in mammalian cells, preferably for expression in human cells. For generation of bi-specific antibodies the nucleic acid sequences can also be codon-optimized for expression in bacteria, yeast or insect cells. Codon-optimization refers to the exchange in a sequence of interest of codons that are generally rare in highly expressed genes of a given species by codons that are generally frequent in highly expressed genes of such species, such codons encoding the same amino acids as the codons that are being exchanged.

Within the scope of this invention are also the nucleotide sequences obtained due to the degeneration of the genetic code of the above nucleotide sequences.

As discussed above, the present invention provides an expression construct for expressing the T-cell receptor according to the present invention in a cell.

As discussed above, the present invention provides an expression construct for expressing the soluble T-cell receptor construct according to the present invention in a cell.

As discussed above, the present invention provides an expression construct for expressing the chimeric T-cell receptor according to the present invention in a cell.

As discussed above, the present invention provides an expression construct for expressing the bi-specific antibody according to the present invention as well as the expressed and purified bi-specific antibody.

Preferably, the expression constructs further comprise promoter and terminator sequences.

An "expression or gene construct" (wherein both terms are used interchangeably throughout this specification) refers to a nucleic acid construct, usually an expression vector or plasmid that is used to introduce a specific gene sequence into a target cell. Once the expression or gene construct is inside the cell, the protein that is encoded by the gene is produced by the cellular transcription and translation machinery. The expression or gene construct is designed to contain respective regulatory sequences that act as enhancer and promoter regions and lead to efficient transcription of the gene carried on the construct, including promoter and terminator sequences. The goal of a well-designed expression or gene construct is the production of large amounts of stable mRNA, and therefore proteins. Alternatively, an RNA is synthesized or *in vitro* transcribed and introduced directly into the cells for protein expression.

The skilled artisan can select further suitable components of expression or gene constructs.

Examples for suitable expression constructs are viral vectors, such as
- retroviral vectors (as described in Verra et al, 2004; Porter et al, 2011), such as MP71 vectors (as described in Engels et al., 2003), or retroviral SIN vectors (as described in Yu et al., 1986);
   and
- lentiviral vectors (as described in Josef et al, 2008) or lentiviral SIN vectors, as described in Miyoshi et al., 1998.
   Viral vectors containing TCR-encoding genes can infect lymphocytes and express functional TCR in infected lymphocytes. Preferred is a SIN vector construct.

Another example for a suitable expression construct is the *Sleeping Beauty* (SB) transposon transposase DNA plasmid system, SB DNA plasmid (as described in Maiti et al., 2013).

The nucleic acids and/or in particular expression constructs of the invention can also be transferred into cells by transient RNA transfection.

The nucleic acids and/or in particular expression constructs of the invention are capable of directing the synthesis/expression of the TCRs of the invention in a suitable host cell.

As discussed above, the present invention provides a cell comprising the T-cell receptor according to the present invention or the soluble T-cell receptor construct according to the present invention or the chimeric T-cell receptor of the present invention or the bi-specific antibody of the present invention or the nucleic acid(s) of the present invention or the expression construct of the present invention.

Preferably, the cell expresses the T cell-receptor of the present invention.

Preferably, the cell expresses the soluble T cell-receptor construct of the present invention.

Preferably, the cell expresses the chimeric T-cell receptor according to the present invention.

Preferably, the cell expresses the bi-specific antibody according to the present invention.

Preferably, the cell is selected from lymphocytes including but not limited to cytotoxic lymphocytes (CTLs), CD8+ T cells, CD4+ T cells, natural killer (NK) cells, natural killer T (NKT) cells, gamma/delta-T-cells.

Preferably the cell for expression of soluble T-cell receptors or bi-specific antibodies is selected from E. coli, insect cells or mammalian calls such as HEK293, which express high amounts of recombinant protein.

The transfer of GPC3-specific TCR genes into (primary) T cells by e.g. (retro-)viral vectors, SB DNA or by transient RNA transfection represents a promising means and method to generate GPC3 antigen-specific T cells. Adoptive transfer of such engineered T cells into hosts represents a promising approach for the immunotherapy of liver cancer, in particular HCC, or other cancers expressing GPC3.

The application of GPC3-specific bi-specific antibodies or soluble T-cell receptors represents an alternative promising means and method to target GPC3-positive tumor cells by redirected T cells or by the soluble T-cell receptor construct. This represents a promising approach for the immunotherapy of liver cancer, in particular HCC, or other cancers expressing GPC3.

### Pharmaceutical compositions

As discussed above, the present invention provides a pharmaceutical composition comprising one or more of:
(i) the T-cell receptor of the present invention;
(ii) the soluble T-cell receptor construct of the present invention;
(iii) the chimeric T-cell receptor of the present invention;
(iv) the bi-specific antibody of the present invention;
(v) the nucleic acid(s) of any one of claims 7 to 10 or the expression construct of the present invention; and/or
(vi) the cell the present invention,
and, optionally, pharmaceutically excipient(s).

### Medical uses of the GPC3-specific TCR

As discussed above, the present invention provides the use of a T-cell receptor of the present invention, a chimeric T-cell receptor of the present invention, a bi-specific antibody of the present invention, a nucleic acid of the present invention or an expression construct of the present invention for generating genetically modified lymphocytes.

Preferably the genetically modified lymphocytes are T cells, NK cells and/or NKT cells.

In one embodiment, the generated genetically modified lymphocytes, preferably T cells and/or NK cells, are used for genetic immunization, preferably for preventing and/or treating liver cancer, in particular HCC, or other cancers expressing GPC3.

The transfer of GPC3-specific TCR genes into (primary) T cells by e.g. (retro-)viral vectors or by transient RNA transfection represents a promising means and method to generate GPC3 antigen-specific T cells. Adoptive transfer of such engineered T cells into hosts represents a promising approach for the immunotherapy of liver cancer, in particular hepatocellular carcinoma (HCC), or other cancers expressing GPC3. A new therapeutic approach is the adoptive T cell therapy of HCC. The transferred T cells can recognize tumour antigen and thereby specifically eliminate tumour cells. HCC is a potential target for T cell therapy because HCC expresses like many other tumours tumour-associated antigens, such as glypican-3 (GPC3).

In one embodiment, the chimeric T-cell receptor(s) of the present invention are used to generate genetically modified lymphocytes, preferably NK cells and/or T cells, such as CD8⁺ T cells.

The transfer of chimeric receptors containing the whole or parts of the GPC3-specific TCR genes linked to the CD3zeta chain into primary NK cells by e.g. (retro-)viral vectors or by transient RNA transfection represents a promising means and method to generate GPC3 antigen-specific NK cells. Adoptive transfer of such engineered NK cells into hosts represents a promising approach for the immunotherapy of liver cancer, in particular hepatocellular carcinoma (HCC), or other cancers expressing GPC3.

As discussed above, the present invention provides the T-cell receptor of the present invention, the soluble T-cell receptor construct of the present invention, the chimeric T-cell receptor of the present invention, the bi-specific antibody of the present invention, the nucleic acid of the present invention, the expression construct of the present invention or the host cell of the present invention for use as a medicament.

As discussed above, the present invention provides the T-cell receptor of the present invention, the soluble T-cell receptor construct of the present invention, the chimeric T-cell receptor of the present invention, the bi-specific antibody of the present invention, the nucleic acid of the present invention, the expression construct of the present invention or the host cell of the present invention for use in the detection, diagnosis, prognosis, prevention and/or treatment of liver cancer, in particular hepatocellular carcinoma (HCC), or other cancers expressing GPC3.

There are others cancers or tumors expressing GPC3, such as described by Baumhoer et al., 2008, exemplified but not restricted to:
- squamous cell carcinoma of the lung,
- testicular nonseminomatous germ cell tumors,
- liposarcoma,
- cervical intraepithelial neoplasia.

Preferably, the use comprises adoptive, target-cell specific immunotherapy or genetic immunization.

The patients are preferably HLA-A2-positive.

### Methods of detecting, presenting and/or treating liver cancer, in particular HCC or other cancers expressing GPC3

As discussed above, the present invention provides methods of preventing and/or treating liver cancer, in particular hepatocellular carcinoma (HCC) or other cancers expressing GPC3.

In one embodiment, said method comprises the steps of
(a) providing lymphocytes of a patient or a blood donor;
(b) providing one or more of
   (i) a T-cell receptor of the present invention or a soluble T-cell receptor construct or a chimeric T-cell receptor of the present invention or a bi-specific antibody of the present invention,
   (ii) a nucleic acid of the present invention,
   (iii) an expression construct of the present invention,
   (iv) a cell of the present invention, and
   (v) a pharmaceutical composition of the present invention;
(c) *ex vivo* introduction of one or more of (i) to (v) of step (b) into the lymphocytes of step (a) and, thereby, obtaining genetically modified lymphocytes,
(d) administering the genetically modified lymphocytes of step (c) to a subject or patient in need thereof;
(e) optional, combining any of the steps (b) to (d) with radiation, checkpoint inhibitor or cancer chemotherapy.

In one embodiment, said method comprises the steps of
(a) providing one or more of
   (i) a T-cell receptor of the present invention or a soluble T-cell receptor construct or a chimeric T-cell receptor of the present invention or a bi-specific antibody of the present invention,
   (ii) a nucleic acid of the present invention,
   (iii) an expression construct of the present invention,
   (iv) a cell of the present invention, and
   (v) a pharmaceutical composition of the present invention;
(b) direct application, preferably via injection or infusion, of one or more of (i) to (v) of step (a) to a subject or patient in need thereof,
(c) optional, combining step (b) with radiation, checkpoint inhibitor or cancer chemotherapy.

In this embodiment, preferably the bi-specific antibodies or the soluble TCR constructs of the present invention are utilized. Preferably, the bi-specific antibodies or the soluble TCR constructs of the present invention are used as recombinant proteins.

In this embodiment, in step (b) the one or more of (i) to (v) of step (a) are used as recombinant proteins.

The recombinant proteins, preferably the bi-specific antibodies or the soluble TCR constructs of the present invention, are expressed, preferably in E.coli or mammalian cells, and then purified before the application.

T cells express inhibitory receptors such as cytotoxic T-lymphocyte-associated antigen 4 (CTLA-4) or programmed cell death 1 (PD-1), especially after long term exposure to the antigen. The ligands of CTLA-4 and PD-1, B7 and PD-L1 respectively, as well as the ligand of TIM-3, Galectin 9, are frequently expressed on tumor tissue and are involved in induction of T cell tolerance and thus are called immune checkpoints. To avoid silencing of transferred GPC3 specific T cells checkpoint inhibitors or immune checkpoint inhibitors, exemplified by but not restricted to anti-CTLA-4, anti-PD-L1 or anti-PD-1 antibodies, can be used to avoid inhibition of T cells within the tumor microenvironment (described in Hamid et al. (2013)).

The subjects or patients are preferably HLA-A2-positive.

Preferably, the lymphocytes provided in step (a) are T cells, NK cells and/or NKT cells, preferably CD8⁺ T cells.

The lymphocytes provided in step (a) can be obtained from the subject or patient, such as from the blood or by lymphapheresis of allogeneic blood donors.

Preferably, the *ex vivo* introduction in step (c) is carried out via electroporation of a nucleic acid of the present invention or of an expression construct of the present invention, or by transfection reagents, such as liposomes, or by transient RNA transfection.

The expression construct is preferably, as described above,
- a viral construct,
   preferably a lentiviral vector, retroviral vector, or other state of the art viral vector.

Examples for suitable expression constructs are viral vectors, such as
- retroviral vectors (as described in Verra et al, 2004; Porter et al, 2011), such as MP71 vectors (as described in Engels et al., 2003), or retroviral SIN vectors (as described in Yu et al., 1986);
   and
- lentiviral vectors (as described in Josef et al, 2008) or lentiviral SIN vectors, as described in Miyoshi et al., 1998.

Another example for a suitable expression construct is the *Sleeping Beauty* (SB) transposon transposase DNA plasmid system, SB DNA plasmid (as described in Maiti et al., 2013).

As discussed above, the present invention provides a method of detecting, diagnosing, prognosing, preventing and/or treating liver cancer, in particular hepatocellular carcinoma (HCC), or other cancers expressing GPC3.

Said method comprises the detection and/or destruction of liver cancer cells, in particular hepatocellular carcinoma (HCC) cells or of cancer cells of other cancers expressing GPC3, of a patient with the use of the soluble T-cell receptor construct of the present invention,

Preferably, the soluble T-cell receptor constructs are applied to patients by intravenous, subcutaneous or intramuscular infusion or injections, respectively, or by application to the mucosa of the respiratory tract by inhalation or by spraying.

Soluble T-cell receptor constructs can be applied to patients:
- by intravenous infusions,
- by subcutaneous injection or infusion,
- by intramuscular injection,
- by inhalation or by spraying to the mucosal surfaces of the respiratory tract.

Suitable dose ranges for application of soluble T-cell receptor constructs vary between 1 mg to 10 000 mg dependent on the application method and application site. Soluble T-cell receptors can bind to cells expressing GPC3 peptides on their HLA-A2 molecules, namely liver cancer cells, in particular hepatocellular carcinoma (HCC) cells, (or cells of other cancers expressing GPC3) in which GPC3 peptide 367 or GPC3 peptide 326 is presented by MHC I. Soluble T-cell receptors can induce destruction of the cells via different methods, such as radiation by linked radionuclides or killing by toxins such as pseudomonas exotoxin. In addition, soluble T-cell receptors with linked Fc-domains can redirect and activate NK-cells and/or monocytes for the lysis of liver cancer cells, in particular hepatocellular carcinoma (HCC) cells, or cells of other cancers expressing GPC3. Similarly, soluble T-cell receptors linked to anti-CD3-antibodies can redirect and activate T-cells for lysis of liver cancer cells, in particular hepatocellular carcinoma (HCC) cells, or cells of other cancers expressing GPC3.

In one embodiment the method of the invention is for detecting liver cancer, in particular hepatocellular carcinoma (HCC), or other cancers expressing GPC3,
comprising *the in vitro* staining of liver cancer cells, in particular hepatocellular carcinoma (HCC) cells, or cells of other cancers expressing GPC3, by using the soluble T-cell receptor construct of the present invention which is labelled.

The label can be fluorophor(s), gold and/or radionuclide(s).

Soluble T-cell receptors linked to fluorescent molecules such as fluorescein can be used to stain liver cancer cells, in particular hepatocellular carcinoma (HCC) cells, or cells of other cancers expressing GPC3 *in vitro.* For this purpose, liver cancer cells, in particular hepatocellular carcinoma (HCC) cells, or cells of other cancers expressing GPC3, are incubated *in vitro* with the soluble T-cell receptor constructs, and cells expressing HLA-A2/GPC3 peptide-complexes on the cell surface can be detected according to their fluorescence by a Fluorescence activated cell sorter (FACS) or by microscope.

Alternatively, labeled soluble T-cell receptor constructs (e.g. with flourochromes, gold or radionuclides) are injected into a subject or patient and allow detection and subsequent destruction of GP3 positive tumor, tumor metastasis or minimal residual disease after tumor resection.

The soluble TCR-constructs of the invention are suitable to be directly used for the detection and destruction of liver cancer cells, in particular hepatocellular carcinoma (HCC) cells, or cells of other cancers expressing GPC3, without the need of T-cells.

The subjects or patients are preferably HLA-A2-positive.

### GPC3-epitopes and peptides and vaccines and their uses

As discussed above, the present invention provides peptides comprising at least one GPC3 epitope.

The inventors have identified immunodominant GPC3 epitopes, in particular the HLA-A2 bound GPC3 peptide 367.

The at least one GPC3 epitope comprises or consists of the amino acid sequence of SEQ ID NO. 14 *(GPC3 peptide 367).*

SEQ ID NO. 14 shows the amino acid sequence of the GPC3 peptide 367:
FIDKKVLKV

Glypican-3 is a protein that in humans is encoded by the *GPC3* gene. The protein encoded by this gene is a member of the glypican family.
GenBank Accession No.: NM_004484.3, 580 amino acids
UniProt Accession No.: P51654

The amino acid sequence of GPC3 is also shown in SEQ ID NO. 15.

ATGGCCGGGACCGTGCGCACCGCGTGCTTGGTGGTGGCGATGCTGCTCAGCTTGGACTTCCCGGGACAGG
CGCAGCCCCCGCCGCCGCCGCCGGACGCCACCTGTCACCAAGTCCGCTCCTTCTTCCAGAGACTGCAGCC
CGGACTCAAGTGGGTGCCAGAAACTCCCGTGCCAGGATCAGATTTGCAAGTATGTCTCCCTAAGGGCCCA
ACATGCTGCTCAAGAAAGATGGAAGAAAAATACCAACTAACAGCACGATTGAACATGGAACAGCTGCTTC
AGTCTGCAAGTATGGAGCTCAAGTTCTTAATTATTCAGAATGCTGCGGTTTTCCAAGAGGCCTTTGAAAT
TGTTGTTCGCCATGCCAAGAACTACACCAATGCCATGTTCAAGAACAACTACCCAAGCCTGACTCCACAA
GCTTTTGAGTTTGTGGGTGAATTTTTCACAGATGTGTCTCTCTACATCTTGGGTTCTGACATCAATGTAG

ATGACATGGTCAATGAATTGTTTGACAGCCTGTTTCCAGTCATCTATACCCAGCTAATGAACCCAGGCCT
GCCTGATTCAGCCTTGGACATCAATGAGTGCCTCCGAGGAGCAAGACGTGACCTGAAAGTATTTGGGAAT
TTCCCCAAGCTTATTATGACCCAGGTTTCCAAGTCACTGCAAGTCACTAGGATCTTCCTTCAGGCTCTGA
ATCTTGGAATTGAAGTGATCAACACAACTGATCACCTGAAGTTCAGTAAGGACTGTGGCCGAATGCTCAC
CAGAATGTGGTACTGCTCTTACTGCCAGGGACTGATGATGGTTAAACCCTGTGGCGGTTACTGCAATGTG
GTCATGCAAGGCTGTATGGCAGGTGTGGTGGAGATTGACAAGTACTGGAGAGAATACATTCTGTCCCTTG
AAGAACTTGTGAATGGCATGTACAGAATCTATGACATGGAGAACGTACTGCTTGGTCTCTTTTCAACAAT
CCATGATTCTATCCAGTATGTCCAGAAGAATGCAGGAAAGCTGACCACCACTATTGGCAAGTTATGTGCC
CATTCTCAACAACGCCAATATAGATCTGCTTATTATCCTGAAGATCTCTTTATTGACAAGAAAGTATTAA
AAGTTGCTCATGTAGAACATGAAGAAACCTTATCCAGCCGAAGAAGGAACTAATTCAGAAGTTGAAGTC
TTTCATCAGCTTCTATAGTGCTTTGCCTGGCTACATCTGCAGCCATAGCCCTGTGGCGGAAAACGACACC
CTTTGCTGGAATGGACAAGAACTCGTGGAGAGATACAGCCAAAAGGCAGCAAGGAATGGAATGAAAAACC
AGTTCAATCTCCATGAGCTGAAAATGAAGGGCCCTGAGCCAGTGGTCAGTCAAATTATTGACAAACTGAA
GCACATTAACCAGCTCCTGAGAACCATGTCTATGCCCAAAGGTAGAGTTCTGGATAAAAACCTGGATGAG
GAAGGGTTTGAAAGTGGAGACTGCGGTGATGATGAAGATGAGTGCATTGGAGGCTCTGGTGATGGAATGA
TAAAAGTGAAGAATCAGCTCCGCTTCCTTGCAGAACTGGCCTATGATCTGGATGTGGATGATGCGCCTGG
AAACAGTCAGCAGGCAACTCCGAAGGACAACGAGATAAGCACCTTTCACAACCTCGGGAACGTTCATTCC
CCGCTGAAGCTTCTCACCAGCATGGCCATCTCGGTGGTGTGCTTCTTCTTCCTGGTGCACTGA

The nucleotide sequence of GPC3 is also shown in SEQ ID NO. 16.

In one embodiment, the amino acid sequence of the epitope has a length of at least 8 amino acids. In one embodiment, the amino acid sequence of the epitope has a length of 9 amino acids. Preferably, the amino acid sequence of the epitope has a length of 8, 9 or 10 amino acids.

In an embodiment, the peptide has a length in the range of 5 to 50 amino acids, preferably 8 to 25 amino acids, more preferably 8 to 15 amino acids or 9 to 15 amino acids. In an embodiment, the peptide is a nonamere, i.e. has a length of 9 amino acids.

In one embodiment, for MHC I presentation (which is a preferred embodiment), the peptide has a length of 8 to 10 amino acids, preferably 9 amino acids.

In one embodiment, for MHC II presentation, the peptide has a length of 15 to 24 amino acids, preferably 18, 19 or 20 amino acids.

In one embodiment, the peptide of the present invention comprises further GPC3 epitope(s), such as a GPC3 epitope comprising or consisting of the amino acid sequence of SEQ ID NO. *17 (GPC3 peptide 326*).

For example, the peptide of the present invention can comprise or consist of the amino acid sequence of SEQ ID NO. 14 *(GPC3 peptide 367)* and the amino acid sequence of SEQ ID NO. 17 *(GPC3 peptide 326*).

SEQ ID NO. 17 shows the amino acid sequence of the GPC3 peptide 326:
TIHDSIQYV

In one embodiment, the peptide comprises further component(s), such as
- tag(s),
   such as 6-His-tag, Flag-tag,
- linker,
   such as polylinkers to link one peptide/epitope to another peptide/epitope
- label(s),
   such as radioisotopes, enzymes, gold, luminescent or fluorescent labels,
- N- and/or C-terminal modification(s),
   such as comprising acetylation and/or amidation of the N- and/or *C*-terminus,
- drug(s),
   such as tumor chemotherapeutics, kinase inhibitors,
- agent(s),
and/or
combinations thereof.

The skilled artisan will be able to select suitable further components.

Said further component(s) can be covalently attached to the peptide of the vaccine.

In one embodiment, the peptide comprises modified amino acid(s), unnatural amino acid(s) or peptidomimetic(s).

As discussed above, the present invention provides a nucleic acid molecule coding for at least one peptide according to the present invention or a plasmid comprising at least one such nucleic acid molecule.

As discussed above, the present invention provides an antibody against a peptide according to the present invention.

As discussed above, the present invention provides a composition comprising
(i) at least one peptide according to the present invention or at least one nucleic acid according to the present invention,
(ii) optionally, a carrier,
(iii) optionally, an adjuvant.

Preferably, the composition comprises at least two peptides according to the present invention or at least two nucleic acids according to the present invention.

The compositions according to the invention can further comprise
- a carrier
   and/or
- an adjuvant.

Suitable adjuvants are known in the art.

Since peptides are usually poor immunogens, the efficacy of peptide-based vaccine depends on the adequate presentation of the epitopes to the immune system.

The skilled artisan will be able to select suitable carrier(s) and/or adjuvant(s).

In one embodiment, the carrier is a virus particle or parts thereof, an envelope protein of a viral vector or of a virus particle, a nanocarrier or a liposomal compound enveloping the peptide(s) in a micellular structure.

As discussed above, the present invention provides the peptide of the present invention, the nucleic acid molecule of the present invention or the pharmaceutical composition of the present invention for use in medicine.

As discussed above, the present invention provides the peptide of the present invention, the nucleic acid molecule of the present invention or the pharmaceutical composition of the present invention for use as a vaccine.

As discussed above, the present invention provides a vaccine comprising at least one peptide of the invention, a nucleic acid molecule of the present invention or the pharmaceutical composition of the present invention.

The vaccines are preferably peptide-based vaccines.

The vaccines of the present invention can further comprise excipient(s), such as pharmaceutically acceptable excipient(s), which can be necessary for the different administration regimes (e.g. via injection, such as subcutaneously or intradermally).

Further suitable excipient(s) are known to the skilled artisan.

A peptide of the invention or the peptide comprised in a vaccine of the invention does not comprise the full-length glypican-3 (GPC3) protein.

As discussed above, the present invention provides the use of the vaccine of the present invention for preventing and/or treating liver cancer, in particular hepatocellular carcinoma (HCC) or other cancers expressing GPC3.

As discussed above, the present invention provides a method of preventing and/or treating liver cancer, in particular hepatocellular carcinoma (HCC), or other cancers expressing GPC3,
comprising the step of
administering a peptide of the present invention, a nucleic acid molecule of the present invention, a pharmaceutical composition of the present invention or a vaccine of the present invention
to a subject in need thereof.

### Further description of preferred embodiments

The inventors have identified T cell clones that specifically recognise Glypican-3 expressing cells and demonstrate effector functions such as cytokine production and effective killing of target cells. The T cell clones were generated using an allorestricted stimulation of T cells with peptide or mRNA pulsed dendritic cells (DCs).

After successful generation and characterisation of functional T cell clones, the T cell receptor (TCR) sequence was sequenced and subsequently cloned into a retroviral-vector construct. The retroviral-vector based transfer of previously cloned T cell receptors can be used to equip T cells with the GPC3-specific effector function. Functionality of transduced T cells was evaluated *in vitro.*

The identified GPC3 specific TCRs are suitable for adoptive T cell therapy of HCC. HCC expresses like many other tumours tumour-associated antigens (TAAs), one of them is glypican-3 (GPC3). T cells that would naturally respond to TAAs are removed from the system in a process called negative selection during T cell development because TAAs are derived from self-tissue. Nevertheless T cells can be reactivated against tumours by genetic engineering more precisely insertion of a TAA-specific T cell receptor.

T cells carrying the TCRs of the present invention can recognize and specifically eliminate GPC3 expressing tumour cells. Genes coding for the GPC3 specific TCRs of the invention can be inserted into patients' T cells to enable them to recognize and kill cancer cells, especially HCC. Genetic modifications of the TCRs of the present invention to improve migration of T cells into the tumour tissue, to enhance proliferation or resistance to tumour-derived immunosuppressive factors are optional. Additionally, the insertion of suicidal genes that allow the elimination of transferred T cells may be advantageous. T cells that were *ex vivo* equipped with the TCRs of the present invention can be transferred back to the patient where they can exert their anti-tumour activity.

According to the present invention, GPC3 peptide 367 presented by MHC-I is recognized by T cells carrying the T cell receptors of the present invention or GPC3 peptide 326 presented by MHC-I is recognized by T cells carrying the T cell receptors of the present invention, respectively. Furthermore it has been shown that GPC3+ human hepatoma cells are effectively killed by said T cells. Hence, adoptive T-cell immunotherapy with the TCRs of the present invention allows recognition as well as cytotoxic elimination of glypican-3 (GPC3) expressing malignant cells, which makes the TCRs of the present invention a very promising tool to treat HCC patients.

### Identification of immunodominant GPC3 peptides

Immunodominant epitopes for GPC3 have not been described yet. In this study we used Ultra Nano HPLC coupled on-line to the Q Exactive mass spectrometer to obtain a comprehensive HLA class I peptidome from a GPC3 and HLA-A2 positive hepatoma cell line. The resulting data were analysed using the MaxQuant bioinformatics platform.

Two HLA-A2 bound GPC3 peptides were identified, referred to as "GPC3 peptide 367" (GPC3₃₆₇: FIDKKVLKV) and "GPC3 peptide 326" (GPC3₃₂₆: TIHDSIQYV), wherein GPC3 peptide 326 is described in Komori et al., 2006.

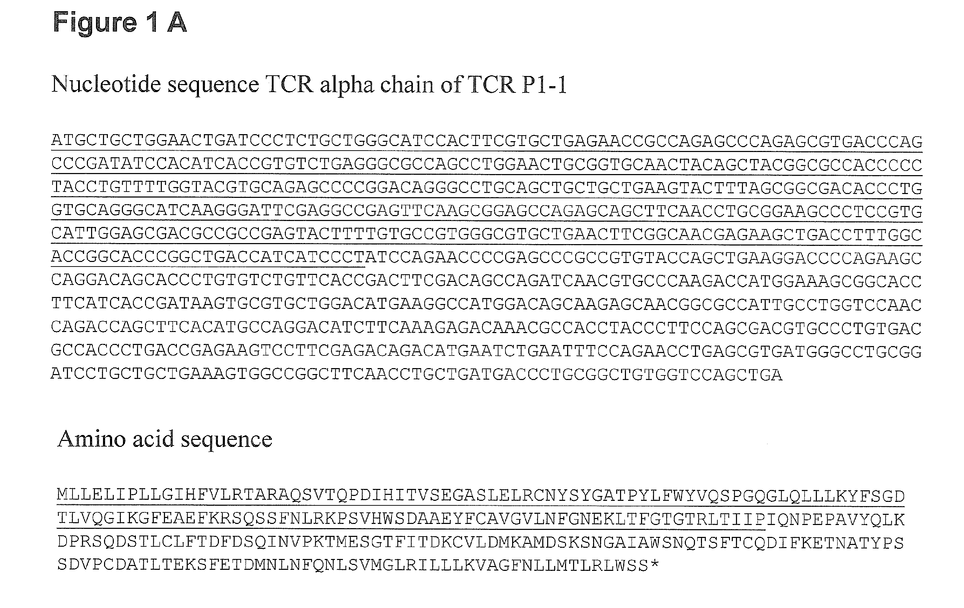

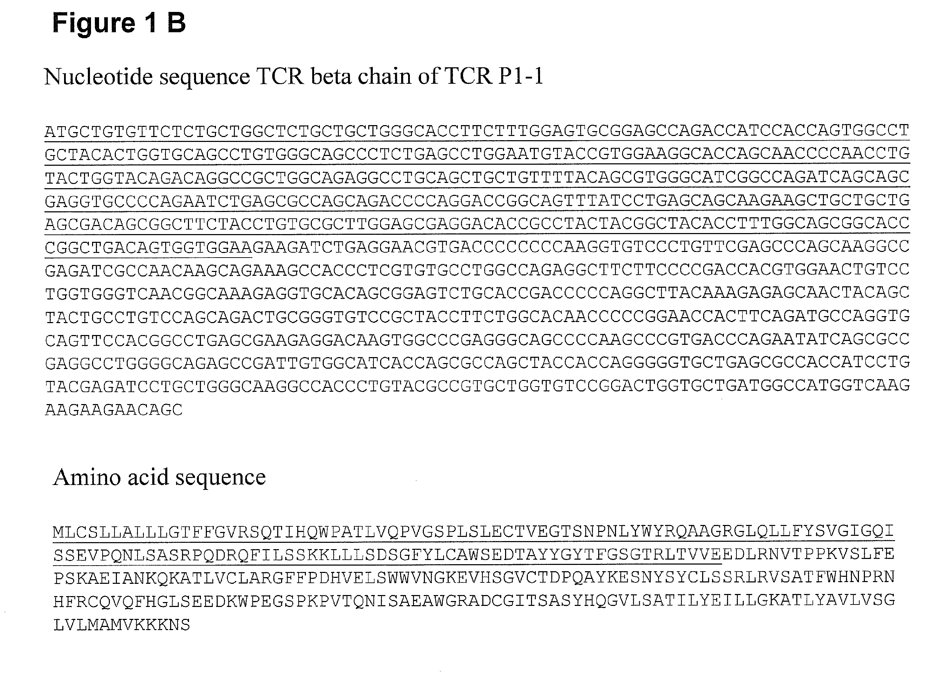

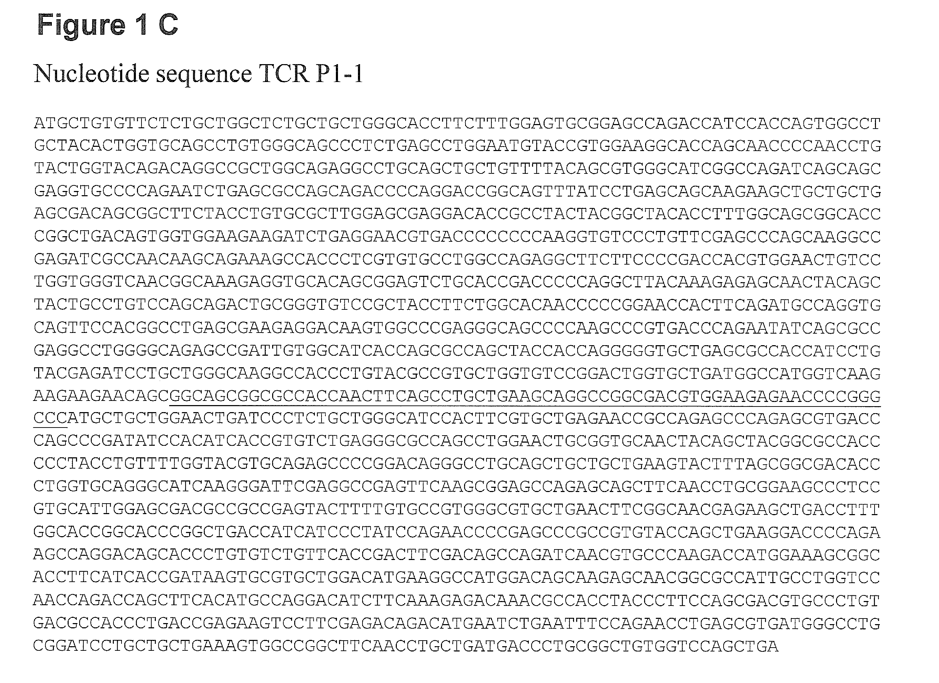

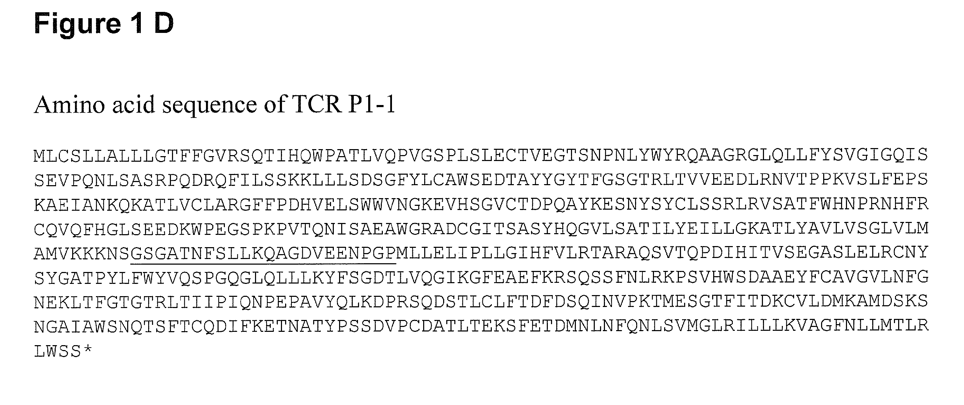

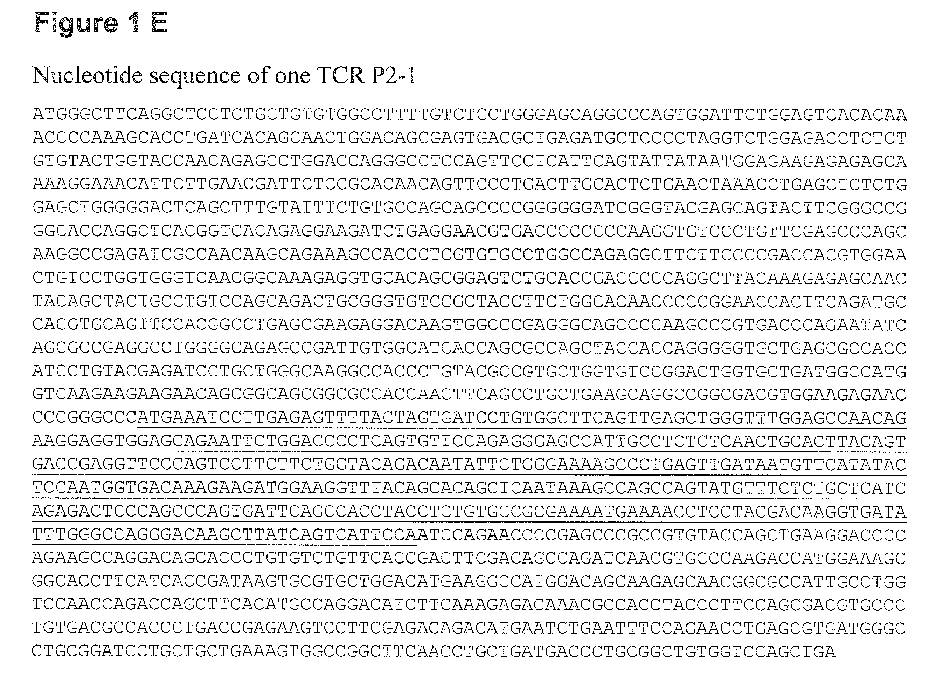

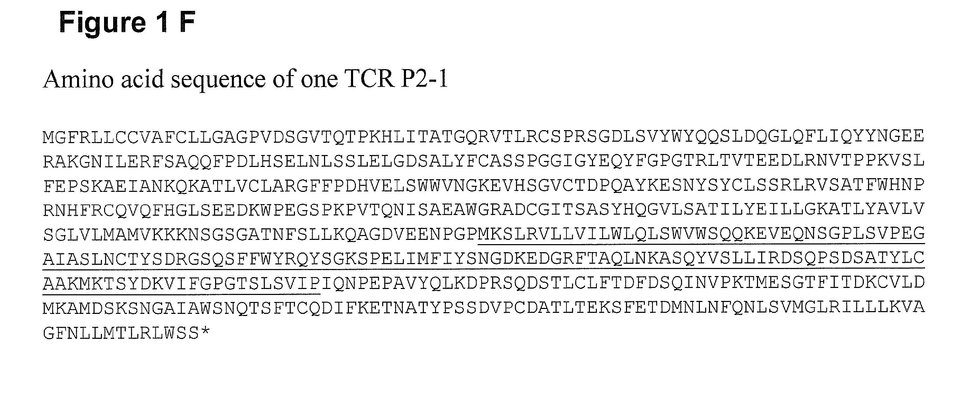

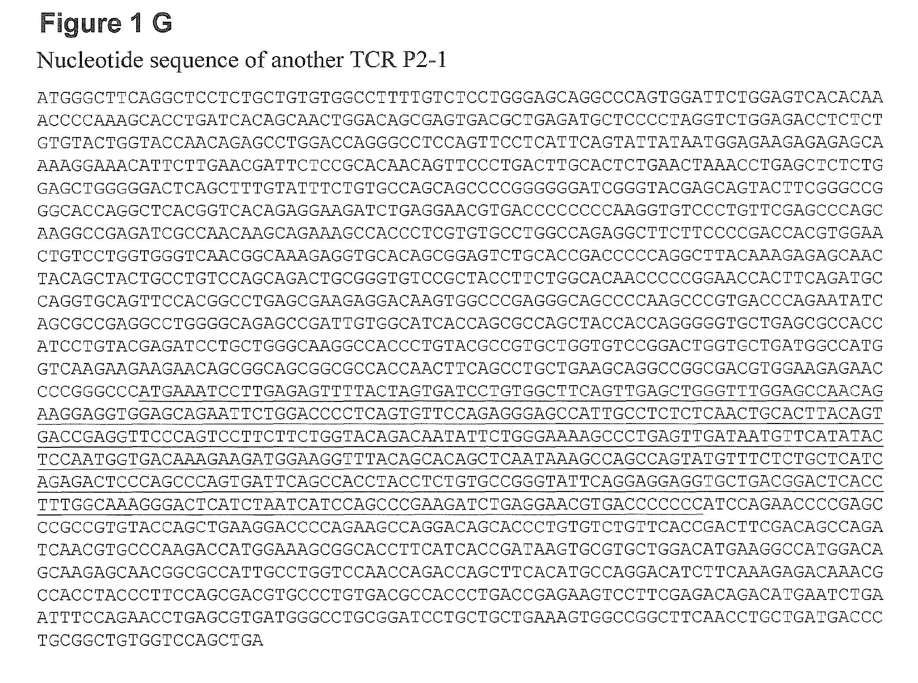

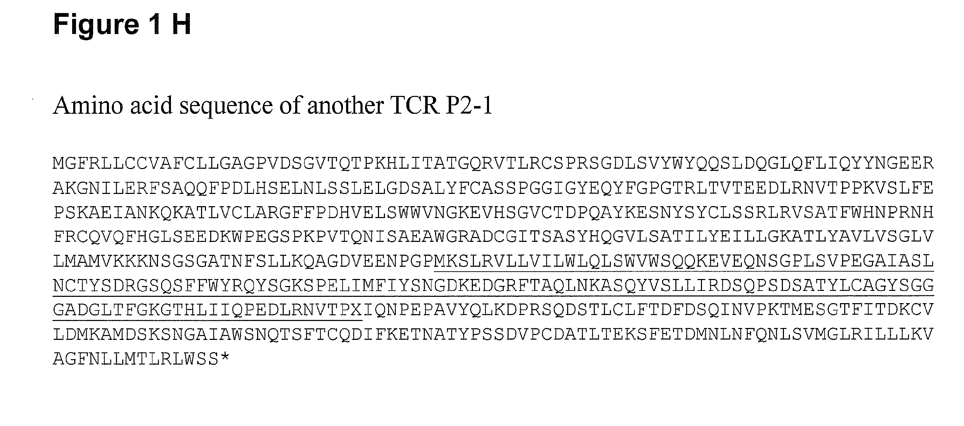

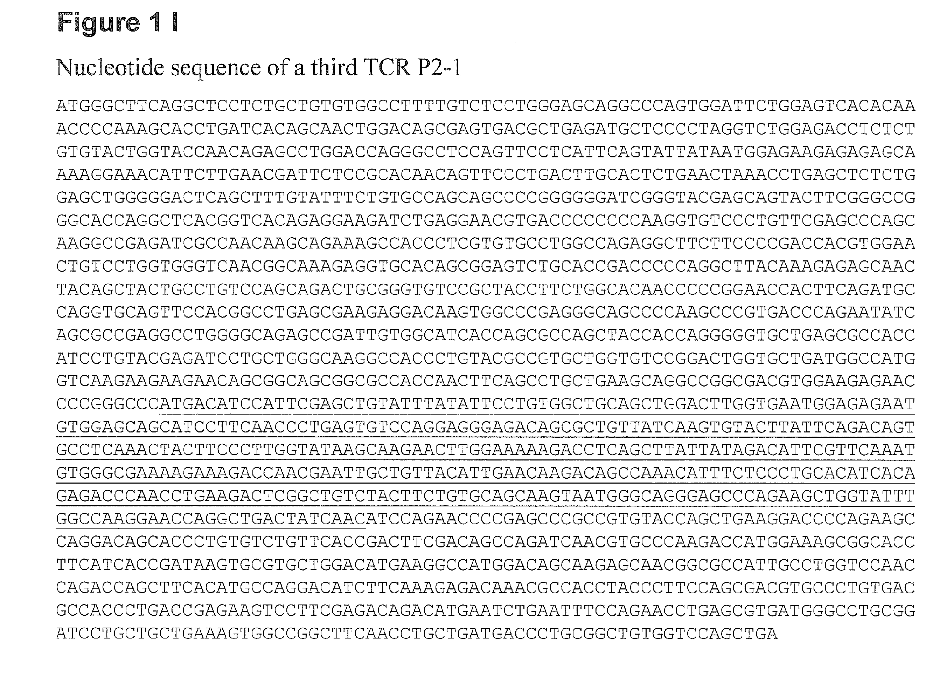

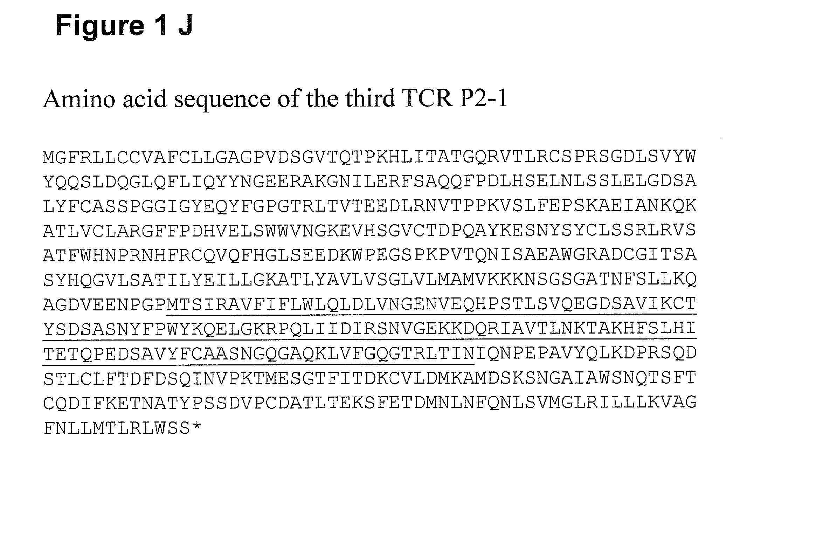

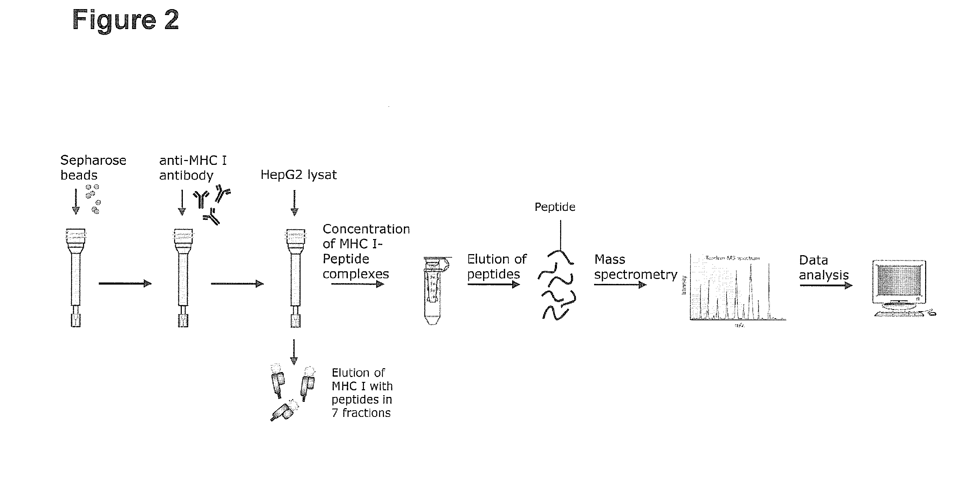

The knowledge of these two peptides enables us to target GPC3 epitopes that are presented on GPC3 positive HCC cells. See also Figure 2.

### T cells and TCR specific for peptide GPC3₃₆₇

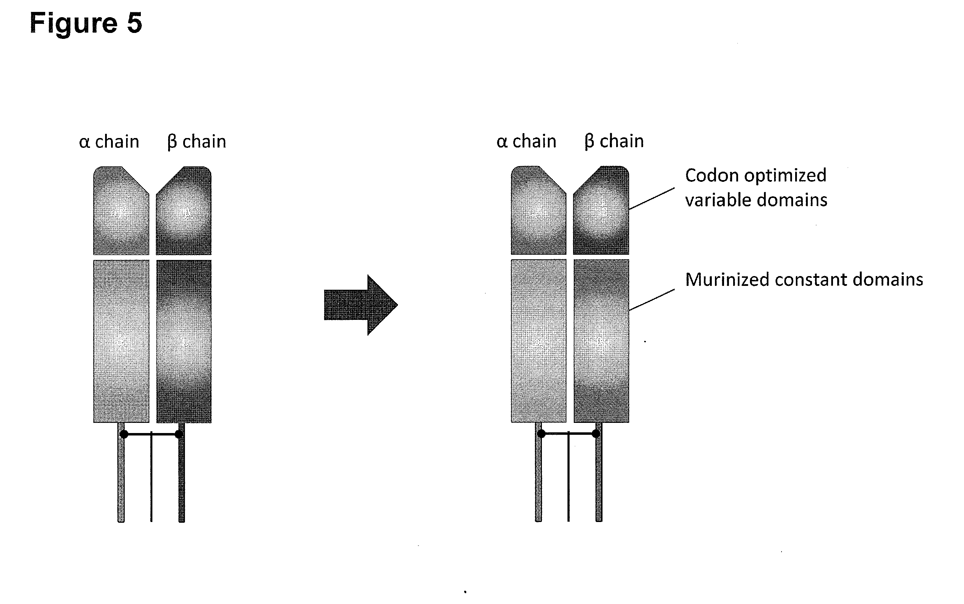

We have identified and cloned the T cell receptor (TCR) specific for GPC3 peptide 367 from respective T cell clones. This TCR is also referred to as P1-1. The T cell receptor sequence coding for the variable parts of alpha and beta chain of P1A was codon optimized to increase the level of expression after viral transfer into T cells. Furthermore the constant domains were exchanged with murine constant domains to avoid miss pairing with endogenous TCR chains and hence ensure optimal expression (see Figure 5).

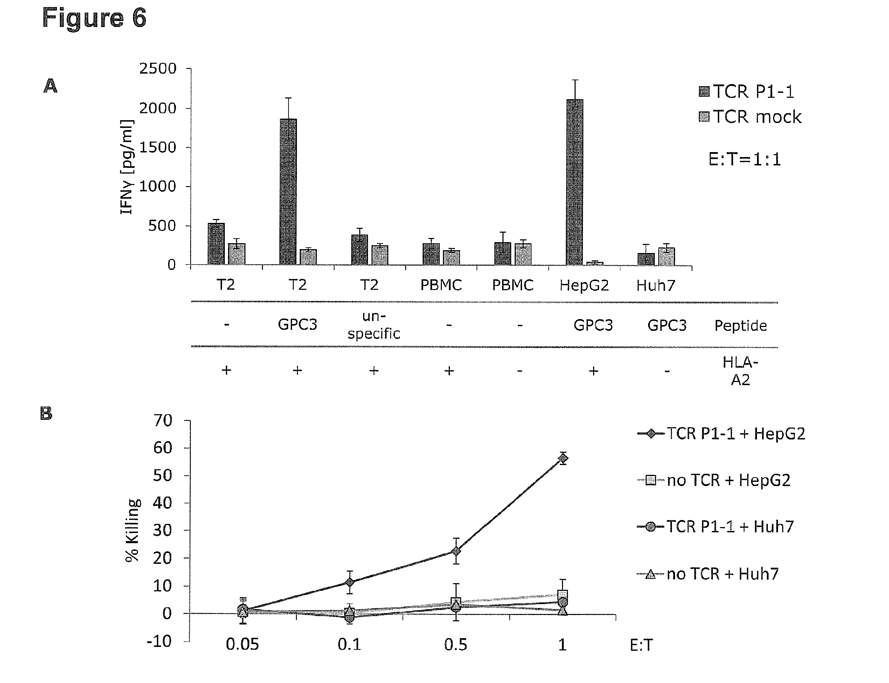

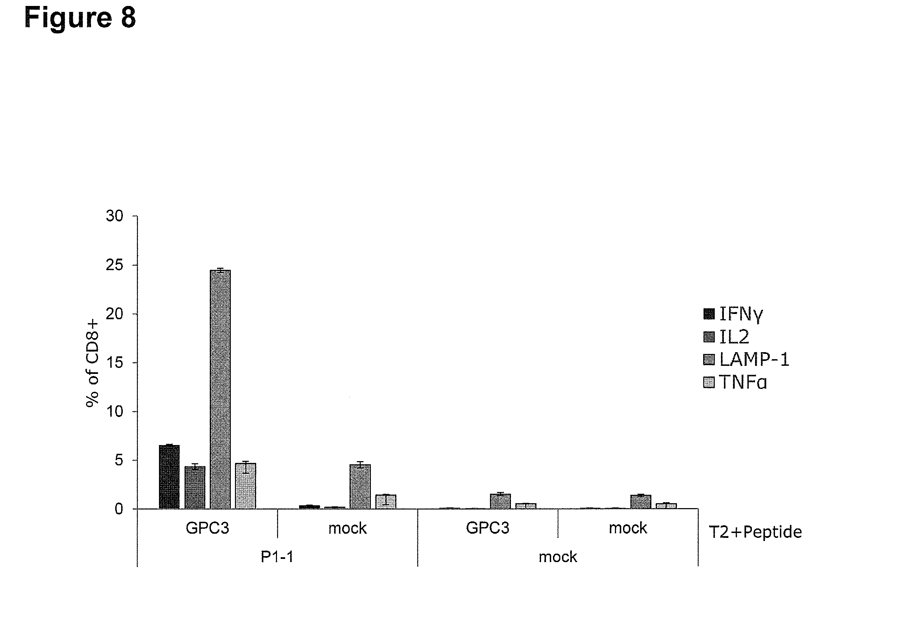

T cells engrafted with this optimized GPC3-specific TCR P1-1 showed strong GPC3 MHC streptamer binding. When co-cultured with GPC3 peptide loaded target cells or a GPC3 expressing hepatoma cell line (HepG2), TCR P1-1 transduced T cells secreted IFNγ, TNFα and IL2 and delocalized the degranulation marker Lamp-1 (see Figure 6A and Figure 8).

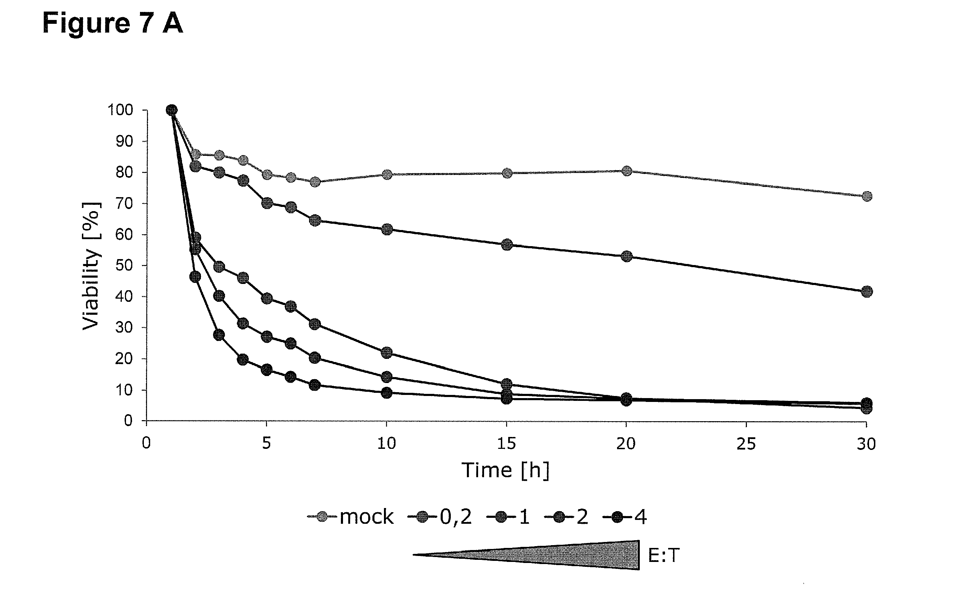

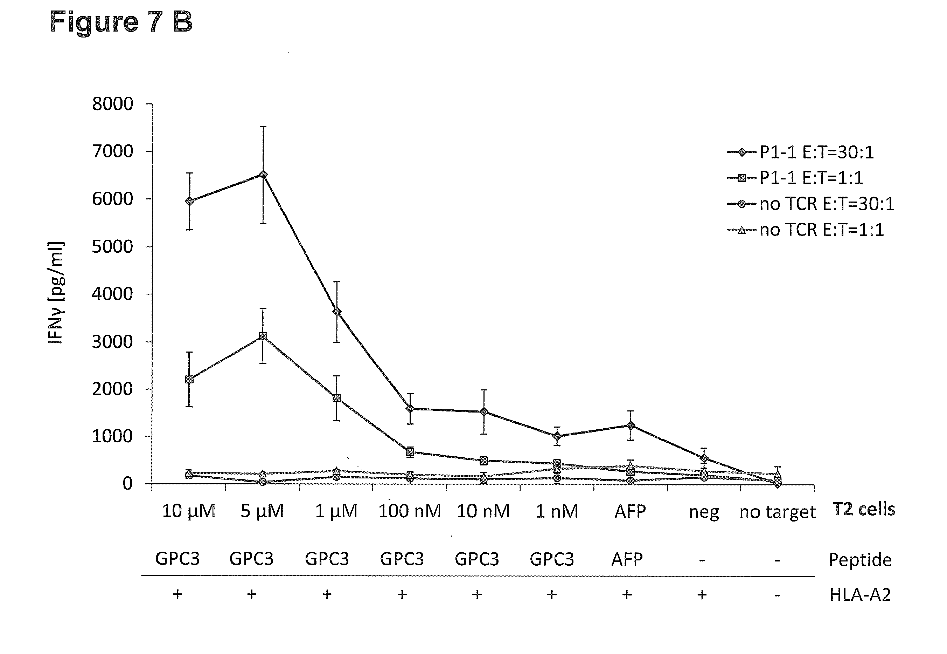

Cytotoxicity was observed by killing of HepG2 cells that are GPC3 and HLA-A2 positive. The cytotoxic effect depends on GPC3 peptide presentation on HLA-A2 since no killing was observed on HLA-A2- GPC3+ Huh7 cells (see Figure 6B). Killing of target cell already occurred at low effector: target (E:T) ratios of 0,1:1 and up to 60% of killing was reached when an E:T ratio of 1:1 was used. More detailed investigation of the killing capacity showed very rapid killing of around 90% of target cells when E:T ratios of 1:1 or higher were used. At lower E:T ratios around 50% of target cells were killed by P1-1 equipped T cells (Figure 7A). To analyse the TCR avidity TCR P1-1 transduced T cells were incubated with T2 cells loaded with peptide GPC3 367 in different concentration. A clear concentration dependent IFNγ secretion was observed that indicated a medium avidity of the TCR of the present invention (Figure 7B).

### TCR specific for peptide GPC3₃₂₆

We have identified and cloned the T cell receptor (TCR) specific for GPC3 peptide 326 from respective T cell clones. This TCR is also referred to as P2-1.

The following examples and drawings illustrate the present invention without, however, limiting the same thereto.

### BRIEF DESCRIPTION OF THE DRAWINGS

**Figure 1****.**
   **A to D)** *Sequence of the TCR P1-1*
      **A)** Sequence of the TCR P1-1 alpha chain.
         Nucleotide and amino acid sequence of the TCR alpha chain: underlined is the variable alpha region.
      **B)** Sequence of the TCR P1-1 beta chain.
         Nucleotide and amino acid sequence of the TCR beta chain: underlined is the variable beta region.
      **C and D)** Sequence of the TCR P1-1.
         Nucleotide and amino acid sequence of the TCR: underlined is the linker region.
   **E to J)** *Sequences of the TCR P2-1*
      **E and F)** Sequence of one TCR P2-1.
         Nucleotide and amino acid sequence of the TCR: underlined is the TCR alpha chain.
      **G and H)** Sequence of another TCR P2-1.
         Nucleotide and amino acid sequence of the TCR: underlined is the TCR alpha chain.
      **I and J)** Sequence of a third TCR P2-1.
         Nucleotide and amino acid sequence of the TCR: underlined is the TCR alpha chain.
**Figure 2****.** *Schematic illustration of mass spectrometric analysis of MHC I bound peptides on HepG2 cells.*
   MHC-I peptide complexes were isolated from total cell lysate by sepharose bound anti-MHC I antibodies. After concentration, peptides were eluted from MHC I and subsequently analysed using Ultra Nano HPLC coupled on-line to the Q Exactive mass spectrometer.
**Figure 3****.** *Time schedule for the allorestricted stimulation of T cells.*
   according to Wilde et al., 2012. DCs are prepared from fresh blood, maturated and electroporated with HLA-A2 RNA. GPC3 can be loaded either in form of peptide or GPC3 mRNA can be electroporated together with HLA-A2. Ten hours after electroporation DCs are co-incubated with CLTs from the same HLA-A2 negative donor. On day 14 and 21, the GPC3-specific HLA-A2-restricted T cell cultures are stained with multimer and sorted via fluorescence activated cell sorting. The sorted T cells are cloned in limiting dilution cultures or expanded as bulk lines. Functional assay for evaluation of their specific effector functions are performed.
**Figure 4****.**
   **A**) Streptamer staining (P1; 367) of stimulated T cells, corresponding T cell lines, and one exemplary T cell clone. Streptamer binding T cell population was enriched from 0.6% in the primary co-culture to 57% in the T cell line. T cell clones showed strong streptamer binding.
   **B**) T cells clones specific for P1 or P2 were identified in an IFNγ secretion assay. Specific T cell clones released IFNγ on T2 cells loaded with GPC3 peptides but not on T2 cells without or with control peptides.
**Figure 5****.** *TCR Modifications:*
   1) Codon optimization for higher expression levels.
   2) Murinized constant domains to avoid miss pairing with endogenous TCR chains.

### Figure 6.

**A)** P1-1 transduced T cells specifically secrete IFNγ when co-incubated with T2 cells loaded with GPC3 peptide 367 but not on other target cells.
**B)** Killing of GPC3+ human hepatoma cells by T cells engrafted with the *GPC3₃₆₇* specific TCR P1-1. GPC3+ HLA-A2+ HepG2 cells and GPC3+ HLA-A2- Huh7 cells were incubated with P1-1 transduced T cells at different effector target (E:T) ratios. P1-1 enables T cells to effectively kill target cells at low E:T ratios in an HLA-A2 dependent manner.

### Figure 7.

**A)** Killing of GPC3+ human hepatoma cells by T cells engrafted with the GPC3 367 specific TCR P1-1. GPC3+ HLA-A2+ HepG2 cells were incubated with P1-1 transduced T cells at different effector target (E:T) ratios and target cell viabitity was measured over time. P1-1 enables T cells to rapidily kill target cells at low E:T ratios.
**B)** P1-1 transduced T cells specifically secrete IFNγ when co-incubated with T2 cells loaded with GPC3 peptide 367 in a peptide concentration dependent fashion.

### Figure 8.

P1-1 transduced T cells specifically secrete IFNγ, IL-2 and TNF-α and delocalize the degranulation marker LAMP-1 when co-incubated with T2 cells loaded with GPC3 peptide 367 but not on mock peptide loaded target cells.

### EXAMPLES

### 1. Material and Methods

### 1.1 PBMC and cell lines

Peripheral blood mononuclear cells (PBMC) from healthy human donors were isolated by Ficoll density gradient centrifugation.

T2 is a HLA-A2+ TAP deficient human lymphoma cell line that was used as a target in IFNγ-release assays. LCL-B27 is a HLA-B27+ B-lymphoblastoid cell line that was used as a feeder cell line for unspecific T cell restimulation.

Human Embryonic Kidney 293 (293T) cells were used as producer cell line of virus supernatant for T cell transduction.

HepG2 is a human hepatoma cell line expressing HLA-A2 and GPC3, which was used as a target in killing assays.

Mammalian cell culture was performed under sterile cell culture conditions and cell were incubated at 37°C and 5% CO₂.

### 1.2 Media

All T cell cultures were maintained in human T cell medium (hTCM) compromised of RPMI1640 medium supplemented with 10 % human serum, 4 mM L-glutamine, 100 IU/ml penicillin, 100 µg/ml streptomycin, 6 µg/ml gentamycin, 1 mM sodium pyruvate and 1x Non-Essential Amino Acids (NEAA). Human serum was isolated in our laboratory from healthy donors.

Dendritic cell cultures were maintained in DC medium consisting of very low endotoxin RPMI 1640 medium supplemented with 1.5 % human serum, 100 IU/ml penicillin and 100 µg/ml streptomycin.

LCL and T2 cells were maintained in RPMI1640 medium supplemented with 10 % fetal bovine serum, 4 mM L-glutamine, 100 IU/ml penicillin, 100 µg/ml streptomycin, 1 mM sodium pyruvate and 1x NEAA.

HepG2 cells were maintained in DMEM medium supplemented with 10 % fetal bovine serum, 4 mM L-glutamine, 100 IUhnl penicillin, 100 µg/ml streptomycin, 1 mM sodium pyruvate and 1x NEAA.

### 1.3 Cytokines, peptides multimers and antibodies

Recombinant human IL-4 and IL-6 were obtained from GellGenix. Recombinant human GM-CSF, IL-7, IL-15, TNFα, IL-1β, PGE₂ and IL-2 were purchased from Genzyme, Promokine, Peprotech, Miltenyi Biotec, R&D, Pfizer and Novartis, respectively.

The HLA-A2-restricted peptides representing defined epitopes from GPC3 (367: FIDKKVLKV; 326: TIHDSIQYV) were synthesized by iba.

HLA-A2-GPC3-multimers were generated by AG Busch, Institute for Medical Microbiology, Immunology and Hygiene, TU Munich (Knabel et al., 2002). Strap-Tactin PE for labelling of MHC-I multimers was purchased from iba.

Anti-human CD8-APC, CD8-PE, anti-CD14, anti-CD83 and anti-CD80 antibodies were obtained from BD, Immunotools, eBioscience and Immunotech, respectively. Propidiumiodide (1mg/ml) was obtained from Roth.

Anti-human CD3 (OKT3) was kindly provided by AG Kremmer, Institute for Molecular Immunology, Helmholtz Zentrum Munich.

### 1.4 Generation of dendritic cells (DC) for T cell stimulation

To generate DC PBMC from a healthy HLA-A2 negative donor were resuspended in DC medium and plated at 5x10⁶ cell per 75 cm² NUNC culture flask. After incubated for 1.5 hours at 37°C non-adherent cells were removed by extensive washing. The remaining adherent monocytes were cultured in medium containing 100 ng/ml GM-CSF and 20 ng/ml IL-4 for 24 hours. On day 2 immature DC were differentiated by addition of maturation cytokines (10 ng/ml TNFα, 10 ng/ml IL-1β, 15 ng/ml IL-6 and 1 µg/ml PGE2) directly to the culture. DC were incubated for an additional day before mature DC were harvested. Maturation was confirmed by staining with anti-CD14, anti-CD83 and anti-CD80 and subsequent flow cytometry a FACSCanto II (BD Biosciences).

Mature DC were transfected with GPC3 and HLA-A2 by RNA electroporation. 2x10⁶ cells were resuspended in 200 µl OptiMEM I medium and mixed with 25 µg HLA-A2 RNA and 50 µg GPC3 RNA. The DC-RNA mixture was placed into a 0.4 cm electroporation cuvette and incubated on ice for 3 min. Electroporation was performed with the Gene Pulser Xcell (Bio-Rad) using the exponential electroporation protocol at 300 V and 150 µF. HLA-A2 RNA and GPC3 RNA were *in vitro* (ivt) transcribed using the mMESSAGE mMACHINE T7 kit and Poly(A) tailing kit (both Ambion), according to the manufacturer's instructions. HLA-A2 and GPC3 expression was assed via flow cytometry.

### 1.5 Allorestricted stimulation of T cells

CD8+ T lymphocytes were enriched from total PBMCs via negative selection using the Dynabeads® Untouched™ Human CD8 T Cells Kit from Invitrogen. Primary cultures contained 1×10⁶ CD8+ T cells and 1x10⁵ HLA-A2 and GPC3 ivt-RNA pulsed mature DC in 2 ml of hTCM medium per well of a 24-well tissue culture plate. 5 ng/ml IL-7 was added immediately to the stimulation on day 0. 50 IU/ml IL-2 were added on day 2 and thereafter every 3^{rd} day. Primed T cells were restimulated seven days later using freshly generated ivt-RNA pulsed mature DC.

### 1.6 Multimer staining and cloning of T cells

After 14 days of T cell stimulation HLA-A2-restricted GPC3-specific T cells were detected by MHC-1-multimer staining. GPC3 MHC-multimers were labelled with streptactin-PE prior to staining. For each 1x10⁶ T cells 0.2 µg PE-labelled GPC3 MHC-multimers were used. T cells were incubated with PE-labelled GPC3 MHC-multimers for 20 min in 50 µl PBS + 0.5 % human serum on ice and in the dark. APC-labelled anti-human CD8 was added and incubated for an additional 20 min, before washing and analysis by flow cytometry. Dead cells were excluded by propidium iodide staining. For sorting up to 10x10⁶ cells were stained as described above and sorted on a FACS Aria sorter (BD Biosciences). Sorted CD8+ GPC3 MHC-multimer+ T cells were cloned by limiting dilution. Therefore 0.3 CD8+ GPC3 MHC-multimer+ T cells were seeded per well of a 96-well round-bottom plate. In Addition each well contained a feeder mixture consisting of 1x10⁵ allogeneic PBMC (irradiated to 35 Gy), 1x10⁴ LCL-B27 (irradiated to 50 Gy), 30 ng/ml OKT3 and 50 IU/ml IL-2 in 200 µl of hTCM. After 2 weeks of incubation at 37°C aliquots of wells containing proliferating cells were analysed with regard to their GPC3 specificity in an IFN-γ release assay and by multimer staining as described above. GPC3-speicific CD8+ GPC3 MHC-multimer+ T cells were further expanded by restimulation with the feeder mixture as discribed.

### 1.7 IFN-γ release assay

T2 cells, which were used as targets, were loaded with 5 µM GPC3 peptide for 1.5 h prior to co-incubation with T cells. 2x10³ T cells were co-incubated with 1x10⁴ peptide loaded T2 cells in round-bottom 96-well plates yielding in an effector to target ratio of 0.2:1. T cells co-incubated with mock-peptide loaded T2 cells served as negative controls. After 24 h of co-culture supernatants were harvested and investigated by standard anti-human IFN-γ ELISA (BioLegend).

### 1.8 T cell receptor (TCRs) analysis and cloning of reactive TCR

For TCR analysis of GPC3 specific T cell clones RNA was extracted from T cell clones with Trizol and cDNA was synthesized using superscript II reverse transcriptase (Invitrogen). Variable parts of α- and β-chains were amplified with degenerated primer (Table 1) that cover 99 % of the TCR variable gene segment families followed by agarose gel purification and DNA sequencing (GATC).

**Table 1:**

| | **FORWARD** | **REVERSE** |
|---|---|---|
| **ALPHA** | 5'human panVa ("VPANHUM"): | 3'human CA2: |
| | 5'- TGAGTGTCCCPGAPGG2P-3' (P = A or G; 2 = A, G or T) | 5'-GTGACACATTTGTTTGAGAATC-3' |
| | [SEQ ID NO. 42] | [SEQ ID NO. 43] |
| **BETA** | VP1:GCIITKTIYTGGTAYMGACA | CP1: |
| | [SEQ ID NO. 44] | GCACCTCCTTCCCATTCAC |
| | or | [SEQ ID NO. 46] |
| | VP2: CTITKTTnITTGGTAYCIKCAG | |
| | (I = inosine, W = A/T, M = A/C, Y = C/T, K = G/T) | |
| | [SEQ ID NO. 45] | |

Variable parts of α and -β chains of a GPC3 specific TCR were codon optimized for improved gene expression (Geneart) and cloned into the retroviral vector pMP71 via EcoRI and NotI restriction sites. Constant chains were murinized to improve pairing of the transgenic TCR and α- and β-chain were linked via a P2A element.

### 1.9 Retroviral TCR transfer into human PBMC

293T cells were co-transfected with 2 µg retroviral plasmid pMP71-GPC3-TCR, 1 µg pcDNA3.1-Mo-MLV and 1 µg pALF-10A1 the later representing plasmids that carry the retroviral genes for gag/pol and env, respectively. Transfection was performed with 10 µl lipofectamine according to the standard lipofectamin transfection protocol. Freshly isolated human PBMCs were activated on anti-CD28 and OKT3 coated 24-well plates in hTCM supplemented with 300 IU/ml IL-2 for two days. Activated PBMCs were transduced twice, on day 3 and 4. Virus containing supernatant of transfected 293T cells was harvested two days after transfection and added to RetroNectin-coated 24-well culture plates. The virus was spinoculated for 2 h at 32°C and 2000 g. After centrifugation media was replaced with 1x10⁶ activated T cells/well in hTCM supplemented with 100 IU/ml IL-2. T cells were spinoculated with 1000 g for 10 min at 32°C. After 24 h of incubation medium was replaced by fresh medium containing 50 IU/ml IL2 and T cells were incubated for additional 48 h. Transduced PBMC were analysed by GPC3 MHC-multimer staining and in functional assay at multiple time points.

### 1.10 Functional assays

Transduced PBMC were analysed with regard to their IFN-γ secretion capacity after co-incubation with different target cells as described above. Peptide concentrations and effector to target ratios varied. To assess killing ability of transduced PBMC the cells were co-incubated with HepG2 cells that were seeded on E-plate 96 culture plates (Roche). Target cell viability was measured on the XCelligence (Roche) over time. Alternatively cell titre blue assay was performed after 24 h of co-incubation.

### 2. Results

### 2.1 Identification of immunodominant GPC3 peptides presented on MHC class I molecules of human hepatoma cells

Immunodominant epitopes for GPC3 have not been described yet. In this study we used Ultra Nano HPLC coupled on-line to the Q Exactive mass spectrometer to obtain a comprehensive HLA class I peptidome from a GPC3 and HLA-A2 positive hepatoma cell line. The resulting data were analysed using the MaxQuant bioinformatics platform. Two HLA-A2 bound GPC3 peptides could be identified, herein referred to as
- GPC3 peptide 367 or GPC3₃₆₇ or GPC3-P1:
   FIDKKVLKV [SEQ ID NO. 14]
   and
   GPC3 peptide 326 or GPC3₃₂₆ or GPC3-P2: (described in Komori et al., 2006)
   TIHDSIQYV [SEQ ID NO. 16].

The knowledge of these two peptides enables us to target GPC3 epitopes that are presented on GPC3 positive HCC cells.

### 2.2 In vitro production of Glypican-3 mRNA and expression of GPC3 in DCs after mRNA electroporation

A construct containing GPC3 has been cloned, from which GPC3 mRNA can be produced. Glypican-3 expression has been shown from the plasmid pcDNA3.1(-)GPC3 after transfection of 293T cells. Expression of GPC3 from this construct after electroporation of DCs was optimized. We were able to detect GPC3 after electroporation of DCs. GPC3 was also expressed when co-electroporated with HLA-A2. HLA-A2 expression was stable for at least 24 h. In contrast GPC3 could only be detected up to 2 h post electroporation and was rapidly degraded thereafter.

### 2.3 AUorestricted stimulation of T cells

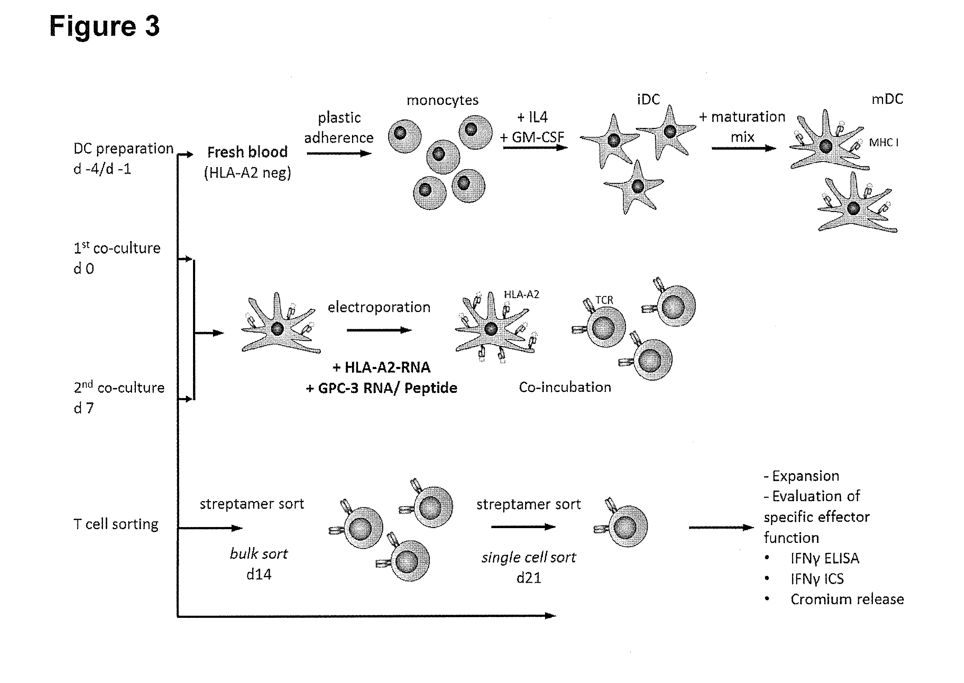

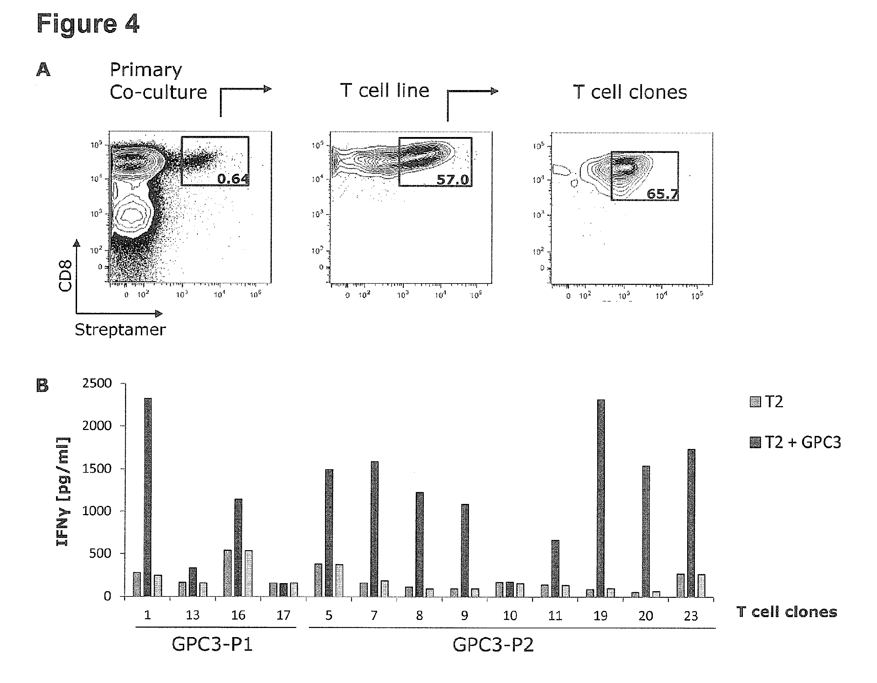

To isolate tumour reactive high avidity T cells, an allo-restricted stimulation approach (see Figure 3) was used, as described by Wilde et. al., 2012. For stimulation of naïve T cells, autologous dendritic cells were co-transfected with GPC3 and HLA-A2 RNA and used as antigen presenting cells. T cells from the naïve T cell repertoire of HLA-A2 negative donors were co-cultured with and expanded on these HLA-A2+ GPC3+ DCs. After two weeks, MHC streptamer-positive CD8+ T cells specific for both targeted GPC3 epitopes were detected (<1%). We were able to enrich these cell populations further to 57% GPC3-P1- and 35% GPC3-P2-MHC streptamer-positive T cell lines and grew T cell clones from them. In a co-culture with GPC3-P1/ -P2 peptide loaded T2 cells we identified T cell clones displaying specific effector function by IFNγ secretion (see Figure 4). The specific effector function could be verified in an intracellular cytokine staining for IFNγ and TNFα. Furthermore T cell clones secreted IFNγ when co-cultured with HLA-A2+ GPC3+ hepatoma cells (HepG2). Functional T cell clones showed strong GPC3 MHC streptamer binding.

### 2.4 T cells specific for peptide GPC3₃₆₇

We have identified and cloned the T cell receptor (TCR) specific for GPC3 peptide 367 from our T cell clones. This TCR is also referred to as P1-1. The T cell receptor sequence coding for the variable parts of alpha and beta chain of P1-1 were codon optimized to increase the level of expression after viral transfer into T cells. Furthermore the constant domains were exchanged with murine constant domains to avoid miss pairing with endogenous TCR chains and hence ensure optimal expression (see Figure 5).

T cells engrafted with this optimized GPC3 specific TCR showed strong GPC3 MHC streptamer binding. When co-cultured with GPC3 peptide loaded target cells or a GPC3 expressing hepatoma cell line (HepG2), GPC3 TCR transduced T cells secreted IFNγ, TNFα, IL2 and the degranulation marker Lamp-1 (see Figure 6A and data not shown). Furthermore cytotoxicity was observed by killing of up to 60% of HepG2 cells (see Figure 6B).

The features disclosed in the foregoing description, in the claims and/or in the accompanying drawings may, both separately and in any combination thereof, be material for realizing the invention in diverse forms thereof.

### REFERENCES

Baumhoer D, Tornillo L, Stadlmann S, Roncalli M, Diamantis EK, Terracciano LM. Glypican 3 expression in human nonneoplastic, preneoplastic, and neoplastic tissues: a tissue microarray analysis of 4,387 tissue samples. Am J Clin Pathol. 2008 Jun;129(6):899-906. Boulter JM and Jakobsen BK. Stable, soluble, high-affinity, engineered T cell receptors: novel antibody-like proteins for specific targeting of peptide antigens. Clin Exp Immunol. 2005; 142(3): 454-460.
Csoka M, Strauss G, Debatin KM, Moldenhauer G. Activation of T cell cytotoxicity against autologous common acute lymphoblastic leukemia (cALL) blasts by CD3xCD19 bispecific antibody. Leukemia 1996 Nov; 10(11): 1765-72.
Engels B, Cam H, Schüler T, Indraccolo S, Gladow M, Baum C, Blankenstein T, Uckert W. Retroviral vectors for high-level transgene expression in T lymphocytes. Hum Gene Ther. 2003 Aug 10;14(12):1155-68*.*
Hamid O et al. Safety and tumor responses with lambrolizumab (anti-PD-1) in melanoma. N Engl J Med. 2013 Jul 11;369(2):134-44.
Han EQ, Li XL, Wang CR, Li TF, Han SY. Chimeric antigen receptor-engineered T cells for cancer immunotherapy: progress and challenges. J Hematol Oncol. 2013 Jul 8;6:47.
Knabel, M., Franz, T.J., Schiemann, M., Wulf, A., Villmow, B., Schmidt., B., Bernhard, H., Wagner, H. and Busch, D. Reversible MHC multimer staining for functional isolation of T-cell populations and effective adoptive transfer. Nature Medicine 2002, 8 (6), 631-637.
Komori H, Nakatsura T, Senju S, Yoshitake Y, Motomura Y, Ikuta Y, Fukuma D, Yokomine K, Harao M, Beppu T, Matsui M, Torigoe T, Sato N, Baba H, Nishimura Y. Identification of HLA-A2- or HLA-A24-restricted CTL epitopes possibly useful for glypican-3-specific immunotherapy of hepatocellular carcinoma. Clin Cancer Res. 2006 May 1;12(9):2689-97.
Lunde E, Løset GÅ, Bogen B, Sandlie I. Stabilizing mutations increase secretion of functional soluble TCR-Ig fusion proteins. BMC Biotechnology 2010, 10:61
Maiti SN, Huls H, Singh H, Dawson M, Figliola M, Olivares S, Rao P, Zhao YJ, Multani A, Yang G, Zhang L, Crossland D, Ang S, Torikai H, Rabinovich B, Lee DA, Kebriaei P, Hackett P, Champlin RE, Cooper LJ. Sleeping beauty system to redirect T-cell specificity for human applications. J Immunother. 2013 Feb;36(2):112-23.
Miyoshi H, Blömer U, Takahashi M, Gage FH, Verma IM. Development of a self-inactivating lentivirus vector. J Virol. 1998 Oct;72(10):8150-7.
Yu SF, von Rüden T, Kantoff PW, Garber C, Seiberg M, Rüther U, Anderson WF, Wagner EF, Gilboa E. Self-inactivating retroviral vectors designed for transfer of whole genes into mammalian cells. Proc Natl Acad Sci USA 1986 May;83(10):3194-8.
Wilde S, Geiger C, Milosevic S, Mosetter B, Eichenlaub S, Schendel DJ. Generation of allo-restricted peptide-specific T cells using RNA-pulsed dendritic cells: A three phase experimental procedure. Oncoimmunology. 2012 Mar 1;1(2):129-140.

## Claims

1. A T-cell receptor (TCR) comprising:
(i) a T cell receptor α-chain comprising the amino acid sequence of SEQ ID NO. 1 *(variable alpha)*
or an amino acid sequence that has at least 80 % identity to the amino acid sequence of SEQ ID NO. 1, more preferably at least 85 % identity, more preferably 90 % or 95 % identity,
or any variation of the TCR sequence provided that said variation retains its functional ability to bind to the epitope with the amino acid sequence of SEQ ID NO. 14 *(GPC3 peptide 367)* or to its HLA-A2 bound form
and/or
(ii) a T-cell receptor β-chain comprising the amino acid sequence of SEQ ID NO. 2 *(variable beta)*
or an amino acid sequence that has at least 80 % identity to the amino acid sequence of SEQ ID NO. 2, more preferably at least 85 % identity, more preferably 90 % or 95 % identity,
or any variation of the TCR sequence provided that said variation retains its functional ability to bind to the epitope with the amino acid sequence of SEQ ID NO. 14 *(GPC3 peptide 367)* or to its HLA-A2 bound form.

2. The T-cell receptor (TCR) of claim 1, comprising
(i) a T cell receptor α-chain further comprising the amino acid sequence of SEQ ID NO. 3 *(murine alpha)*
or an amino acid sequence that has at least 80 % identity to the amino acid sequence of SEQ ID NO. 3, more preferably at least 85 % identity, more preferably 90 % or 95 % identity,
and/or
(ii) a T-cell receptor β-chain further comprising the amino acid sequence of SEQ ID NO. 4 *(murine beta)*
or an amino acid sequence that has at least 80 % identity to the amino acid sequence of SEQ ID NO. 4, more preferably at least 85 % identity, more preferably 90 % or 95 % identity,
and/or
(iii) a linker or hinge region comprising the amino acid sequence of SEQ ID NO. 5 *(P2A)*
or an amino acid sequence that has at least 80 % identity to the amino acid sequence of SEQ ID NO. 5.

3. The T-cell receptor (TCR) of claim 1 or 2, comprising
(i) a T cell receptor α-chain comprising the amino acid sequence of SEQ ID NO. 1 *(variable alpha)* and SEQ ID NO. 3 *(murine alpha),* preferably comprising the amino acid sequence of SEQ ID NO. 6 *(variable alpha* + *murine alpha)*
or an amino acid sequence that has at least 80 % identity to the amino acid sequence of SEQ ID NO. 6, more preferably at least 85 % identity, more preferably 90 % or 95 % identity,
or any variation of the TCR sequence provided that said variation retains its functional ability to bind to the epitope with the amino acid sequence of SEQ ID NO. 14 *(GPC3 peptide* 367) or to its HLA-A2 bound form
and/or
(ii) a T-cell receptor β-chain comprising the amino acid sequence of SEQ ID NO. 2 *(variable beta)* and SEQ ID NO. 4 *(murine beta),* preferably comprising the amino acid sequence of SEQ ID NO. 7 *(variable beta* + *murine beta)*
or an amino acid sequence that has at least 80 % identity to the amino acid sequence of SEQ ID NO. 7, more preferably at least 85 % identity, more preferably 90 % or 95 % identity,
or any variation of the TCR sequence provided that said variation retains its functional ability to bind to the epitope with the amino acid sequence of SEQ ID NO. 14 *(GPC3 peptide* 367) or to its HLA-A2 bound form;
or comprising the amino acid sequence of SEQ ID NO. 8 *(aa sequence of the entire TCR P1-*1).

4. A soluble T-cell receptor (sTCR) construct comprising
(1) at least one of
T-cell receptor α-chain(s) as defined in claim 1 or 3,
T-cell receptor β-chain(s) as defined in claim 1 or 3,
preferably covalently linked to each other to form TCR heterodimers or multimers,
and
(2) fusion component(s)
preferably selected from
Fc receptors and/or Fc domains,
cytokines, such as IL-2 or IL-15,
toxins,
antibodies, such as anti-CD3, anti-CD28, anti-CDS, anti-CD16 or anti-CD56 antibodies,
or combinations thereof,
wherein the at least one T-cell receptor chain (1) is bound to the fusion component(s) (2),
wherein the soluble T-cell receptor comprises preferably furthermore labels, such as radionuclides, fluorophors, gold particles.

5. A chimeric T-cell receptor comprising
at least one of
the T-cell receptor α-chain as defined in claim 1 or 3,
the T-cell receptor β-chain as defined in claim 1 or 3, or
the T-cell receptor of any of claims 1 to 3,
wherein the TCR α-chain and/or the TCR β-chain is/are fused to CD3-zeta chain(s) and/or other TCR stimulation domains, such as the intracellular CD28, CD137 or CD134 domain, preferably via a linker.

6. A bi-specific antibody comprising
(a) the T-cell receptor α- and β-chain(s) of the TCR as defined in claim 1 or 3, which are linked with each other
and fused, preferably via a linker, to
(b) an antibody or a single chain antibody fragment (scFv) which is directed against an antigen or epitope on the surface of lymphocytes.

7. A nucleic acid encoding the T-cell receptor according to any one of claims 1 to 3 or encoding the soluble T-cell receptor construct according to claim 4 or encoding the chimeric T-cell receptor according to claim 5 or encoding the bi-specific antibody according to claim 6.

8. The nucleic acid of claim 7, comprising
the nucleic acid encoding for the amino acid sequence of SEQ ID NO. 1 *(variable alpha)* and/or SEQ ID NO. 2 (*variable beta)*
or the nucleotide sequence of SEQ ID NO. 9 *(variable alpha)* and/or SEQ ID NO. 10 *(variable beta)*
or their complementary sequence(s)
or sequence(s) that have at least 80 % identity to the nucleotide sequence of SEQ ID NO. 9 or 10, more preferably at least 85 % identity, more preferably 90 or 95 or 99 % identity;
or comprising
the nucleic acid encoding for the amino acid sequence of SEQ ID NO. 6 *(variable alpha + murine alpha)* and/or SEQ ID NO. 7 *(variable beta + murine beta)*
the nucleotide sequence of SEQ ID NO. 11 *(Variable alpha* + *murine alpha)* and/or SEQ ID NO. 12 *(variable beta + murine beta)*
or their complementary sequence(s)
or sequence(s) that have at least 80 % identity to the nucleotide sequence of SEQ ID NO. 11 or 12, more preferably at least 85 % identity, more preferably 90 or 95 or 99 % identity;
or comprising
the nucleic acid encoding for the amino acid sequence of SEQ ID NO. 8 *(aa sequence of the entire TCR P1-1)*
the nucleotide sequence of SEQ ID NO. 13 *(entire TCR P1-1)*
or their complementary sequence(s)
or sequence(s) that have at least 80 % identity to the nucleotide sequence of SEQ ID NO. 13, more preferably at least 85 % identity, more preferably 90 or 95 or 99 % identity.

9. An expression construct for expressing the T-cell receptor or soluble T-cell receptor construct or the chimeric T-cell receptor or the bi-specific antibody according to any one of claims 1 to 6 in a cell, preferably further comprising promoter and terminator sequences.

10. A cell comprising the T-cell receptor or soluble T-cell receptor construct or chimeric T-cell receptor or bi-specific antibody according to any one of claims 1 to 6 or the nucleic acid(s) of claim 7 or claim 8 or the expression construct of claim 9,
preferably expressing the T-cell receptor or soluble T-cell receptor construct according or chimeric T-cell receptor or bi-specific antibody according to any one of claims 1 to 6,
which is selected from lymphocytes including but not limited to cytotoxic T lymphocytes (CTLs), CD8+ T cells, CD4+ T cells, natural killer (NK) cells, natural killer T (NKT) cells, gammaldelta-T-cells.

11. A pharmaceutical composition comprising one or more of:
(i) the T-cell receptor of any one of claims 1 to 3;
(ii) the soluble T-cell receptor construct of claim 4;
(iii) the chimeric T-cell receptor of claim 5;
(iv) the bi-specific antibody of claim 6;
(v) the nucleic acid(s) of claim 7 or 8 or the expression construct of claim 9; and/or
(vi) the cell of claim 10,
and, optionally, pharmaceutically excipient(s).

12. Use of a T-cell receptor of claims 1 to 3, a chimeric T-cell receptor of claim 5, a bispecific antibody of claim 6, a nucleic acid of claim 7 or 8 or an expression construct of claim 9 for generating genetically modified lymphocytes, preferably T cells, NK cells and/or NKT cells,
wherein preferably the generated genetically modified lymphocytes, preferably T cells are used for genetic immunization, more preferably for preventing and/or treating liver cancer, in particular hepatocellular carcinoma (HCC), or other cancers expressing GPC3.

13. The T-cell receptor of claims 1 to 3, the soluble T-cell receptor construct of claim 4, the chimeric T-cell receptor of claim 5, the bi-specific antibody of claim 6, the nucleic acid of claims 7 or 8, the expression construct of claim 9 or the host cell of claim 10 for use as a medicament.

14. The T-cell receptor of claims 1 to 3, the soluble T-cell receptor construct of claim 4, the chimeric T-cell receptor of claim 5, the bi-specific antibody of claim 6, the nucleic acid of claims 7 or 8, the expression construct of claim 9 or the host cell of claim 10 for use in the detection, diagnosis, prognosis, prevention and/or treatment of liver cancer, in particular hepatocellular carcinoma (HCC), or other cancers expressing GPC3.

15. The T-cell receptor, nucleic acid, expression construct or cell of claim 13, comprising the use of adoptive, target-cell specific immunotherapy or genetic immunization,
wherein the patients are preferably HLA-A2-positive.

16. A method of preventing and/or treating liver cancer, in particular hepatocellular carcinoma (HCC), or other cancers expressing GPC3,
comprising the steps of
(a) providing lymphocytes of a patient or a blood donor;
(b) providing one or more of
(i) a T-cell receptor of any of claims 1 to 3 or a soluble T-cell receptor construct of claim 4 or a chimeric T-cell receptor of claim 5 or a bi-specific antibody of claim 6,
(ii) a nucleic acid of any of claims 7 or 8,
(iii) an expression construct of claim 9,
(iv) a cell of claim 10, and
(v) a pharmaceutical composition of claim 11;
(c) *ex vivo* introduction of one or more of (i) to (v) of step (b) into the lymphocytes of step (a) and, thereby, obtaining genetically modified lymphocytes,
(d) administering the genetically modified lymphocytes of step (c) to a subject or patient in need thereof,
(e) optional, combining any of the steps (b) to (d) with radiation, checkpoint inhibitor or cancer chemotherapy,
wherein the patients are preferably HLA-A2-positive,
and/or wherein the lymphocytes isolated in step (a) are preferably T cells, more preferably CD8⁺ T cells, NK cells and/or NKT cells,
and/or wherein the *ex vivo* introduction in step (c) is preferably carried out via electroporation or by transfection reagents, such as liposomes.

17. A method of preventing and/or treating liver cancer, in particular hepatocellular carcinoma (HCC), or other cancers expressing GPC3,
comprising the steps of
(a) providing one or more of
(i) a T-cell receptor of any of claims 1 to 3 or a soluble T-cell receptor construct of claim 4 or a chimeric T-cell receptor of claim 5 or a bi-specific antibody of claim 6,
(ii) a nucleic acid of any of claims 7 or 8,
(iii) an expression construct of claim 9,
(iv) a cell of claim 10, and
(v) a pharmaceutical composition of claim 11;
(b) direct application, preferably via injection or infusion, of one or more of (i) to (v) of step (a) to a subject or patient in need thereof,
(c) optional, combining step (b) with radiation, checkpoint inhibitor or cancer chemotherapy,
wherein the patients are preferably HLA-A2-positive.

18. A method of detecting, diagnosing, prognosing, preventing and/or treating liver cancer, in particular hepatocellular carcinoma (HCC), or other cancers expressing GPC3, comprising the detection and/or destruction of (liver) cancer cells, in particular hepatocellular carcinoma (HCC) cells, of a patient with the use of the soluble T-cell receptor construct of claim 4,
wherein the soluble T-cell receptor is preferably applied by intravenous, subcutaneous or intramuscular infusion or injections, respectively, or by application to the mucosa of the respiratory tract by inhalation or by spraying,
wherein the patients are preferably HLA-A2-positive.

19. The method of claim 18, for detecting liver cancer, in particular hepatocellular carcinoma (HCC), or other cancers expressing GPC3,
comprising *the in vitro* staining of (liver) cancer cells, in particular hepatocellular carcinoma (HCC) cells, by using the soluble T-cell receptor construct of claim 4 which is labelled.

20. A peptide comprising at least one GPC3 epitope comprising the amino acid sequence of SEQ ID NO. 14 *(GPC3 peptide 367).*

21. A nucleic acid molecule coding for at least one peptide according to claim 20 or a plasmid comprising at least one such nucleic acid molecule.

22. An antibody against a peptide according to claim 20.

23. A composition comprising
(i) at least one peptide according to claim 20 or at least one nucleic acid according to claim 21,
(ii) optionally, a carrier,
(iii) optionally, an adjuvant,
preferably comprising at least two peptides according to claim 20 or at least two nucleic acids according to claim 21.

24. The peptide of claim 20, the nucleic acid molecule of claim 21 or the pharmaceutical composition of claim 23 for use in medicine,
preferably for use as a vaccine,
more preferably for preventing and/or treating liver cancer, in particular hepatocellular carcinoma (HCC), or other cancers expressing GPC3.

25. A vaccine comprising
(i) at least one peptide according to claim 20 or at least one nucleic acid according to claim 21 or a composition of claim 23,
(ii) optionally, an excipient.

26. Use of the vaccine of claim 25 for preventing and/or treating liver cancer, in particular hepatocellular carcinoma (HCC).

27. A method of preventing and/or treating liver cancer, in particular hepatocellular carcinoma (HCC), or other cancers expressing GPC3,
comprising the step of
administering a peptide of claim 20, a nucleic acid molecule of claim 21, a pharmaceutical composition of claim 23 or a vaccine of claim 25
to a subj ect in need thereof.
